(19) Europäisches Patentamt
European Patent Office
Office européen des brevets

(11) EP 4 547 208 B1

(12) **EUROPEAN PATENT SPECIFICATION**

(45) Date of publication and mention of the grant of the patent:
**27.05.2026 Bulletin 2026/22**

(21) Application number: **23736334.6**

(22) Date of filing: **29.06.2023**

(51) International Patent Classification (IPC):
***A61K 8/41*** *(2006.01)* ***A61K 8/49*** *(2006.01)*
***A61K 8/88*** *(2006.01)* ***A61Q 5/06*** *(2006.01)*
***A61Q 5/08*** *(2006.01)* ***A46B 5/00*** *(2006.01)*

(52) Cooperative Patent Classification (CPC):
**A61K 8/41; A46B 5/0012; A61K 8/4973; A61K 8/88; A61Q 5/065; A61Q 5/08;**
A46B 2200/104; A61K 2800/87; A61K 2800/882; A61K 2800/884

(86) International application number:
**PCT/EP2023/067913**

(87) International publication number:
**WO 2024/003303 (04.01.2024 Gazette 2024/01)**

# (54) PROCESS FOR REMOVING THE COLOUR FROM PREVIOUSLY DYED KERATINOUS HAIR FIBRES

VERFAHREN ZUR FARBENTFERNUNG VON BEREITS GEFÄRBTEN KERATINISCHEN HAARFASERN

PROCÉDÉ D'ÉLIMINATION DE LA COULEUR DE FIBRES CAPILLAIRES KÉRATINIQUES COLORÉES PRÉCÉDEMMENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.06.2022 FR 2206613**

(43) Date of publication of application:
**07.05.2025 Bulletin 2025/19**

(73) Proprietor: **L'OREAL**
**75008 Paris (FR)**

(72) Inventors:
- **DEBAIN, Jean-Daniel**
**93400 SAINT OUEN (FR)**
- **CARDONNEL, Fanny**
**93400 SAINT OUEN (FR)**

(74) Representative: **Casalonga**
**Casalonga & Partners**
**Bayerstraße 71/73**
**80335 München (DE)**

(56) References cited:
**FR-A1- 2 931 635** **FR-A1- 3 103 385**
**JP-A- 2003 034 619** **US-B1- 10 973 311**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 547 208 B1

## Description

The present invention relates to a process for removing the colour from keratinous hair fibres previously dyed by means of at least one composition for dyeing keratinous hair fibres, comprising at least one (poly)carbodiimide compound; and at least one colouring agent chosen from pigments, direct dyes and mixtures thereof. Said process comprises the following steps: i) applying, to the keratinous hair fibres previously dyed by means of said dyeing composition, at least one colour-removing composition, comprising at least one alkyl or alkylene carbonate; and ii) applying, to said keratinous hair fibres, tongs comprising two arms A and B, between which arms said keratinous hair fibres pass, as described below.

## Technical field

In the field of dyeing keratinous hair fibres, in particular human keratinous hair fibres, it is already known practice to colour keratinous hair fibres via various techniques using direct dyes or pigments for non-permanent colouring, or dye precursors for permanent colouring.

There are essentially three types of process for dyeing the hair:

a) "permanent" dyeing, the function of which is to afford a substantial modification to the natural colour and which uses oxidation dyes which penetrate into the hair fibre and forms the dye via an oxidative condensation process;

b) non-permanent, semi-permanent or direct dyeing, which does not use the oxidative condensation process and withstands four or five shampooing operations; it consists in dyeing keratinous fibres with dye compositions containing direct dyes;

c) temporary dyeing, which gives rise to a modification of the natural colour of the hair that remains from one shampooing operation to the next, and which serves to enhance or correct a shade that has already been obtained. It may also be likened to a "makeup" process.

For this last type of dyeing, it is known practice to use coloured polymers formed by grafting one or more dyes of azo, triphenylmethane, azine, indoamine or anthraquinone nature onto a polymer chain. These coloured polymers are not entirely satisfactory, particularly regarding the homogeneity of the colouring obtained and its resistance, not to mention the problems associated with their manufacture and in particular with their reproducibility.

Another dyeing method consists in using pigments. Specifically, the use of pigment on the surface of keratinous hair fibres generally makes it possible to obtain visible colourings on dark hair, since the surface pigment masks the natural colour of the fibre.

This dyeing method can also afford dye compositions which have the advantage of producing a uniform coloured coating on the keratinous hair fibres, particularly the hair, while at the same time forming a coat which withstands shampooing operations and the various attacking factors to which the hair may be subjected such as brushing and/or friction, without degradation of the hair.

JP2003034619 discloses a process for removing the colour from keratinous hair fibres previously dyed with a composition for dyeing keratinous hair fibres comprising at least one colouring agent chosen from pigments, direct dyes and mixtures thereof.

However, no processes exist for removing the colour from keratinous hair fibres previously dyed using pigments that efficiently implement colour-removing compositions and tools with this type of temporary dye composition which withstands shampooing operations.

A need thus remains for a process for efficiently removing colour from keratinous hair fibres, such as the hair, which have been previously dyed by means of said dye compositions which withstand shampooing operations.

Thus, the aim of the present invention is to develop a process for efficiently removing the colour from keratinous hair fibres, such as the hair.

## Disclosure of the invention

The subject matter of the present invention is therefore a process for removing the colour from keratinous hair fibres which have been previously dyed by means of at least one composition for dyeing keratinous hair fibres, comprising:

- at least one (poly)carbodiimide compound; and
- at least one colouring agent chosen from pigments, direct dyes and mixtures thereof; said process comprising the following steps:

    i) applying, to the keratinous hair fibres previously dyed by means of said dyeing composition, at least one colour-removing composition, comprising:

- at least one alkyl or alkylene carbonate; and

ii) applying, to said keratinous hair fibres, tongs comprising two arms A and B, between which arms said keratinous hair fibres pass,

each arm A and B comprising a supporting part and an active part,
said arms A and B being movable between a spaced-apart position in which the active parts are at a distance from one another, and a brought-together position in which the active parts are brought towards one another,
said active parts comprising an active face and an opposite face, said active face having bristles,
wherein, in the brought-together position of the active parts, said bristles of the arms A and B intermingle so as to grip said keratinous hair fibres without blocking them.

The combination of a composition for removing colour, comprising at least one alkyl or alkylene carbonate, and tongs, as described above, makes it possible to efficiently remove colour from keratinous hair fibres previously dyed by means of the dyeing composition, as described previously.
*"Keratinous hair fibres"* means the hair. In other words, the expressions "keratinous hair fibres" and "hair" are equivalent in the remainder of the description.
For the purposes of the present invention, *"hair"* means head hair. This term does not correspond to body hair, the eyebrows or the eyelashes.
The expression *"at least one"* means one or more.
Unless otherwise indicated, the limits of a range of values are included in that range, particularly in the expressions "between" and "ranging from ... to ...".
For the purposes of the present invention and unless otherwise indicated:

- an *"alkyl"* radical denotes a linear or branched saturated radical containing, for example, from 1 to 20 carbon atoms;
- an *"aminoalkyl"* radical denotes an alkyl radical as defined previously, said alkyl radical comprising an $NH_2$ group;
- a *"hydroxyalkyl"* radical denotes an alkyl radical as defined previously, said alkyl radical comprising an OH group;
- an *"alkylene"* radical denotes a linear or branched divalent saturated $C_2$-$C_4$ hydrocarbon-based group such as methylene, ethylene or propylene;
- a *"cycloalkyl"* or *"alicycloalkyl"* radical denotes a cyclic saturated monocyclic or polycyclic, preferably monocyclic, hydrocarbon-based group comprising from 1 to 3 rings, preferably 2 rings, and comprising from 3 to 24 carbon atoms, in particular comprising from 3 to 20 carbon atoms, more particularly from 3 to 13 carbon atoms, even more particularly from 3 to 12 carbon atoms, preferably between 5 and 10 carbon atoms, such as cyclopropyl, cyclopentyl, cyclohexyl, cycloheptyl or norbornyl, in particular cyclopropyl, cyclopentyl or cyclohexyl, it being understood that the cycloalkyl radical may be substituted with one or more ($C_1$-$C_4$)alkyl groups such as methyl; preferably, the cycloalkyl radical is then an isobornyl group;
- a *"cycloalkylene"* radical denotes a divalent cycloalkyl group with *"cycloalkyl"* as defined previously, preferably of $C_3$-$C_{12}$;
- an *"aryl"* radical is a monocyclic, bicyclic or tricyclic, fused or non-fused, unsaturated and aromatic hydrocarbon-based cyclic radical, comprising from 6 to 14 carbon atoms, preferably between 6 and 12 carbon atoms; preferably, the aryl group comprises 1 ring of 6 carbon atoms such as phenyl, naphthyl, anthryl, phenanthryl and biphenyl, it being understood that the aryl radical may be substituted with one or more ($C_1$-$C_4$)alkyl groups such as methyl, preferably tolyl, xylyl, or methylnaphthyl; preferably, the aryl group represents phenyl;
- an *"arylene"* radical is a divalent aryl radical with *"aryl"* as defined previously; preferably, arylene represents phenylene;
- a *"heterocyclic"* radical denotes a saturated or unsaturated, non-aromatic or aromatic, monocyclic or polycyclic hydrocarbon-based radical, comprising one or more heteroatoms, preferably from 1 to 5 atoms chosen from O, S or N, including from 3 to 20 ring members, preferably between 5 and 10 ring members, such as imidazolyl, pyrrolyl and furanyl;
- a *"heterocycloalkylene"* radical is a divalent heterocyclic group with *"heterocyclic"* as defined previously;
- an *"aryloxy"* radical denotes an aryl-oxy radical with *"aryl"* as defined previously;
- an *"alkoxy"* radical denotes an alkyl-oxy radical with *"alkyl"* as defined previously;
- an *"acyloxy"* radical denotes an ester radical R-C(O)-O- with R being an alkyl group as defined above;
- a "reactive" group is a group that is capable of forming a covalent bond with another identical or different group, by chemical reaction.

Unless otherwise indicated, when compounds are mentioned in the present patent application, this also includes the optical isomers thereof, the geometric isomers thereof, the tautomers thereof or the salts thereof, alone or as a mixture.

*"Keratinous hair fibres"* means the hair. In other words, the expressions "keratinous hair fibres" and "hair" are equivalent in the remainder of the description.

For the purposes of the present invention, *"hair"* means head hair. This term does not correspond to body hair, the eyebrows or the eyelashes.

## Detailed description of the invention

**Dyeing composition:**

### Polycarbodiimide compound

As indicated previously, the composition for dyeing keratinous hair fibres used in the context of the process according to the invention comprises at least one (poly)carbodiimide compound.

The composition may comprise at least two different (poly)carbodiimide compounds, present as a mixture in the composition.

The term *"(poly)carbodiimide compound"* means a compound comprising one or more carbodiimide groups, preferably at least two carbodiimide groups, more preferentially at least three carbodiimide groups; in particular, the number of carbodiimide groups does not exceed 200, preferably 150, more preferentially 100.

The term *"carbodiimide group"* means a divalent linear triatomic fraction of general formula -(N=C=N)-.

The (poly)carbodiimide compound(s) according to the invention may optionally comprise in their structure one or more reactive groups other than carbodiimide groups, chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

The reactive group(s) other than the carbodiimide groups may be pendent or end groups. Preferably, the (poly)carbodiimide compound(s) comprise one or more end groups other than carbodiimide groups, preferably one or more end groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

According to a particular embodiment, the (poly)carbodiimide compound is chosen from the compounds of formula (I) below:

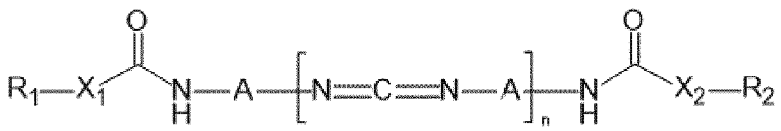

(I),

in which:

- $X_1$ and $X_2$ independently represent an oxygen atom O, a sulfur atom S or an NH group;
- $R_1$ and $R_2$ independently represent a group chosen from a hydrocarbon-based radical, preferably alkyl, optionally interrupted with one or more heteroatom(s), a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatom(s) and with one or more groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

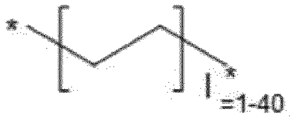 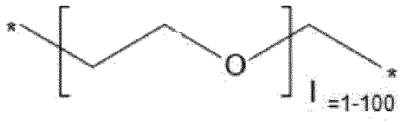

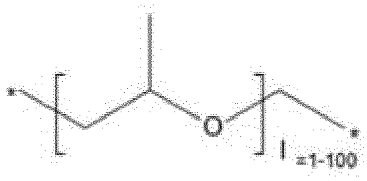 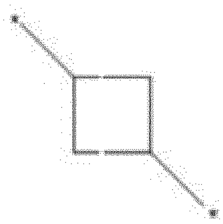

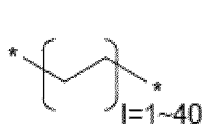 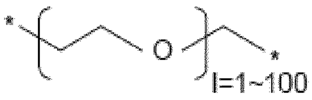 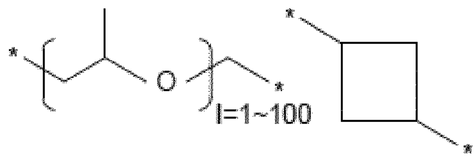

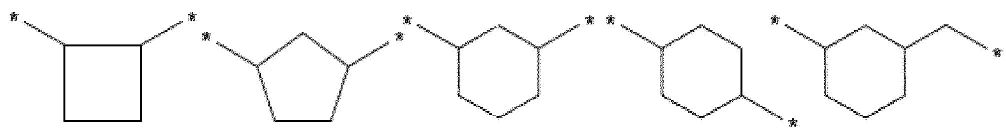

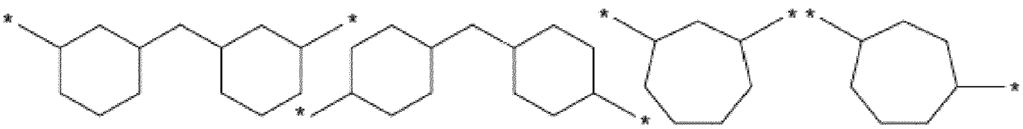

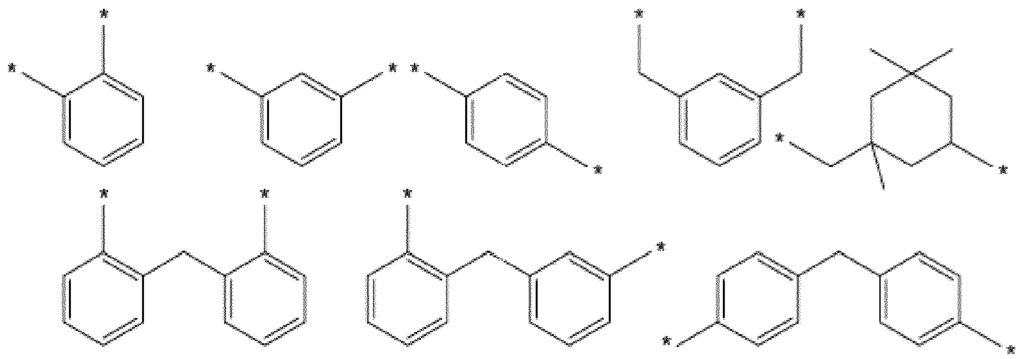

.

According to another embodiment, the (poly)carbodiimide compound is chosen from the compounds of formula (I') below:

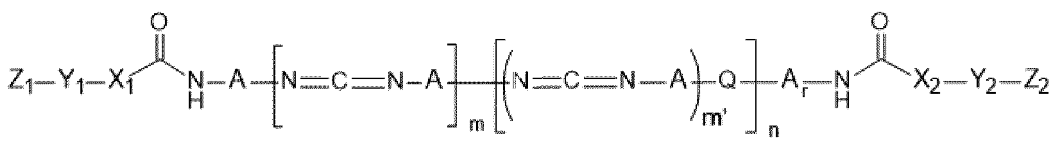

(I')

in which:

- $X_1$ and $X_2$ independently represent an oxygen atom O, a sulfur atom S or an NH group;
- $Y_1$ and $Y_2$ independently represent a divalent organic radical chosen from a saturated $C_1$ to $C_{36}$ aliphatic group or a $C_6$ to $C_{24}$ aromatic or alkylaromatic group, the aliphatic or aromatic group optionally comprising one or more non-pendent heteroatoms, such as a nitrogen atom, an oxygen atom, a sulfur atom, or combinations thereof;
- $Z_1$ and $Z_2$ independently represent a reactive end group or an inert end group;
- as inert end group, $Z_1$ and $Z_2$ may represent, independently, a saturated, linear or branched or cyclic $C_1$ to $C_{50}$ aliphatic group, or a $C_6$ to $C_{18}$ aromatic group, said aliphatic and aromatic groups optionally comprising from 1 to 10 heteroatoms chosen from nitrogen, oxygen, sulfur and combinations thereof, and the aliphatic or aromatic group may be partially or totally fluorinated; in this variant, $Z_1$ and $Z_2$ comprise a bonding group CG connecting $Z_1$ to $Y_1$ and $Z_2$ to $Y_2$, the group CG possibly being a single covalent bond, a saturated C-C bond, an unsaturated covalent C-C bond, an amide group, an ester group, a carbonate group, a thioester group, an ether group, a urethane group, a thiourethane group or a urea group;
- as reactive end group, $Z_1$ and $Z_2$ may be chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- Q represents an organopolymer or an organooligomer comprising repeating units of saturated, linear or branched or cyclic aliphatic groups, or of aromatic groups or alkylaromatic groups, coupled via carbonate, ester, ether, amide, urethane or urea repeating bonds or combinations thereof;
- A represents a divalent aliphatic, aromatic, alkylaromatic or linear, saturated, branched or cyclic radical having from 2 to 30 carbon atoms, which may optionally comprise one or more non-pendent heteroatoms such as a nitrogen atom, an oxygen atom, a sulfur atom, or combinations thereof, in the aliphatic chain or the aromatic chain;
- r denotes an integer equal to 0 or 1;
- m denotes an integer ranging from 0 to 1000, preferably equal to 0 or 1;
- m' denotes an integer ranging from 0 to 1000, preferably equal to 0 or 1;
- n denotes an integer ranging from 0 to 1000, preferably equal to 0 or 1, with $m + (m'*n) \geq 2$.

Preferably, $Z_1$ and $Z_2$ independently represent a reactive end group; more preferentially, $Z_1$ and $Z_2$ independently represent a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

Such (poly)carbodiimide compounds are sold, for example, by the company Stahl B.V, under the name Permutex XR, or under the name RelcaLink10. or under the name Picassian XL, and by the company Nisshinbo under the name Carbodilite with the series V-02, V-02-L2, SV-02, E-02, V-10, SW-12G, E-03A, E-04DG-T, E-05, V-04, V-02B, V-04PF, V-05.

Preferably, the (poly)carbodiimide compound(s) is (are) chosen from the compounds of formula (II) below:

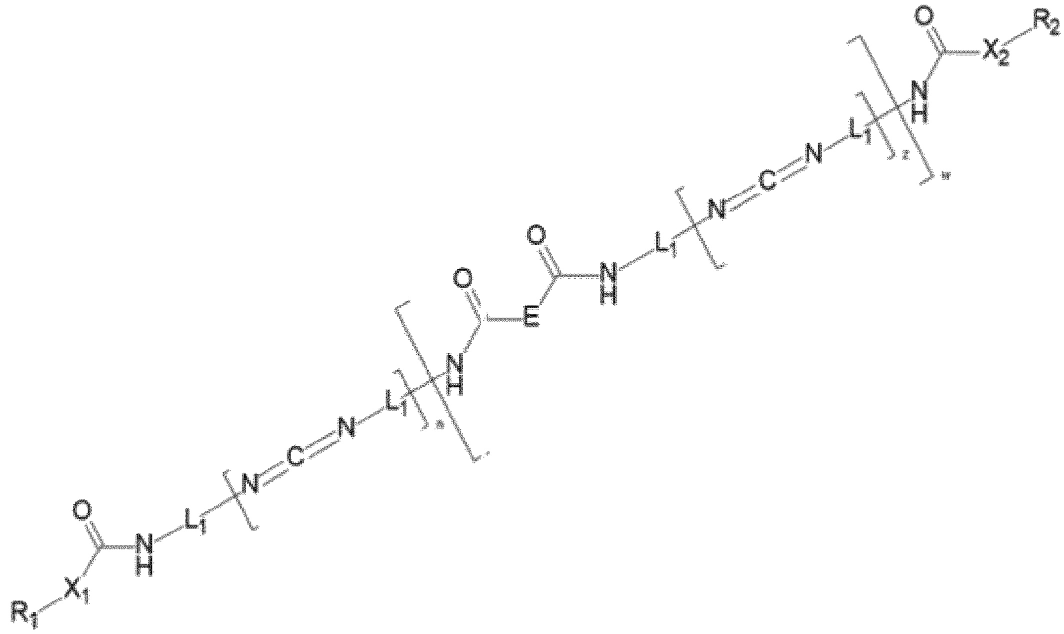

(II),

in which:

- $X_1$ and $X_2$ independently represent an oxygen atom O, a sulfur atom S or an NH group;
- $R_1$ and $R_2$ independently represent a hydrocarbon-based radical optionally interrupted with one or more heteroatom(s);
- n and z denote an integer ranging from 1 to 20, with $n+z \geq 2$ and w denoting an integer ranging from 1 to 3;
- $L_1$ independently represents a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- E independently represents a group chosen from:

  - -O-$R_3$-O-; -S-$R_4$-S-; -$R_5$-N($R_6$)-$R_4$-N($R_6$)-$R_5$-;

  in which $R_3$ and $R_4$ independently represent a divalent hydrocarbon-based radical optionally interrupted with one or more heteroatom(s);
- $R_5$ independently represents a covalent bond or a saturated divalent hydrocarbon-based radical, optionally interrupted with one or more heteroatom(s);
- $R_6$ independently represents a hydrogen atom or a hydrocarbon-based radical, optionally interrupted with one or more heteroatom(s).

The term *"hydrocarbon-based radical"* means a saturated or unsaturated, linear or branched radical having from 1 to 300 carbon atoms, preferably from 1 to 250 carbon atoms, more preferentially from 1 to 200 carbon atoms. Preferably, the hydrocarbon-based radical is a saturated linear radical.

The hydrocarbon-based radical may comprise one or more cyclic groups.

The hydrocarbon-based radical may be interrupted with one or more heteroatom(s), in particular chosen from O, S or N and/or substituted with one or more cation(s), anion(s) or zwitterion(s) or cationic group(s) such as ammonium, anionic group(s) such as carboxylate, or zwitterionic group(s), and/or comprising a metal ion which may be incorporated in the form of a salt.

The term *"heteroatom(s)"* means an oxygen O, sulfur S or nitrogen N atom, and also halogen atoms such as Cl, F, Br and I. If the heteroatom is included in the chain of the hydrocarbon-based radical, the heteroatom is preferably chosen from oxygen O, sulfur S or nitrogen N atoms.

Preferably, $X_1$ and $X_2$ independently represent an oxygen atom.

Preferably, $R_1$ and $R_2$ are independently chosen from dialkylamino alcohols, alkyl esters of hydroxycarboxylic acid and monoalkyl ethers of (poly)alkylene glycol, in which a hydroxyl group has been removed, and mixtures thereof.

In a preferred embodiment, $R_1$ and $R_2$ are independently chosen from groups (i) to (iv) below:

(i) the compound of formula (III) below:

$$R_7\text{-O-C(O)-C}(R_8)\text{(H)-} \quad \text{(III)},$$

in which $R_7$ represents a $C_1$-$C_3$ alkyl group, and $R_8$ represents a hydrogen atom or a $C_1$-$C_3$ alkyl group; preferably, $R_7$ is a methyl and $R_8$ is a hydrogen atom or a methyl.

(ii) the compound of formula (IV) below:

$$R_0\text{-[O-CH}_2\text{-C(H)}(R_{10})]_p\text{-} \quad \text{(IV)},$$

in which $R_9$ represents a $C_1$-$C_4$ alkyl group, $R_{10}$ represents a hydrogen atom or a $C_1$-$C_4$ alkyl group and p denotes an integer ranging from 1 to 3; preferably, $R_9$ is a methyl, ethyl or butyl, $R_{10}$ is a hydrogen atom or a methyl and p is equal to 1.

(iii) the compound of formula (V) below:

$$(R_{11})_2\text{N-CH}_2\text{-C(H)}(R_{12})\text{-} \quad \text{(V)},$$

in which $R_{11}$ represents a $C_1$-$C_4$ alkyl group and $R_{12}$ represents a hydrogen atom or a $C_1$-$C_4$ alkyl group; preferably, $R_{11}$ is a methyl, ethyl or butyl and $R_{12}$ is a hydrogen atom or a methyl.

(iv) the compound of formula (VI) below:

$$R_{13}\text{-[O-CH}_2\text{-C(H)}(R_{14})]_q\text{-} \quad \text{(VI)},$$

in which $R_{13}$ represents a $C_1$-$C_4$ alkyl group or a phenyl, $R_{14}$ represents a hydrogen atom or a $C_1$-$C_4$ alkyl group and q denotes an integer ranging from 4 to 30; preferably, $R_{13}$ is a methyl, ethyl or butyl and $R_{14}$ is a hydrogen atom or a methyl.

Preferably, $R_1$ and $R_2$ independently represent a compound of formula (VI) in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and q denotes an integer ranging from 4 to 30.

According to an alternative embodiment, $R_1$ and $R_2$ are different and one of the radicals $R_1$ or $R_2$ represents a compound of formula (IV) as described above and the other radical $R_1$ or $R_2$ represents a compound of formula (VI) as described above.

Preferably, in formula (IV), $R_9$ is a methyl, ethyl or butyl and $R_{10}$ is a hydrogen atom or a methyl and p is equal to 1.

Preferably, in formula (VI), $R_{13}$ is a methyl, ethyl or butyl and $R_{14}$ is a hydrogen atom or a methyl and q denotes an integer ranging from 4 to 30.

According to another alternative embodiment, $R_1$ and $R_2$ are identical and represent a compound of formula (VI) in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and q denotes an integer ranging from 4 to 30.

Preferably, n denotes an integer ranging from 1 to 20, more preferentially from 2 to 20. Preferably, z denotes an integer ranging from 1 to 20, more preferentially from 2 to 20. Preferably, w is equal to 1.

Preferably, w is equal to 1, n+z denotes an integer ranging from 4 to 10.

Preferably, $L_1$ is chosen from a $C_1$-$C_{18}$ divalent aliphatic hydrocarbon-based radical such as methylene, ethylene and propylene, a $C_3$-$C_{15}$ cycloalkylene radical such as cyclopentylene, cycloheptylene and cyclohexylene, a $C_3$-$C_{12}$ heterocycloalkylene group such as imidazolene, pyrrolene and furanylene, or a $C_6$-$C_{14}$ arylene group such as phenylene, and mixtures thereof.

For example, $L_1$ may be chosen from a radical derived from tolylene diisocyanate, hexamethylene diisocyanate, xylylene diisocyanate, 2,2,4-trimethylhexamethylene diisocyanate, 1,12-dodecane diisocyanate, norbornane diisocyanate, 2,4-bis(8-isocyanatooctyl)-1,3-dioctylcyclobutane, 4,4'-dicyclohexylmethane diisocyanate, tetramethylxylylene diisocyanate, isophorone diisocyanate, 1,5-napththylene diisocyanate, 4,4'-diphenylmethane diisocyanate, 4,4'-diphenyldimethylmethane diisocyanate and phenylene diisocyanate, and mixtures thereof.

Preferably, $L_1$ is chosen from a $C_3$-$C_{15}$ cycloalkylene radical or a $C_6$-$C_{14}$ arylene group, and mixtures thereof, such as the compounds of formula (VII) below:

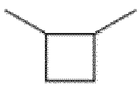
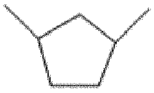
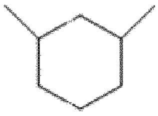
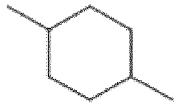
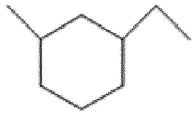
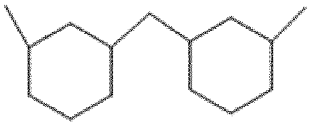
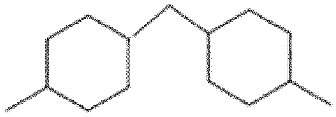
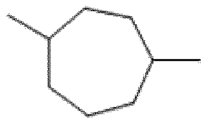
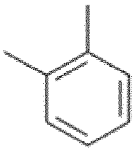
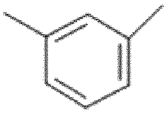
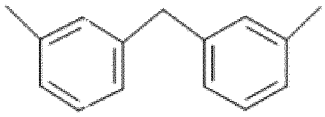
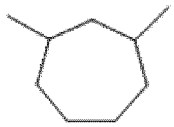
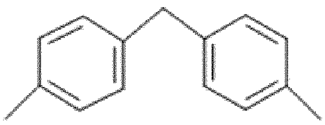
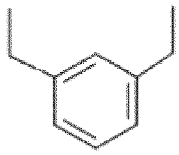

Preferably, $L_1$ is 4,4-dicyclohexylenemethane corresponding to formula (VIII) below:

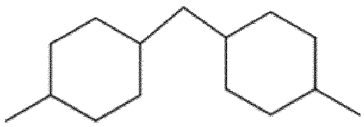
(VIII).

According to another embodiment, when L1 is a $C_6$-$C_{14}$ arylene group, $L_1$ is not the m-tetramethylxylylene radical represented by formula (IX) below:

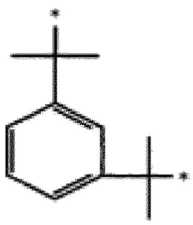
(IX).

As indicated previously, E independently represents a group chosen from:

- $-O-R_3-O-$; $-S-R_4-S-$; $-R_5-N(R_6)-R_4-N(R_6)-R_5-$;

in which $R_3$ and $R_4$ independently represent a divalent hydrocarbon-based radical optionally interrupted with one or more heteroatom(s);

- $R_5$ independently represents a covalent bond or a saturated divalent hydrocarbon-based radical, optionally interrupted with one or more heteroatom(s); and
- $R_6$ independently represents a hydrogen atom or a hydrocarbon-based radical, optionally interrupted with one or more heteroatom(s).

Preferably, $R_3$ and $R_4$ are independently chosen from a $C_6$-$C_{14}$ arylene radical such as phenylene, a $C_3$-$C_{12}$

cycloalkylene radical such as cyclopropylene and cyclobutylene, a linear or branched $C_1$-$C_{18}$ alkylene radical such as methylene and ethylene, optionally interrupted with one or more heteroatom(s), and mixtures thereof.

More preferentially, $R_3$ and $R_4$ are independently chosen from a linear or branched $C_1$-$C_{18}$ alkylene radical such as methylene, butylene, propylene, ethylene, optionally interrupted with one or more heteroatom(s).

Preferably, when $R_5$ is not a covalent bond, $R_5$ is chosen from a $C_6$-$C_{14}$ arylene radical such as phenylene, a $C_3$-$C_{12}$ cycloalkylene radical such as cyclopropylene and cyclobutylene, a linear or branched $C_1$-$C_{18}$ alkylene radical such as methylene and ethylene, optionally interrupted with one or more heteroatom(s), and mixtures thereof. Preferably, $R_6$ is chosen from a $C_6$-$C_{14}$ arylene radical such as phenylene, a $C_3$-$C_{12}$ cycloalkylene radical such as cyclopropylene and cyclobutylene, a linear or branched $C_1$-$C_{18}$ alkylene radical such as methylene and ethylene, optionally interrupted with one or more heteroatom(s), and mixtures thereof.

Preferably, E represents a group -O-$R_3$-O- in which $R_3$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

More preferentially, E represents a group -O-$R_3$-O- in which $R_3$ represents a linear or branched $C_1$-$C_{18}$ alkylene radical such as methylene, butylene, propylene, ethylene, optionally interrupted with one or more heteroatom(s).

According to a particular embodiment, the (poly)carbodiimide compound is a copolymer derived from alpha-methylstyryl isocyanates of formula (X) below:

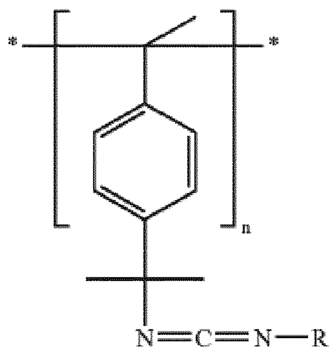

(X),

in which R independently represents an alkyl group having from 1 to 24 carbon atoms, a cycloalkyl group having from 3 to 24 carbon atoms or an aryl group having from 6 to 24 carbon atoms, and
n denotes an integer ranging from 2 to 100.

In this embodiment, the term "alkyl group" is as defined previously.

In this embodiment, the term "cycloalkyl group" is as defined previously.

In this embodiment, n may denote an integer ranging from 2 to 50, preferably from 3 to 30 and even more preferentially from 5 to 10.

According to another particular embodiment, the (poly)carbodiimide compound is a compound of formula (XI) below:

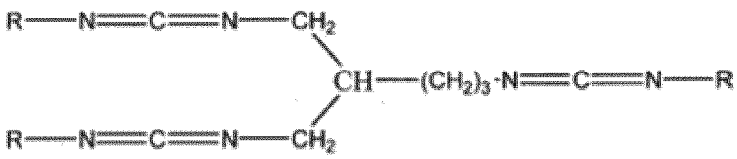

(XI),

in which R independently represents an alkyl group having from 1 to 24 carbon atoms, a cycloalkyl group having from 3 to 24 carbon atoms or an aryl group having from 6 to 24 carbon atoms.

The "alkyl group", the "cycloalkyl group" and the "aryl group" are as defined previously. According to a preferred embodiment, the (poly)carbodiimide compound is chosen from the compounds of formula (I) or of formula (II) in which:

- $X_1$ and $X_2$ independently represent an oxygen atom;
- $R_1$ and $R_2$ are independently chosen from dialkylamino alcohols, alkyl esters of hydroxycarboxylic acid and monoalkyl ethers of (poly)alkylene glycol, in which a hydroxyl group has been removed, and mixtures thereof, preferably

monoalkyl ethers of (poly)alkylene glycol, in which a hydroxyl group has been removed, more preferentially the compound of formula (VI) as described previously in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;

- n and z, when they are present, denote an integer ranging from 1 to 20, with $n+z \geq 2$ and w is equal to 1;
- $L_1$, when it is present, is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof, preferably a $C_3$-$C_{15}$ cycloalkylene radical;
- A, when it is present, is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof, preferably a $C_3$-$C_{15}$ cycloalkylene radical;
- E, when it is present, independently represents a group chosen from:

$$- -O-R_3-O-; -S-R_4-S-; -R_5-N(R_6)-R_4-N(R_6)-R_5-;$$

in which $R_3$ and $R_4$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof;

- when $R_5$ is not a covalent bond, $R_5$, when it is present, is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof; and
- $R_6$, when it is present, is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

Preferably, the (poly)carbodiimide compound is chosen from the compounds of formula (II) in which:

- $X_1$ and $X_2$ independently represent an oxygen atom;
- $R_1$ and $R_2$ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with $n+z \geq 2$ and w is equal to 1;
- $L_1$ is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- E independently represents a group chosen from:

$$- -O-R_3-O-; -S-R_4-S-; -R_5-N(R_6)-R_4-N(R_6)-R_5-;$$

in which $R_3$ and $R_4$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof;

- when $R_5$ is not a covalent bond, $R_5$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof; and
- $R_6$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

More preferentially, the (poly)carbodiimide compound is chosen from the compounds of formula (II) in which:

- $X_1$ and $X_2$ independently represent an oxygen atom;
- $R_1$ and $R_2$ are, independently, monoalkyl ethers of (poly)alkylene glycol, in which a hydroxyl group has been removed;
- n and z denote an integer ranging from 1 to 20, with $n+z \geq 2$ and w is equal to 1;
- $L_1$ is a $C_3$-$C_{15}$ cycloalkylene radical;
- E independently represents a group chosen from:

$$- -O-R_3-O-; -S-R_4-S-; -R_5-N(R_6)-R_4-N(R_6)-R_5-;$$

in which $R_3$ and $R_4$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof;

- when $R_5$ is not a covalent bond, $R_5$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof; and
- $R_6$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

Even more preferentially, the (poly)carbodiimide compound is chosen from the compounds of formula (II) in which:

- $X_1$ and $X_2$ independently represent an oxygen atom;
- $R_1$ and $R_2$ independently represent the compound of formula (VI) below:

$$R_{13}\text{-}[O\text{-}CH_2\text{-}C(H)(R_{14})]_q\text{-} \quad \text{(VI)},$$

  in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;
- n and z denote an integer ranging from 2 to 20, with n+z ranging from 4 to 10 and w is equal to 1;
- $L_1$ is a $C_3$-$C_{15}$ cycloalkylene radical such as cyclopentylene, cycloheptylene, cyclohexylene and 4,4-dicyclohexylenemethane; and
- E represents a group -O-$R_3$-O- wherein $R_3$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

Even more preferentially, the (poly)carbodiimide compound is chosen from the compounds of formula (II) in which:

- $X_1$ and $X_2$ independently represent an oxygen atom;
- $R_1$ and $R_2$ independently represent the compound of formula (VI) below:

$$R_{13}\text{-}[O\text{-}CH_2\text{-}C(H)(R_{14})]_q\text{-} \quad \text{(VI)}$$

  in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;
- n and z denote an integer ranging from 2 to 20, with n+z ranging from 4 to 10 and w is equal to 1;
- $L_1$ is a $C_3$-$C_{15}$ cycloalkylene radical such as cyclopentylene, cycloheptylene, cyclohexylene and 4,4-dicyclohexylenemethane, preferably 4,4-dicyclohexylenemethane; and
- E represents a group -O-$R_3$-O- in which $R_3$ represents a linear or branched $C_1$-$C_{18}$ alkylene radical such as methylene, propylene, butylene, ethylene, optionally interrupted with one or more heteroatom(s).

According to a preferred embodiment, the (poly)carbodiimide compound is a compound of formula (XII) below:

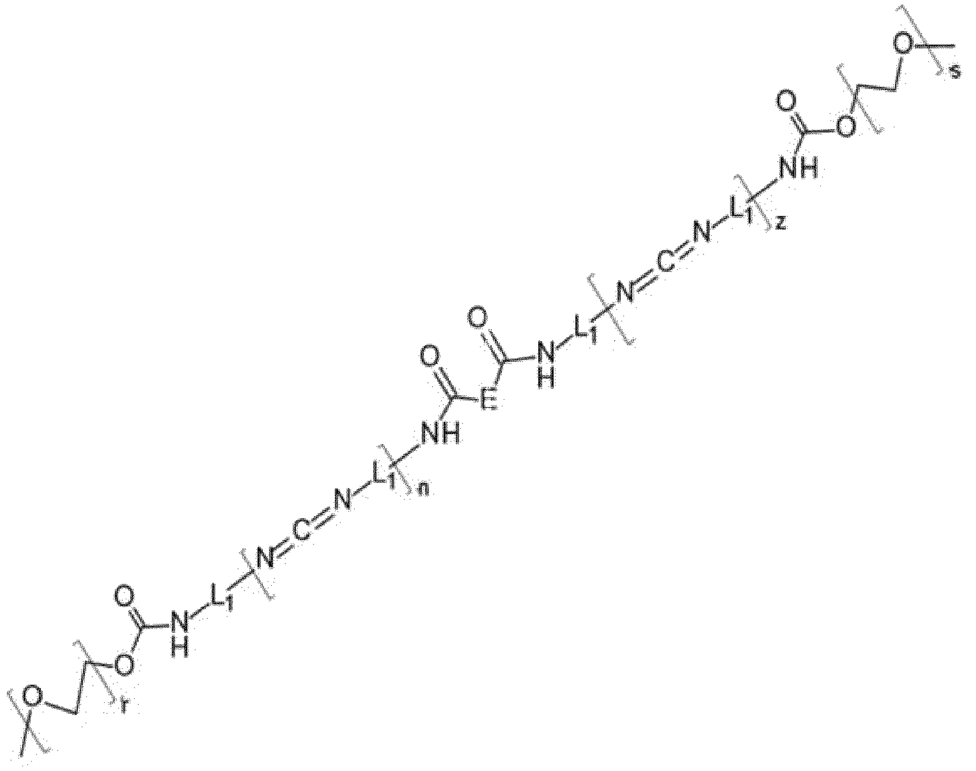

(XII),

in which L1 is 4,4-dicyclohexylenemethane, n and z denote an integer ranging from 2 to 20, with n+z ranging from 4 to 10, E represents a group -O-$R_3$-O- in which $R_3$ represents a linear or branched $C_1$-$C_{18}$ alkylene radical such as methylene, propylene, butylene, ethylene, optionally interrupted with one or more heteroatom(s), and r and s denote an integer ranging from 4 to 30.

The total amount of the (poly)carbodiimide compound(s) present in the dyeing composition preferably ranges from 0.01% to 20% by weight, preferably from 0.1% to 15% by weight, more preferentially from 0.2% to 10% by weight, even more preferentially from 0.5% to 8%, better still from 1% to 6% by weight relative to the total weight of the dyeing composition.

**Colouring agent**

As indicated previously, the composition for dyeing keratinous hair fibres used in the context of the process according to the invention comprises one or more colouring agent(s) chosen from pigments, direct dyes and mixtures thereof.

Preferably, the composition for dyeing keratinous hair fibres comprises one or more pigments.

The term *"pigment"* means any pigment that gives colour to keratinous materials. Their solubility in water at 25°C and at atmospheric pressure (760 mmHg) is less than 0.05% by weight, and preferably less than 0.01%.

The pigments that may be used are particularly chosen from the organic and/or mineral pigments known in the art, particularly those described in Kirk-Othmer's Encyclopedia of Chemical Technology and in Ullmann's Encyclopedia of Industrial Chemistry.

They may be natural, of natural origin, or non-natural.

These pigments may be in pigment powder or paste form. They may be coated or uncoated.

The pigments may be chosen, for example, from mineral pigments, organic pigments, lakes, pigments with special effects such as nacres or glitter flakes, and mixtures thereof. The pigment may be a mineral pigment. "Mineral pigment" means any pigment that satisfies the definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. "Organic pigment" means any pigment that satisfies the definition in Ullmann's Encyclopedia in the chapter on organic pigments.

The organic pigment may particularly be chosen from nitroso, nitro, azo, xanthene, pyrene, quinoline, anthraquinone, triphenylmethane, fluorane, phthalocyanine, metalcomplex, isoindolinone, isoindoline, quinacridone, perinone, perylene, diketopyrrolopyrrole, indigo, thioindigo, dioxazine, triphenylmethane and quinophthalone compounds.

In particular, the white or coloured organic pigments may be chosen from carmine, carbon black, aniline black, azo yellow, quinacridone, phthalocyanine blue, the blue pigments codified in the Colour Index under the references CI 42090, 69800, 69825, 74100, 74160, the yellow pigments codified in the Colour Index under the references CI 11680, 11710, 19140, 20040, 21100, 21108, 47000, 47005, the green pigments codified in the Colour Index under the references CI 61565, 61570, 74260, the orange pigments codified in the Colour Index under the references CI 11725, 45370, 71105, the red pigments codified in the Colour Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:

- Cosmenyl Yellow 10G: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may particularly be composed of particles including an inorganic core, at least one binder for attaching the organic pigments to the core, and at least one organic pigment which at least partially covers the core.

The organic pigment may also be a lake. "Lake" means dyes adsorbed onto insoluble particles, the assembly thus obtained remaining insoluble during use.

The inorganic substrates onto which the dyes are adsorbed are, for example, alumina, silica, calcium sodium borosilicate or calcium aluminium borosilicate and aluminium.

Among the dyes, mention may be made of carminic acid. Mention may also be made of the dyes known under the following names: D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

An example of a lake that may be mentioned is the product known under the following name: D&C Red 7 (CI 15 850:1).

The pigment may also be a pigment with special effects. "Pigments with special effects" means pigments that generally create a coloured appearance (characterized by a certain shade, a certain vivacity and a certain level of luminance) that is non-uniform and that changes based on the conditions of observation (light, temperature, angles of observation, etc.). They thereby differ from coloured pigments, which afford a standard uniform opaque, semi-transparent or transparent shade.

Several types of pigments with special effects exist: those with a low refractive index, such as fluorescent or photochromic pigments, and those with a higher refractive index, such as nacres, interference pigments or glitter flakes.

Examples of pigments with special effects that may be mentioned include nacreous pigments such as mica coated with titanium or with bismuth oxychloride, coloured nacreous pigments such as mica covered with titanium and with iron oxides, mica covered with iron oxide, mica covered with titanium and particularly with ferric blue or with chromium oxide, mica covered with titanium and with an organic pigment as defined previously, and also nacreous pigments based on bismuth oxychloride. Nacreous pigments that may be mentioned include the nacres Cellini sold by BASF (mica-$TiO_2$-lake), Prestige sold by Eckart (mica-$TiO_2$), Prestige Bronze sold by Eckart (mica-$Fe_2O_3$) and Colorona sold by Merck (mica-$TiO_2$-$Fe_2O_3$).

Mention may also be made of the gold-coloured nacres sold particularly by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold particularly by the company Merck under the name Bronze fine (17384) (Colorona) and Bronze (17353) (Colorona) and by the company BASF under the name Super bronze (Cloisonne); the orange nacres sold particularly by the company BASF under the name Orange 363C (Cloisonne) and Orange MCR 101 (Cosmica) and by the company Merck under the name Passion orange (Colorona) and Matte orange (17449) (Microna); the brown nacres sold particularly by the company BASF under the name Nu-antique copper 340XB (Cloisonne) and Brown CL4509 (Chromalite); the nacres with a copper tint sold particularly by the company BASF under the name Copper 340A (Timica); the nacres with a red tint sold particularly by the company Merck under the name Sienna fine (17386)

(Colorona); the nacres with a yellow tint sold particularly by the company BASF under the name Yellow (4502) (Chromalite); the red nacres with a gold tint sold particularly by the company BASF under the name Sunstone G012 (Gemtone); the pink nacres sold particularly by the company BASF under the name Tan opale G005 (Gemtone); the black nacres with a gold tint sold particularly by the company BASF under the name Nu antique bronze 240 AB (Timica), the blue nacres sold particularly by the company Merck under the name Matte blue (17433) (Microna), the white nacres with a silvery tint sold particularly by the company Merck under the name Xirona Silver, and the golden-green pink-orange nacres sold particularly by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are particularly sold under the name Metashine MC1080RY by the company Toyal.

Finally, examples of nacres that may also be mentioned include polyethylene terephthalate glitter flakes, particularly those sold by the company Meadowbrook Inventions under the name Silver 1P 0.004X0.004 (silver glitter flakes). It is also possible to envisage multilayer pigments based on synthetic substrates, such as alumina, silica, calcium sodium borosilicate, calcium aluminium borosilicate and aluminium.

The pigments with special effects may also be chosen from reflective particles, i.e. particularly from particles whose size, structure, particularly the thickness of the layer(s) of which they are made and their physical and chemical nature, and surface state, allow them to reflect incident light. This reflection may, where appropriate, have an intensity sufficient to create, at the surface of the composition or of the mixture, when it is applied to the support to be made up, highlight points that are visible to the naked eye, i.e. more luminous points that contrast with their environment by appearing to sparkle.

The reflective particles may be selected so as not to significantly alter the colouring effect generated by the colouring agents with which they are combined, and more particularly so as to optimize this effect in terms of colour rendition. They may more particularly have a yellow, pink, red, bronze, orangey, brown, gold and/or coppery colour or tint.

These particles may have varied forms and may particularly be in platelet or globular form, in particular in spherical form.

The reflective particles, regardless of their form, may or may not have a multilayer structure and, in the case of a multilayer structure, may have, for example, at least one layer of uniform thickness, particularly of a reflective material.

When the reflective particles do not have a multilayer structure, they may be composed, for example, of metal oxides, particularly titanium or iron oxides obtained synthetically.

When the reflective particles have a multilayer structure, they may include, for example, a natural or synthetic substrate, particularly a synthetic substrate at least partially coated with at least one layer of a reflective material, particularly of at least one metal or metallic material. The substrate may be made of one or more organic and/or inorganic materials.

More particularly, it may be chosen from glasses, ceramics, graphite, metal oxides, aluminas, silicas, silicates, particularly aluminosilicates and borosilicates, and synthetic mica, and mixtures thereof, this list not being limiting.

The reflective material may include a layer of metal or of a metallic material.

Reflective particles are particularly described in JP-A-09188830, JP-A-10158450, JP-A-10158541, JP-A-07258460 and JP-A-05017710.

Again as an example of reflective particles including a mineral substrate coated with a layer of metal, mention may also be made of particles including a silver-coated borosilicate substrate.

Particles with a silver-coated glass substrate, in the form of platelets, are sold under the name Microglass Metashine REFSX 2025 PS by the company Toyal. Particles with a glass substrate coated with a nickel/chromium/molybdenum alloy are sold under the names Crystal Star GF 550 and GF 2525 by this same company.

Use may also be made of particles comprising a metal substrate, such as silver, aluminium, iron, chromium, nickel, molybdenum, gold, copper, zinc, tin, magnesium, steel, bronze or titanium, said substrate being coated with at least one layer of at least one metal oxide, such as titanium oxide, aluminium oxide, iron oxide, cerium oxide, chromium oxide, silicon oxides and mixtures thereof.

Examples that may be mentioned include aluminium powder, bronze powder or copper powder coated with $SiO_2$ sold under the name Visionaire by the company Eckart.

Mention may also be made of interference pigments which are not attached to a substrate, such as liquid crystals (Helicones HC from Wacker) or interference holographic glitter flakes (Geometric Pigments or Spectra f/x from Spectratek). Special effect pigments also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colours, and also particular optical effects such as metallic effects or interference effects.

The size of the pigment used in the composition according to the present invention is generally between 10 nm

and 200 μm, preferably between 20 nm and 80 μm and more preferentially between 30 nm and 50 μm.

The pigments may be dispersed in the composition by means of a dispersant.

The dispersant serves to protect the dispersed particles against agglomeration or flocculation thereof. This dispersant may be a surfactant, an oligomer, a polymer or a mixture of several thereof, bearing one or more functionalities with strong affinity for the surface of the particles to be dispersed. In particular, they may become physically or chemically attached to the surface of the pigments. These dispersants also contain at least one functional group that is compatible with or soluble in the continuous medium. In particular, esters of 12-hydroxystearic acid in particular and of C8 to C20 fatty acid and of polyols such as glycerol or diglycerol are used, such as poly(12-hydroxystearic acid) stearate with a molecular weight of approximately 750 g/mol, such as the product sold under the name Solsperse 21 000 by the company Avecia, polyglyceryl-2 dipolyhydroxystearate (CTFA name) sold under the reference Dehymyls PGPH by the company Henkel, or else polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the composition may be surface-treated with an organic agent.

Thus, the pigments surface-treated beforehand that are useful in the context of the invention are pigments which have been completely or partially subjected to a surface treatment of chemical, electronic, electrochemical, mechanochemical or mechanical nature with an organic agent, such as those described particularly in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, for example silicones, particularly polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is particularly described in patent US 4 578 266.

An organic agent covalently bonded to the pigments will preferably be used.

The agent for the surface treatment may represent from 0.1% to 50% by weight of the total weight of the surface-treated pigment, preferably from 0.5% to 30% by weight and even more preferentially from 1% to 20% by weight of the total weight of the surface-treated pigment.

Preferably, the surface treatments of the pigments are chosen from the following treatments:

- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethylsiloxysilicate treatment, for instance the Covasil 4.05 surface treatment sold by LCW;
- a magnesium myristate treatment, for instance the MM surface treatment sold by LCW;
- an aluminium dimyristate treatment, for instance the MI surface treatment sold by Miyoshi;
- a perfluoropolymethyl isopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrogenosiloxane/perfluoroalkyl phosphate treatment, for instance the FS01 surface treatment sold by Daito;

- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

According to a particular embodiment of the invention, the dispersant is present with organic or inorganic pigments in submicron-sized particulate form in composition B.
*"Submicron-sized"* or *"submicronic"* means pigments having a particle size that has been micronized by a micronization method and having a mean particle size of less than a micrometre ($\mu$m), in particular between 0.1 and 0.9 $\mu$m, and preferably between 0.2 and 0.6 $\mu$m.
According to one embodiment, the dispersant and the pigment(s) are present in a (dispersant:pigment) amount, according to a weight ratio, of between 1: 4 and 4: 1, particularly between 1.5: 3.5 and 3.5: 1 or better still between 1.75: 3 and 3: 1.
The dispersant(s) may therefore have a silicone backbone, such as silicone polyether, and dispersants of aminosilicone type other than the aminosilicones mentioned below. Among the suitable dispersants that may be mentioned are:

- aminosilicones, i.e. silicones comprising one or more amino groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego® RC 902, Tego® RC 922, Tego® RC 1041, and Tego® RC 1043, sold by Evonik,
- polydimethylsiloxane (PDMS) silicones bearing carboxylic groups such as X-22162 and X-22370 by Shin-Etsu, epoxy silicones such as GP-29, GP-32, GP-502, GP-504, GP-514, GP-607, GP-682, and GP-695 by Genesee Polymers, or Tego® RC 1401, Tego® RC 1403, Tego® RC 1412 by Evonik.

According to one particular embodiment, the dispersant(s) are of aminosilicone type other than the aminosilicones mentioned below and are cationic.
Preferably, the pigment(s) is (are) chosen from mineral, mixed mineral-organic or organic pigments.
In one variant of the invention, the pigment(s) are organic pigments, preferentially organic pigments surface-treated with an organic agent chosen from silicone compounds. In another variant of the invention, the pigment(s) are mineral pigments.
Preferably, the pigment(s) are chosen from iron oxides, particularly red, brown or black iron oxide, nacres, particularly mica coated with titanium or with bismuth oxychloride, mica coated with titanium and iron oxides, mica coated with iron oxide, mica coated with titanium, and mixtures thereof.
Mention may be made, as an example of iron oxide, of the iron oxide sold by the company Sun Chemical under the trade name SunPuro® Red Iron Oxide.
Mention may be made, as an example of nacre, of the mica and iron oxide sold by the company Sudarshan Chemical under the trade name Pearlescent Pigment Prestige soft bronze.
According to a preferred embodiment, the dyeing composition according to the invention comprises a mixture of pigments, particularly iron oxides, and of nacres, particularly mica coated with titanium or with bismuth oxychloride, mica coated with titanium and iron oxides, mica coated with iron oxide, mica coated with titanium.

*Direct dye*

The composition for dyeing keratinous hair fibres used in the context of the process according to the invention may comprise one or more direct dye(s).
*"Direct dye"* means natural and/or synthetic dyes, other than oxidation dyes. These are dyes which will spread superficially over the fibre.
They may be ionic or nonionic, preferably cationic or nonionic.
Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.
The direct dyes are preferably cationic direct dyes. Mention may be made of the hydrazono cationic dyes of formulae (XIII) and (XIV) and the azo cationic dyes (XV) and (XVI) below:

$$Het^+\text{-}N(Ra)\text{-}N{=}C(Rb)\text{-}Ar,\ Q^- \qquad (XIII),$$

Het$^+$-C(Ra)=N-N(Rb)-Ar, Q (XIV),

Het$^+$-N=N-Ar, Q- (XV),

Ar$^+$-N=N-Ar", Q- (XVI),

in which formulae (XIII) to (XVI):

- Het+ represents a cationic heteroaryl radical, preferentially bearing an endocyclic cationic charge, such as imidazolium, indolium or pyridinium, which is optionally substituted, preferentially with at least one ($C_1$-$C_8$)alkyl group such as methyl;
- Ar+ represents an aryl radical, such as phenyl or naphthyl, bearing an exocyclic cationic charge, preferentially ammonium, particularly tri($C_1$-$C_8$)alkylammonium, such as trimethylammonium;
- Ar represents an aryl group, particularly phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted N-($C_1$-$C_8$)alkyl-N-aryl($C_1$-$C_8$)alkylamino or alternatively Ar represents a julolidine group;
- Ar" represents an optionally substituted (hetero)aryl group, such as phenyl or pyrazolyl, which are optionally substituted, preferentially with one or more ($C_1$-$C_8$)alkyl, hydroxyl, (di)($C_1$-$C_8$)(alkyl)amino, ($C_1$-$C_8$)alkoxy or phenyl groups;
- Ra and Rb, which are identical or different, represent a hydrogen atom or a ($C_1$-$C_8$)alkyl group, which is optionally substituted, preferentially with a hydroxyl group;
  or else the substituent Ra with a substituent of Het+ and/or Rb with a substituent of Ar form, together with the atoms that bear them, a (hetero)cycloalkyl; in particular, Ra and Rb represent a hydrogen atom or a ($C_1$-$C_4$)alkyl group optionally substituted with a hydroxyl group;
- Q- represents an organic or mineral anionic counterion, such as a halide or an alkyl sulfate.

In particular, mention may be made of the azo and hydrazono direct dyes bearing an endocyclic cationic charge of formulae (XIII) to (XVI) as defined previously; more particularly, the cationic direct dyes bearing an endocyclic cationic charge described in patent applications WO 95/15144, WO 95/01772 and EP 714 954; preferentially, the following direct dyes:

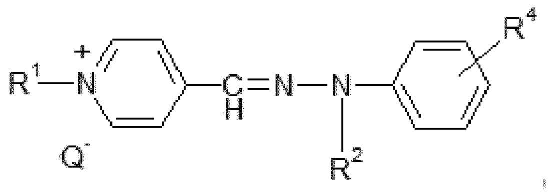

(XVII)

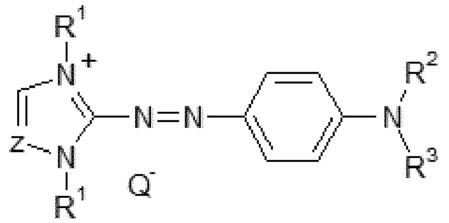

(XVIII),

in which formulae (XVII) and (XVIII):

- $R_1$ represents a ($C_1$-$C_4$)alkyl group such as methyl;
- $R^2$ and $R^3$, which are identical or different, represent a hydrogen atom or a ($C_1$-$C_4$)alkyl group, such as methyl; and
- $R_4$ represents a hydrogen atom or an electron-donating group such as optionally substituted ($C_1$-$C_8$)alkyl, optionally substituted ($C_1$-$C_8$)alkoxy, or (di)($C_1$-$C_8$)(alkyl)amino optionally substituted on the alkyl group(s) with a hydroxyl group; in particular, $R^4$ is a hydrogen atom;
- Z represents a CH group or a nitrogen atom, preferentially CH;

- Q- is an anionic counterion as defined previously, in particular a halide, such as chloride, or an alkyl sulfate, such as methyl sulfate or mesityl.

In particular, the dyes of formulae (XVII) and (XVIII) are chosen from Basic Red 51, Basic Yellow 87 and Basic Orange 31 or derivatives thereof with Q' being an anionic counterion as defined previously, particularly halide such as chloride, or an alkyl sulfate such as methyl sulfate or mesityl.

The direct dyes may be chosen from anionic direct dyes. The anionic direct dyes of the invention are dyes commonly referred to as "acid" direct dyes owing to their affinity for alkaline substances. "Anionic direct dye" means any direct dye including in its structure at least one $CO_2R$ or $SO_3R$ substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes. As acid dyes which are useful for the invention, mention may be made of the dyes of formulae (XIX), (XIX'), (XX), (XX'), (XXI), (XXI'), (XXII), (XXII'), (XXIII), (XXIV), (XXV) and (XXVI) below:

a) the diaryl anionic azo dyes of formula (XIX) or (XIX'):

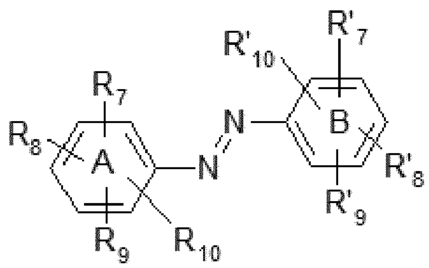

(XIX),

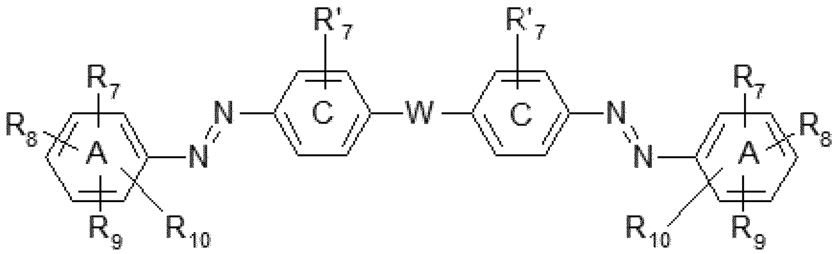

(XIX'),

in which formulae (XIX) and (XIX'):

- $R_7$, $R_8$, $R_9$, $R_{10}$, $R'_7$, $R'_8$, $R'_9$ and $R'_{10}$, which are identical or different, represent a hydrogen atom or a group chosen from:
  - alkyl;
  - alkoxy, alkylthio;
  - hydroxyl, mercapto;
  - nitro, nitroso;
  - $R^O$-C(X)-X'-, $R^O$-X'-C(X)-, R°-X'-C(X)-X''- with $R^O$ representing a hydrogen atom or an alkyl or aryl group; X, X' and X'', which are identical or different, representing an oxygen or sulfur atom, or NR with R representing a hydrogen atom or an alkyl group;
  - $(O)_2S(O-)-$, M+ with M+ representing a hydrogen atom or a cationic counterion;
  - (O)CO--, M+ with M+ as defined previously;
  - R''-$S(O)_2$-, with R'' representing a hydrogen atom or an alkyl group, an aryl, (di)(alkyl)amino or aryl(alkyl) amino group; preferentially a phenylamino or phenyl group;
  - R'''-$S(O)_2$-X'- with R''' representing an optionally substituted alkyl or aryl group, X' as defined previously;
  - (di)(alkyl)amino;
  - aryl(alkyl)amino optionally substituted with one or more groups chosen from i) nitro; ii) nitroso; iii) $(O)_2S(O-)-$, M+ and iv) alkoxy with M+ as defined previously;

- optionally substituted heteroaryl; preferentially a benzothiazolyl group;
- cycloalkyl, particularly cyclohexyl;
- Ar-N=N- with Ar representing an optionally substituted aryl group; preferentially a phenyl optionally substituted with one or more alkyl, $(O)_2S(O\text{-})$-, M+ or phenylamino groups;
- or alternatively two contiguous groups $R_7$ with $R_8$ or $R_8$ with $R_9$ or $R_9$ with $R_{10}$ together form a fused benzo group A'; and $R'_7$ with $R'_8$ or $R'_8$ with $R'_9$ or $R'_9$ with $R'_{10}$ together form a fused benzo group B'; with A' and B' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)-; ix) R°-X' -C(X)-X" -; x) Ar-N=N-and xi) optionally substituted aryl(alkyl)amino; with $M^+$, R°, X, X', X'' and Ar as defined previously;
- W represents a sigma bond $\sigma$, an oxygen or sulfur atom, or a divalent radical i) -NR-with R as defined previously, or ii) methylene -C(Ra)(Rb)- with Ra and Rb, which are identical or different, representing a hydrogen atom or an aryl group, or alternatively Ra and Rb form, together with the carbon atom that bears them, a spiro cycloalkyl; preferentially, W represents a sulfur atom or Ra and Rb together form a cyclohexyl;

it being understood that formulae (XIX) and (XIX') comprise at least one sulfonate radical $(O)_2S(O\text{-})$-, M+ or one carboxylate radical (O)CO--, M+ on one of the rings A, A', B, B' or C; preferentially sodium sulfonate.
As examples of dyes of formula (XIX), mention may be made of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food Yellow 3 or Sunset Yellow;
and, as examples of dyes of formula (XIX'), mention may be made of: Acid Red 111, Acid Red 134, Acid Yellow 38;

b) the pyrazolone anionic azo dyes of formulae (XX) and (XX'):

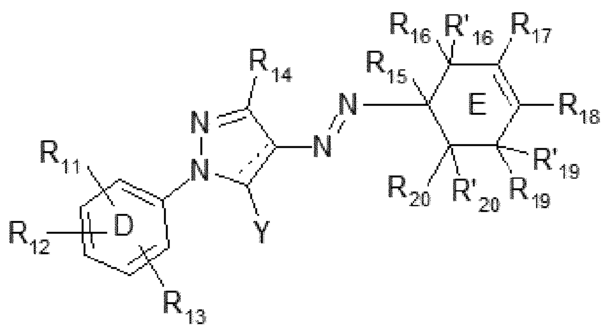

(XX),

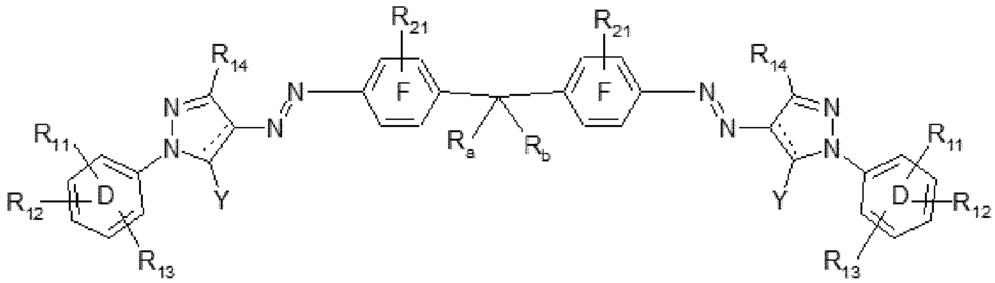

(XX'),

in which formulae (XX) and (XX'):

- $R_{11}$, $R_{12}$ and $R_{13}$, which are identical or different, represent a hydrogen or halogen atom, an alkyl group or $-(O)_2S(O\text{-})$, M+ with M+ as defined previously;
- $R_{14}$ represents a hydrogen atom, an alkyl group or a group $-C(O)O^-$, $M^+$ with $M^+$ as defined previously;

- $R_{15}$ represents a hydrogen atom;
- $R_{16}$ represents an oxo group, in which case $R'_{16}$ is absent, or alternatively $R_{15}$ with $R_{16}$ together form a double bond;
- $R_{17}$ and $R_{18}$, which are identical or different, represent a hydrogen atom, or a group chosen from:

  - $(O)_2S(O\text{-})\text{-}$, M+ with M+ as defined previously;
  - $Ar\text{-}O\text{-}S(O)_2\text{-}$ with Ar representing an optionally substituted aryl group; preferentially a phenyl optionally substituted with one or more alkyl groups;
  - $R_{19}$ and $R_{20}$ together form either a double bond, or a benzo group D', which is optionally substituted;
  - $R'_{16}$, $R'_{19}$ and $R'_{20}$, which are identical or different, represent a hydrogen atom or an alkyl or hydroxyl group;
  - $R_{21}$ represents a hydrogen atom or an alkyl or alkoxy group;
  - Ra and Rb, which are identical or different, are as defined previously; preferentially, Ra represents a hydrogen atom and Rb represents an aryl group;
  - Y represents either a hydroxyl group or an oxo group;
  - ---- represents a single bond when Y is an oxo group; and represents a double bond when Y represents a hydroxyl group;

it being understood that formulae (XX) and (XX') comprise at least one sulfonate radical $(O)_2S(O\text{-})\text{-}$, M+ or one carboxylate radical -C(O)O-, M+ on one of the rings D or E; preferentially sodium sulfonate;
As examples of dyes of formula (XX), mention may be made of: Acid Red 195, Acid Yellow 23, Acid Yellow 27, Acid Yellow 76, and, as an example of a dye of formula (XX'), mention may be made of: Acid Yellow 17;

c) the anthraquinone dyes of formulae (XXI) and (XXI'):

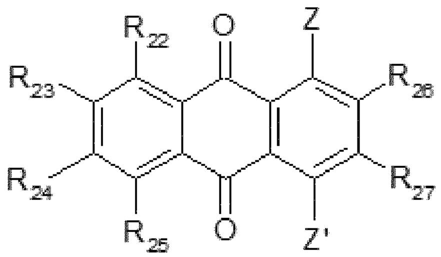

(XXI),

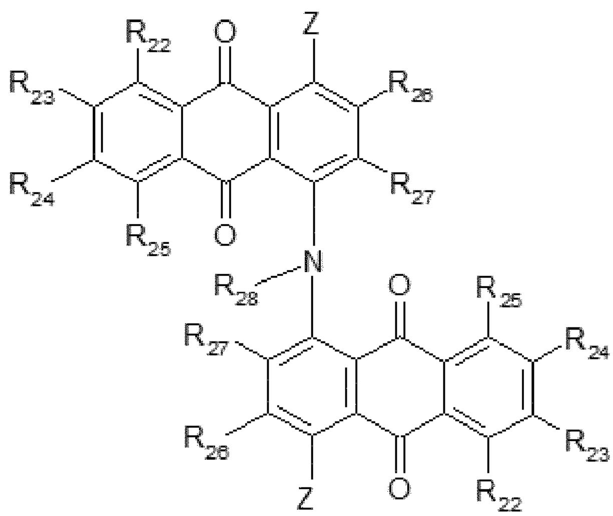

(XXI'),

in which formulae (XXI) and (XXI'):

- $R_{22}$, $R_{23}$, $R_{24}$, $R_{25}$, $R_{26}$ and $R_{27}$, which are identical or different, represent a hydrogen or halogen atom, or a

group chosen from:

- alkyl;
- hydroxyl, mercapto;
- alkoxy, alkylthio;
- optionally substituted aryloxy or arylthio, preferentially substituted with one or more groups chosen from alkyl and $(O)_2S(O^-)$-, $M^+$ with $M^+$ as defined previously;
- aryl(alkyl)amino optionally substituted with one or more groups chosen from alkyl and $(O)_2S(O-)$-, $M^+$ with $M^+$ as defined previously;
- (di)(alkyl)amino;
- (di)(hydroxyalkyl)amino;
- $(O)_2S(O-)$-, M+ with M+ as defined previously;
- Z' represents a hydrogen atom or a group $NR_{28}R_{29}$ with $R_{28}$ and $R_{29}$, which are identical or different, representing a hydrogen atom or a group chosen from:

  - alkyl;
  - polyhydroxyalkyl such as hydroxyethyl;
  - aryl optionally substituted with one or more groups, particularly i) alkyl such as methyl, n-dodecyl, n-butyl; ii) $(O)_2S(O-)$-, $M^+$ with $M^+$ as defined previously; iii) R°-C(X)-X'-, R°-X'-C(X)-, R°-X'-C(X)-X''- with R°, X, X' and X" as defined previously, preferentially R° represents an alkyl group;
  - cycloalkyl, particularly cyclohexyl;
  - Z represents a group chosen from hydroxyl and $NR'_{28}R'_{29}$ with $R'_{28}$ and $R'_{29}$, which are identical or different, representing the same atoms or groups as $R_{28}$ and $R_{29}$ as defined previously;

it being understood that formulae (XXI) and (XXI') comprise at least one sulfonate radical $(O)_2S(O-)$-, M+ or one carboxylate radical -C(O)O-, M+; preferentially sodium sulfonate; As examples of dyes of formula (XXI), mention may be made of: Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3; EXT Violet No. 2; and, as an example of a dye of formula (XXI'), mention may be made of: Acid Black 48;

d) the nitro dyes of formulae (XXII) and (XXII'):

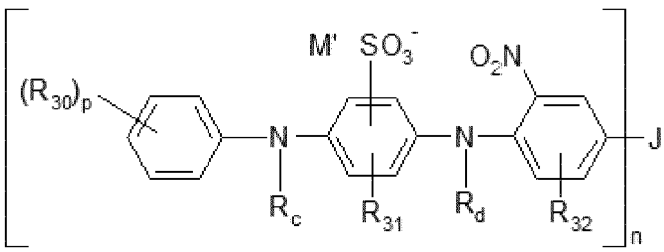

(XXII),

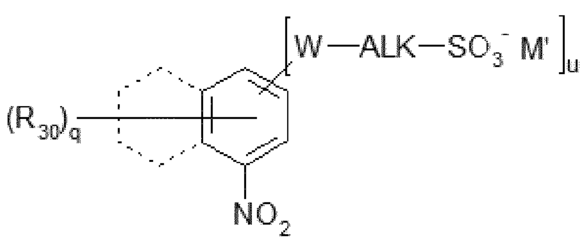

(XXII'),

in which formulae (XXII) and (XXII'):

- $R_{30}$, $R_{31}$ and $R_{32}$, which are identical or different, represent a hydrogen or halogen atom, or a group chosen from:

  - alkyl;

- alkoxy optionally substituted with one or more hydroxyl groups, alkylthio optionally substituted with one or more hydroxyl groups;
- hydroxyl, mercapto;
- nitro, nitroso;
- polyhaloalkyl;
- R°-C(X)-X'-, R°-X'-C(X)-, R°-X'-C(X)-X''- with R°, X, X' and X'' as defined previously;
- $(O)_2S(O-)$-, M+ with M+ as defined previously;
- (O)CO--, M+ with M+ as defined previously;
- (di)(alkyl)amino;
- (di)(hydroxyalkyl)amino;
- heterocycloalkyl such as piperidino, piperazino or morpholino; in particular, $R_{30}$, $R_{31}$ and $R_{32}$ represent a hydrogen atom;
- Rc and Rd, which are identical or different, represent a hydrogen atom or an alkyl group;
- W is as defined previously; W particularly represents an -NH- group;
- ALK represents a linear or branched divalent $C_1$-$C_6$ alkylene group; in particular, ALK represents a $-CH_2-CH_2-$ group;
- n is 1 or 2;
- p represents an integer inclusively between 1 and 5;
- q represents an integer inclusively between 1 and 4;
- u is 0 or 1;
- when n is 1, J represents a nitro or nitroso group; particularly nitro;
- when n is 2, J represents an oxygen or sulfur atom, or a divalent radical $-S(O)_n-$ with m representing an integer 1 or 2; preferentially, J represents an $-SO_2-$ radical;
- M' represents a hydrogen atom or a cationic counterion;

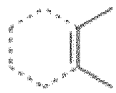,

which may be present or absent, represents a benzo group optionally substituted with one or more groups $R_{30}$ as defined previously;

it being understood that formulae (XXII) and (XXII') comprise at least one sulfonate radical $(O)_2S(O-)$-, M+ or one carboxylate radical -C(O)O-, M+; preferentially sodium sulfonate.
As examples of dyes of formula (XXII), mention may be made of: Acid Brown 13 and Acid Orange 3; as examples of dyes of formula (XXII'), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;

e) the triarylmethane dyes of formula (XXIII):

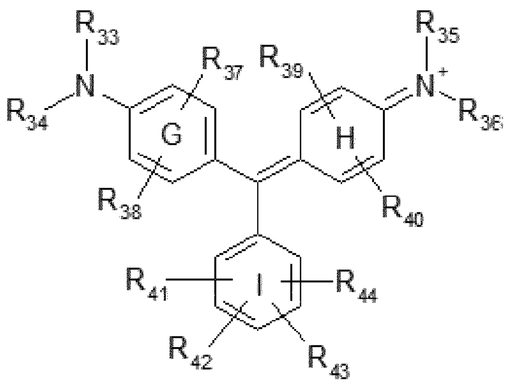

(XXIII),

in which formula (XXIII):

- $R_{33}$, $R_{34}$, $R_{35}$ and $R_{36}$, which are identical or different, represent a hydrogen atom or a group chosen from alkyl, optionally substituted aryl and optionally substituted arylalkyl; particularly an alkyl and benzyl group optionally substituted with a group (O)mS(O-)-, M+ with M+ and m as defined previously;
- $R_{37}$, $R_{38}$, $R_{39}$, $R_{40}$, $R_{41}$, $R_{42}$, $R_{43}$ and $R_{44}$, which are identical or different, represent a hydrogen atom or a group chosen from:

  - alkyl;
  - alkoxy, alkylthio;
  - (di)(alkyl)amino;
  - hydroxyl, mercapto;
  - nitro, nitroso;
  - R°-C(X)-X'-, R°-X'-C(X)-, R°-X'-C(X)-X''- with R° representing a hydrogen atom or an alkyl or aryl group; X, X' and X'', which are identical or different, representing an oxygen or sulfur atom, or NR with R representing a hydrogen atom or an alkyl group;
  - $(O)_2S(O-)$-, M+ with M+ representing a hydrogen atom or a cationic counterion;
  - (O)CO--, M+ with M+ as defined previously;
  - or alternatively two contiguous groups $R_{41}$ with $R_{42}$ or $R_{42}$ with $R_{43}$ or $R_{43}$ with $R_{44}$ together form a fused benzo group: I'; with I' optionally substituted by one or more groups chosen from i) nitro; ii) nitroso; iii) $(O)_2S(O-)$-, M+; iv) hydroxyl; v) mercapto; vi) (di)(alkyl)amino; vii) R°-C(X)-X'-; viii) R°-X'-C(X)-; ix) R°-X'-C(X)-X"-; with M+, R°, X, X', X" as defined above; in particular, $R_{37}$ to $R_{40}$ represent a hydrogen atom and $R_{41}$ to $R_{44}$, which are identical or different, represent a hydroxyl or $(O)_2S(O-)$-, M+ group; and, when $R_{43}$ with $R_{44}$ together form a benzo group, it is preferentially substituted by an $(O)_2S(O-)$- group;

it being understood that at least one of the rings G, H, I or I' comprises at least one sulfonate radical $(O)_2S(O-)$- or one carboxylate radical -C(O)O-; preferentially sulfonate.

As examples of dyes of formula (XXIII), mention may be made of: Acid Blue 1; Acid Blue 3; Acid Blue 7, Acid Blue 9; Acid Violet 49; Acid Green 3; Acid Green 5 and Acid Green 50.

f) the xanthene-based dyes of formula (XXIV):

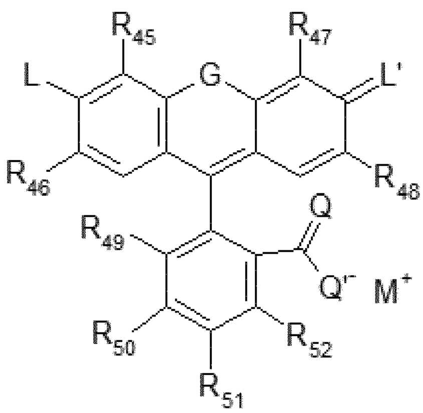

(XXIV),

in which formula (XXIV):

- $R_{45}$, $R_{46}$, $R_{47}$ and $R_{48}$, which are identical or different, represent a hydrogen or halogen atom;
- $R_{49}$, $R_{50}$, $R_{51}$ and $R_{52}$, which are identical or different, represent a hydrogen or halogen atom, or a group chosen from:

  - alkyl;
  - alkoxy, alkylthio;
  - hydroxyl, mercapto;
  - nitro, nitroso;
  - $(O)_2S(O-)$-, M+ with M+ representing a hydrogen atom or a cationic counterion;
  - (O)CO--, M+ with M+ as defined previously;
  
  particularly, $R_{49}$, $R_{50}$, $R_{51}$ and $R_{52}$ represent a hydrogen or halogen atom;

- G represents an oxygen or sulfur atom or a group NRe with Re as defined previously; particularly, G represents an oxygen atom;
- L represents an alkoxide $O^-$, $M^+$; a thioalkoxide $S^-$, $M^+$ or a group NRf, with Rf representing a hydrogen atom or an alkyl group, and $M^+$ as defined above; $M^+$ is particularly sodium or potassium;
- L' represents an oxygen or sulfur atom or an ammonium group: N+RfRg, with Rf and Rg, which are identical or different, representing a hydrogen atom or an optionally substituted alkyl or aryl group; L' particularly represents an oxygen atom or a phenylamino group optionally substituted with one or more alkyl or (O)mS(O-)-, M+ groups with m and M+ as defined previously;
- Q and Q', which are identical or different, represent an oxygen or sulfur atom; particularly, Q and Q' represent an oxygen atom;
- $M^+$ is as defined previously.

As examples of dyes of formula (XXIV), mention may be made of: Acid Yellow 73; Acid Red 51; Acid Red 52; Acid Red 87; Acid Red 92; Acid Red 95; Acid Violet 9;

g) the indole-based dyes of formula (XXV):

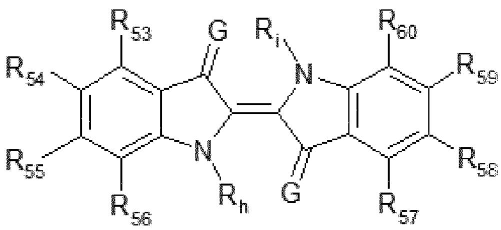

(XXV),

in which formula (XXV):

- $R_{53}$, $R_{54}$, $R_{55}$, $R_{56}$, $R_{57}$, $R_{58}$, $R_{59}$ and $R_{60}$, which are identical or different, represent a hydrogen atom or a group chosen from:

  - alkyl;
  - alkoxy, alkylthio;
  - hydroxyl, mercapto;
  - nitro, nitroso;
  - R°-C(X)-X'-, R°-X'-C(X)-, R°-X'-C(X)-X''- with R° representing a hydrogen atom or an alkyl or aryl group; X, X' and X'', which are identical or different, representing an oxygen or sulfur atom, or NR with R representing a hydrogen atom or an alkyl group;
  - $(O)_2S(O-)$-, M+ with M+ representing a hydrogen atom or a cationic counterion;
  - (O)CO--, M+ with M+ as defined previously;
  - G represents an oxygen or sulfur atom or a group NRe with Re as defined previously; particularly, G represents an oxygen atom;
  - Ri and Rh, which are identical or different, represent a hydrogen atom or an alkyl group; it being understood that formula (XXV) comprises at least one sulfonate radical (O)2S(O-)-, M+ or one carboxylate radical -C(O)O-, M+; preferentially sodium sulfonate.

As an example of dyes of formula (XXV), mention may be made of: Acid Blue 74;

h) the quinoline-based dyes of formula (XXVI):

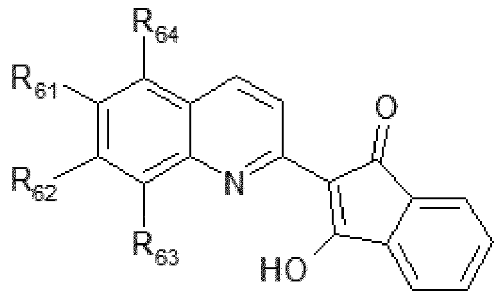

(XXVI),

in which formula (XXVI):

- $R_{61}$ represents a hydrogen or halogen atom or an alkyl group;
- $R_{62}$, $R_{63}$ and $R_{64}$, which are identical or different, represent a hydrogen atom or a group $(O)_2S(O-)-$, M+ with M+ representing a hydrogen atom or a cationic counterion;

or alternatively $R_{61}$ with $R_{62}$, or $R_{61}$ with $R_{64}$, together form a benzo group optionally substituted with one or more groups $(O)_2S(O-)-$, M+ with M+ representing a hydrogen atom or a cationic counterion;
it being understood that formula (XXVI) comprises at least one sulfonate radical $(O)_2S(O-)-$, M+, preferentially sodium sulfonate.

As examples of dyes of formula (XXVI), mention may be made of: Acid Yellow 2, Acid Yellow 3 and Acid Yellow 5.
Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions containing these natural dyes and particularly henna-based poultices or extracts.
Preferably, the direct dyes are chosen from anionic direct dyes.
The colouring agent(s) may be present in a total amount ranging from 0.001% to 20% by weight and preferably from 0.005% to 15% by weight relative to the total weight of the dyeing composition; preferably, the colouring agent(s) are chosen from pigments.
The pigment(s) may be present in a total amount ranging from 0.05% to 20% by weight, preferably from 0.1% to 15% by weight and better still from 0.5% to 10% by weight, relative to the total weight of the dyeing composition.
The direct dye(s) can be present in a total amount ranging from 0.001% to 10% by weight of the total weight of the composition, preferably from 0.005% to 5% by weight relative to the total weight of the dyeing composition.

**Non-carboxylic anionic thickener**

The composition for dyeing keratinous hair fibres may also comprise a non-carboxylic anionic thickener.
For the purposes of the present invention, "non-carboxylic agent" means an agent which does not comprise any carboxylic acid functions (-COOH) or carboxylate functions ($-COO^-$).
For the purposes of the present invention, "thickener" means a compound which increases the viscosity of a composition into which it is introduced at a concentration of 0.05% by weight relative to the total weight of the composition, by at least 20 cps, preferably by at least 50 cps, at room temperature (25°C), at atmospheric pressure and at a shear rate of 1 $s^{-1}$ (the viscosity may be measured using a cone/plate viscometer, a Haake R600 rheometer or the like).
Preferably, the non-carboxylic anionic thickener(s) are chosen from non-carboxylic anionic polymers, more preferentially from anionic polymers bearing a sulfonic group or groups.
For the purposes of the invention, "anionic polymer" means a polymer comprising one or more anionic or anionizable groups, and not comprising any cationic or cationizable groups.
Advantageously, the non-carboxylic anionic thickener(s) are chosen from anionic polymers including at least one ethylenically unsaturated monomer bearing a sulfonic group, in free form or partially or totally neutralized form.
These polymers may be crosslinked or non-crosslinked. They are preferably crosslinked. These polymers may be associative or non-associative, preferably non-associative.
It is recalled that "associative polymers" are polymers that are capable, in an aqueous medium, of reversibly associating with each other or with other molecules.
Their chemical structure more particularly comprises at least one hydrophilic zone and at least one hydrophobic zone.

"Hydrophobic group" means a radical or polymer with a saturated or unsaturated, linear or branched hydrocarbon-based chain, comprising at least 8 carbon atoms, preferably from 10 to 30 carbon atoms, in particular from 12 to 30 carbon atoms and more preferentially from 18 to 30 carbon atoms.

Preferentially, the hydrocarbon-based group originates from a monofunctional compound. By way of example, the hydrophobic group may be derived from a fatty alcohol such as stearyl alcohol, dodecyl alcohol or decyl alcohol. It may also denote a hydrocarbon-based polymer, for instance polybutadiene.

The ethylenically unsaturated monomers bearing a sulfonic group are particularly chosen from vinylsulfonic acid, styrenesulfonic acid, (meth)acrylamido($C_1$-$C_{22}$)alkylsulfonic acids, N-($C_1$-$C_{22}$)alkyl(meth)acrylamido($C_1$-$C_{22}$)alkylsulfonic acids such as undecylacrylamidomethanesulfonic acid, and also partially or totally neutralized forms thereof.

More preferentially, use will be made of (meth)acrylamido($C_1$-$C_{22}$)alkylsulfonic acids, for example acrylamidomethanesulfonic acid, acrylamidoethanesulfonic acid, acrylamidopropanesulfonic acid, 2-acrylamido-2-methylpropanesulfonic acid, methacrylamido-2-methylpropanesulfonic acid, 2-acrylamido-n-butanesulfonic acid, 2-acrylamido-2,4,4-trimethylpentanesulfonic acid, 2-methacrylamidododecylsulfonic acid or 2-acrylamido-2,6-dimethyl-3-heptanesulfonic acid, and also their partially or completely neutralized forms.

2-Acrylamido-2-methylpropanesulfonic acid (AMPS), and also partially or totally neutralized forms thereof, will more particularly be used.

Among the 2-acrylamido-2-methylpropanesulfonic acid copolymers, mention may be made of partially or totally neutralized crosslinked copolymers of 2-acrylamido-2-methylpropanesulfonic acid and of acrylamide; mention may be made in particular of the product described in Example 1 of EP 503 853, and reference may be made to said document as regards these polymers.

Mention may also be made of copolymers of 2-acrylamido-2-methylpropanesulfonic acid or salts thereof and of hydroxyethyl acrylate, such as the compound sold under the name Sepinov EMT 10 by the company SEPPIC (INCI name: hydroxyethylacrylate/sodium acryloyldimethyl taurate copolymer).

The associative AMPS polymers may particularly be chosen from random associative AMPS polymers modified by reaction with a $C_6$-$C_{22}$ n-monoalkylamine or di-n-alkylamine, and such as those described in patent application WO 00/31154 (forming an integral part of the content of the description). These polymers may also contain other ethylenically unsaturated hydrophilic monomers chosen, for example, from (meth)acrylic acid derivatives, such as esters thereof obtained with monoalcohols or mono- or polyalkylene glycols, (meth)acrylamides, vinylpyrrolidone, or mixtures of these compounds.

The preferred polymers of this family are chosen from associative copolymers of AMPS and of at least one ethylenically unsaturated hydrophobic monomer.

These same copolymers may also contain one or more ethylenically unsaturated monomers not including a fatty chain, such as (meth)acrylic acid derivatives, particularly esters thereof obtained with monoalcohols or mono- or polyalkylene glycols, (meth)acrylamides, vinylpyrrolidone, or mixtures of these compounds.

These copolymers are described particularly in patent application EP-A 750 899, patent US 5 089 578 and in the following publications from Yotaro Morishima:

- Self-assembling amphiphilic polyelectrolytes and their nanostructures, Chinese Journal of Polymer Science, Vol. 18, No. 40, (2000), 323-336;
- Micelle formation of random copolymers of sodium 2-(acrylamido)-2-methylpropanesulfonate and a non-ionic surfactant macromonomer in water as studied by fluorescence and dynamic light scattering, Macromolecules, Vol. 33, No. 10, (2000), 3694-3704;
- Solution properties of micelle networks formed by non-ionic moieties covalently bound to an polyelectrolyte: salt effects on rheological behavior - Langmuir, Vol. 16, No. 12, (2000) 5324-5332;
- Stimuli responsive amphiphilic copolymers of sodium 2-(acrylamido)-2-methylpropanesulfonate and associative macromonomers, Polym. Preprint, Div. Polym. Chem., 40(2), (1999), 220-221.

Among these polymers, mention may be made of:

- crosslinked or non-crosslinked, neutralized or non-neutralized copolymers, including from 15% to 60% by weight of AMPS units and from 40% to 85% by weight of ($C_8$-$C_{16}$)alkyl(meth)acrylamide or ($C_8$-$C_{16}$)alkyl(meth)acrylate units relative to the polymer, such as those described in patent application EP-A750 899;
- terpolymers including from 10 mol% to 90 mol% of acrylamide units, from 0.1 mol% to 10 mol% of AMPS units and from 5 mol% to 80 mol% of n-($C_6$-$C_{18}$)alkylacrylamide units, such as those described in patent US-5 089 578.

Mention may also be made of copolymers of totally neutralized AMPS and of dodecyl methacrylate, and also crosslinked and non-crosslinked copolymers of AMPS and of n-dodecylmethacrylamide, such as those described in the Morishima articles mentioned above.

Preferably, the non-carboxylic anionic thickener(s) are chosen from sodium 2-acrylamido-2-methylpropanesulfonate/hydroxyethyl acrylate copolymer, sold by the company SEPPIC (INCI name: hydroxyethyl acrylate/sodium acryloyldimethyl taurate copolymer).

Advantageously, the total amount of the non-carboxylic anionic thickener(s) can range from 0.01% to 20% by weight, preferably from 0.1% to 10% by weight, better still from 0.1% to 5% by weight, and even better still from 0.1% to 3% by weight, relative to the total weight of the dyeing composition.

**Silicone**

The composition for dyeing keratinous hair fibres may also comprise at least one silicone.

The silicone(s) are other than the compound(s) having at least one carboxylic acid group as defined hereinbelow.

Preferably, the dyeing composition comprises at least one silicone chosen from nonaminosilicones, aminosilicones and mixtures thereof.

The silicones may be solid or liquid at 25°C and atmospheric pressure ($1.013\times10^5$ Pa), and volatile or non-volatile.

The silicones that may be used may be soluble or insoluble in the composition according to the invention; they may be in the form of oil, wax, resin or gum; silicone oils are preferred.

Silicones are particularly described in detail in Walter Noll's "Chemistry and Technology of Silicones" (1968), Academic Press.

Preferably, the dyeing composition contains one or more silicones that are liquid at 25°C and atmospheric pressure ($1.013\times10^5$ Pa).

The volatile silicones may be chosen from those with a boiling point of between 60°C and 260°C (at atmospheric pressure) and more particularly from:

i) cyclic polydialkylsiloxanes including from 3 to 7 and preferably 4 to 5 silicon atoms, such as

- octamethylcyclotetrasiloxane and decamethylcyclopentasiloxane.
  Mention may be made of the products sold under the name Volatile Silicone 7207 by Union Carbide or Silbione 70045 V 2 by Rhodia, Volatile Silicone 7158 by Union Carbide or Silbione 70045 V 5 by Rhodia;
- cyclocopolymers of the dimethylsiloxane/methylalkylsiloxane type having the chemical structure:
  with D:

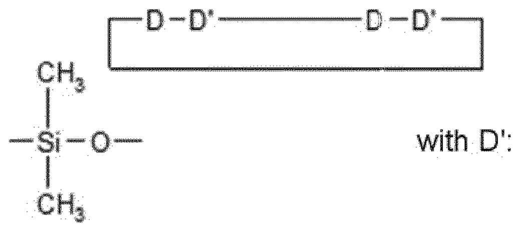

with D':

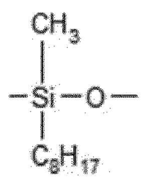

Preferably cyclomethylsiloxane.

Mention may be made of Volatile Silicone FZ 3109 sold by Union Carbide.

- mixtures of cyclic silicones with silicon-derived organic compounds, such as the mixture of octamethylcyclotetrasiloxane and of tetratrimethylsilylpentaerythritol (50/50) and the mixture of octamethylcyclotetrasiloxane and of 1,1'-oxy(2,2,2',2',3,3'-hexatrimethylsilyloxy)bisneopentane;

ii) linear polydialkylsiloxanes having 2 to 9 silicon atoms, which generally have a viscosity of less than or equal to $5\times10^{-6}$ $m^2/s$ at 25°C, such as decamethyltetrasiloxane.

Other silicones belonging to this category are described in the article published in Cosmetics and Toiletries, Vol. 91, Jan. 76, pages 27-32 - Todd & Byers Volatile silicone fluids for cosmetics; mention may be made of the product sold under the name SH 200 by the company Toray Silicone.

Among the non-volatile silicones, mention may be made, alone or as a mixture, of polydialkylsiloxanes and particularly polydimethylsiloxanes (PDMS), polydiarylsiloxanes, polyalkylarylsiloxanes, silicone gums and resins, and also organopolysiloxanes (or organomodified polysiloxanes, or alternatively organomodified silicones) which are polysiloxanes including in their structure one or more organofunctional groups, generally attached via a hydrocarbon group, and preferably chosen from aryl groups, amine groups, alkoxy groups and polyoxyethylene or polyoxypropylene groups. Preferably, the non-volatile silicones are chosen from polydimethyl/methylsiloxanes which are optionally oxyethylenated

and oxypropylenated. The organomodified silicones may be polydiarylsiloxanes, particularly polydiphenylsiloxanes, and polyalkylarylsiloxanes functionalized with the organofunctional groups mentioned previously. The polyalkylarylsiloxanes are particularly chosen from linear and/or branched polydimethyl/methylphenylsiloxanes and polydimethyl/diphenylsiloxanes.

Among the organomodified silicones, mention may be made of organopolysiloxanes including:

- polyoxyethylene and/or polyoxypropylene groups optionally including $C_6$-$C_{24}$ alkyl groups, such as dimethicone copolyols, and particularly those sold by the company Dow Corning under the name DC 1248 or the oils Silwet® L 722, L 7500, L 77 and L 711 from the company Union Carbide; or alternatively (C12)alkylmethicone copolyols, and particularly those sold by the company Dow Corning under the name Q2-5200;
- substituted or unsubstituted amine groups, in particular $C_1$-$C_4$ aminoalkyl groups; mention may be made of the products sold under the names GP4 Silicone Fluid and GP7100 by the company Genesee, or under the names Q2-8220 and DC929 or DC939 by the company Dow Corning;
- thiol groups, such as the products sold under the names GP 72 A and GP 71 from Genesee;
- alkoxylated groups, such as the product sold under the name Silicone Copolymer F-755 by SWS Silicones and Abil Wax® 2428, 2434 and 2440 by Goldschmidt;
- hydroxylated groups, such as polyorganosiloxanes bearing a hydroxyalkyl function;
- acyloxyalkyl groups, such as the polyorganosiloxanes described in patent US-A-4 957 732;
- anionic groups of the carboxylic acid type, as described, for example, in EP 186 507, or of the alkylcarboxylic type, such as the product X-22-3701E from the company Shin-Etsu; or alternatively of the 2-hydroxyalkylsulfonate or 2-hydroxyalkylthiosulfate type, such as the products sold by the company Goldschmidt under the names Abil® S201 and Abil® S255;
- hydroxyacylamino groups, such as the polyorganosiloxanes described in patent application EP 342 834; mention may be made, for example, of the product Q2-8413 from Dow Corning.

The silicones may also be chosen from polydialkylsiloxanes, among which mention may be made mainly of polydimethylsiloxanes bearing trimethylsilyl end groups. Among these polydialkylsiloxanes, mention may be made of the following commercial products:

- the Silbione® oils of the 47 and 70 047 series or the Mirasil® oils sold by Rhodia, for instance the oil 70 047 V 500 000;
- the oils of the Mirasil® series sold by the company Rhodia;
- the oils of the 200 series from the company Dow Corning, such as DC200 with a viscosity of 60 000 $mm^2/s$;
- the Viscasil® oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

In this category of polydialkylsiloxanes, mention may also be made of the products sold under the names Abil Wax® 9800 and 9801 by the company Goldschmidt, which are poly(C1-C20)dialkylsiloxanes.

Products that may be used more particularly in accordance with the invention are mixtures such as:

- mixtures formed from a polydimethylsiloxane with a hydroxy-terminated chain, or dimethiconol (CTFA), and from a cyclic polydimethylsiloxane, also known as cyclomethicone (CTFA), such as the product Q2-1401 sold by the company Dow Corning,
- mixtures formed from a polydimethylsiloxane with a hydroxy-terminated chain, or dimethiconol (CTFA), and from a polydimethylsiloxane, also known as dimethicone (CTFA), such as the product Xiameter® PMX-1503 Fluid sold by the company Dow Corning.

The polyalkylarylsiloxanes are particularly chosen from linear and/or branched polydimethyl/methylphenylsiloxanes and polydimethyl/diphenylsiloxanes with a viscosity ranging from $1\times10^{-5}$ to $5\times10^{-2}$ $m^2/s$ at 25°C.

Among these polyalkylarylsiloxanes, mention may be made of the products sold under the following names:

- the Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Preferably, the dyeing composition comprises at least one aminosilicone. "Aminosilicone" means any silicone including at least one primary, secondary, tertiary amine or a quaternary ammonium group.

The weight-average molecular masses of these aminosilicones may be measured by gel permeation chromatography (GPC) at room temperature (25°C), as polystyrene equivalent. The columns used are μ styragel columns. The eluent is THF and the flow rate is 1 ml/min. 200 μl of a 0.5% by weight solution of silicone in THF are injected. Detection is performed by refractometry and UV-metry.

Preferably, the aminosilicone(s) that may be used in the context of the invention are chosen from:

a) the polysiloxanes corresponding to formula (A):

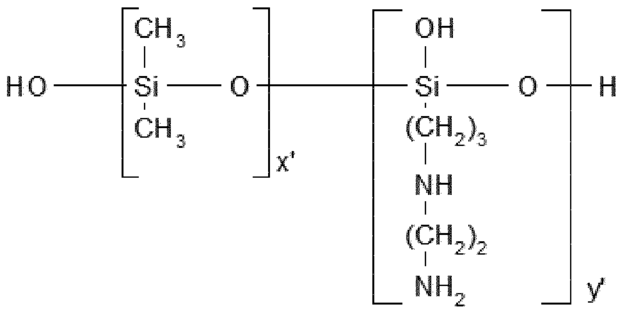

(A)

in which x' and y' are integers such that the weight-average molecular weight (Mw) is between 5000 and 500 000 approximately;

b) the aminosilicones corresponding to formula (B):

$$R'_aG_{3-a}\text{-}Si(OSiG_2)_n\text{-}(OSiG_dR'_{2-b})_m\text{-}O\text{-}SiG_{3-a}\text{-}R'_a \qquad (B)$$

in which:

- G, which is identical or different, denotes a hydrogen atom or a group from among phenyl, OH, $C_1$-$C_a$ alkyl, for example methyl, or $C_1$-$C_8$ alkoxy, for example methoxy;
- a, which is identical or different, denotes 0 or an integer from 1 to 3, in particular 0,
- b denotes 0 or 1, in particular 1,
- m and n are numbers such that the sum (n + m) ranges from 1 to 2000 and in particular from 50 to 150, it being possible for n to denote a number from 0 to 1999 and particularly from 49 to 149, and it being possible for m to denote a number from 1 to 2000 and particularly from 1 to 10;
- R', which is identical or different, denotes a monovalent radical of formula -CqH2qL in which q is a number ranging from 2 to 8 and L is an amine group, optionally quaternized, chosen from the following groups:

  - $N(R'')_2$; $-N+(R'')_3$ A-; $-NR''-Q-N(R'')_2$ and $-NR''-Q-N+(R'')_3$ A-,

  in which R", which is identical or different, denotes hydrogen, phenyl, benzyl, or a saturated monovalent hydrocarbon-based radical, for example a $C_1$-$C_{20}$ alkyl radical; Q denotes a linear or branched group of formula $C_rH_{2r}$, r being an integer ranging from 2 to 6, preferably from 2 to 4; and $A^-$ represents a cosmetically acceptable anion, particularly a halide such as fluoride, chloride, bromide or iodide.

Preferably, the aminosilicone(s) are chosen from the aminosilicones of formula (B). Preferably, the aminosilicones of formula (B) are chosen from the aminosilicones corresponding to formulae (C), (D), (E), (F) and/or (G) below.

According to a first embodiment, the aminosilicones corresponding to formula (B) are chosen from the silicones known as "trimethylsilyl amodimethicone" corresponding to formula (C):

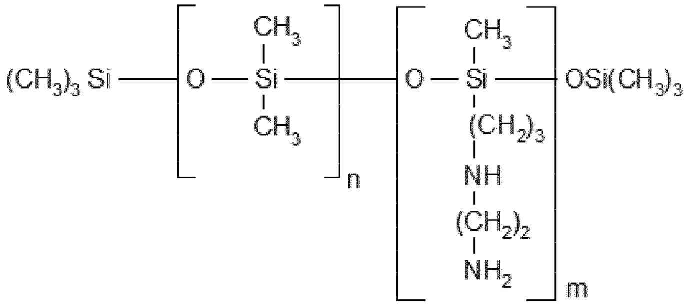

(C)

in which m and n are numbers such that the sum (n + m) ranges from 1 to 2000 and in particular from 50 to 150, it being possible for n to denote a number from 0 to 1999 and particularly from 49 to 149, and it being possible for m to denote a number from 1 to 2000 and particularly from 1 to 10.

According to a second embodiment, the aminosilicones corresponding to formula (B) are chosen from the silicones of formula (D) below:

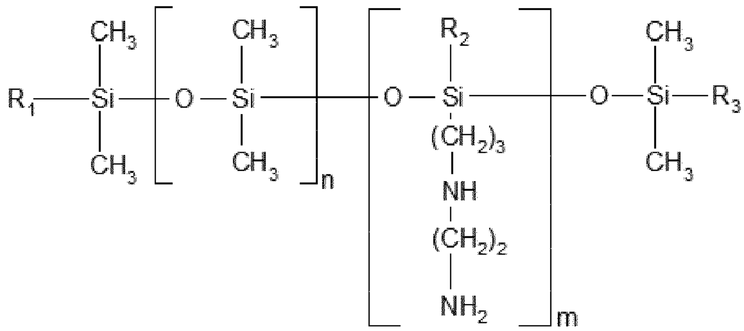

(D)

in which:

- m and n are numbers such that the sum (n + m) ranges from 1 to 1000, in particular from 50 to 250 and more particularly from 100 to 200; it being possible for n to denote a number from 0 to 999, particularly from 49 to 249 and more particularly from 125 to 175, and it being possible for m to denote a number from 1 to 1000, particularly from 1 to 10 and more particularly from 1 to 5;
- $R_1$, $R_2$ and $R_3$, which are identical or different, represent a hydroxyl or $C_1$-$C_4$ alkoxy radical, at least one of the radicals $R_1$ to $R_3$ denoting an alkoxy radical.

Preferably, the alkoxy radical is a methoxy radical.

The hydroxy/alkoxy mole ratio preferably ranges from 0.2:1 to 0.4:1 and preferably from 0.25:1 to 0.35:1 and more particularly is equal to 0.3:1.

The weight-average molecular mass (Mw) of these silicones preferably ranges from 2000 to 1 000 000 and more particularly from 3500 to 200 000.

According to a third embodiment, the aminosilicones corresponding to formula (B) are chosen from the silicones of formula (E) below:

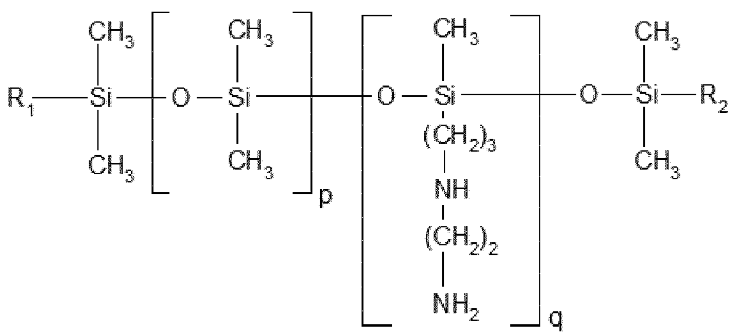

(E)

in which:

- p and q are numbers such that the sum (p + q) ranges from 1 to 1000, in particular from 50 to 350 and more particularly from 150 to 250; it being possible for p to denote a number from 0 to 999 and particularly from 49 to 349 and more particularly from 159 to 239, and it being possible for q to denote a number from 1 to 1000, particularly from 1 to 10 and more particularly from 1 to 5;
- $R_1$ and $R_2$, which are different, represent a hydroxyl or $C_1$-$C_4$ alkoxy radical, at least one of the radicals $R_1$ or $R_2$ denoting an alkoxy radical.

Preferably, the alkoxy radical is a methoxy radical.

The hydroxy/alkoxy mole ratio generally ranges from 1:0.8 to 1:1.1 and preferably from 1:0.9 to 1:1 and more particularly is equal to 1:0.95.

The weight-average molecular mass (Mw) of the silicone preferably ranges from 2000 to 200 000, even more particularly from 5000 to 100 000 and more particularly from 10 000 to 50 000.

The commercial products comprising silicones of structure (D) or (E) may include in their composition one or more other aminosilicones the structure of which is other than formula (D) or (E).

A product containing aminosilicones of structure (D) is sold by the company Wacker under the name Belsil® ADM 652.

A product containing aminosilicones of structure (E) is sold by Wacker under the name Fluid WR 1300® or under the name Belsil® ADM LOG 1.

When these aminosilicones are used, one particularly advantageous embodiment consists in using them in the form of an oil-in-water emulsion. The oil-in-water emulsion may comprise one or more surfactants. The surfactants may be of any nature but are preferably cationic and/or non-ionic. The number-average size of the silicone particles in the emulsion generally ranges from 3 nm to 500 nm. Preferably, particularly as aminosilicones of formula (E), use is made of microemulsions with a mean particle size ranging from 5 nm to 60 nm (limits included) and more particularly from 10 nm to 50 nm (limits included). Thus, use may be made according to the invention of the aminosilicone microemulsions of formula (E) sold under the names Finish CT 96 E® or SLM 28020® by the company Wacker.

According to a fourth embodiment, the aminosilicones corresponding to formula (B) are chosen from the silicones of formula (F) below:

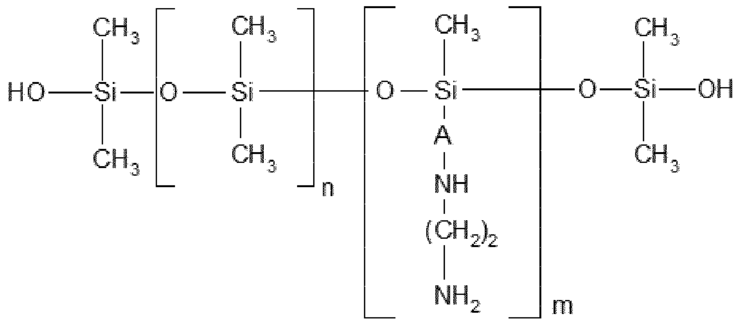

(F)

in which:

- m and n are numbers such that the sum (n + m) ranges from 1 to 2000 and in particular from 50 to 150, it being possible for n to denote a number from 0 to 1999 and particularly from 49 to 149, and it being possible for m to denote a number from 1 to 2000 and particularly from 1 to 10;
- A denotes a linear or branched alkylene radical having from 4 to 8 carbon atoms and preferably 4 carbon atoms. This radical is preferably linear.

The weight-average molecular mass (Mw) of these aminosilicones preferably ranges from 2000 to 1 000 000 and even more particularly from 3500 to 200 000.

Another silicone corresponding to formula (B) is, for example, the Xiameter MEM 8299 Emulsion from Dow Corning (INCI name: amodimethicone and trideceth-6 and cetrimonium chloride).

According to a fifth embodiment, the aminosilicones corresponding to formula (B) are chosen from the silicones of formula (G) below:

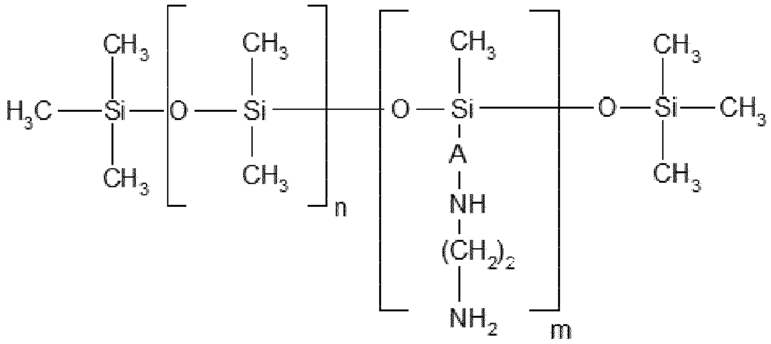

(G)

in which:

- m and n are numbers such that the sum (n + m) ranges from 1 to 2000 and in particular from 50 to 150, it being possible for n to denote a number from 0 to 1,999 and particularly from 49 to 149, and it being possible for m to denote a number from 1 to 2000 and particularly from 1 to 10;
- A denotes a linear or branched alkylene radical having from 4 to 8 carbon atoms and preferably 4 carbon atoms. This radical is preferably branched.

The weight-average molecular mass (Mw) of these aminosilicones preferably ranges from 500 to 1 000 000 and even more particularly from 1000 to 200 000.
A silicone corresponding to this formula is, for example, DC2-8566 Amino Fluid from Dow Corning;
c) the aminosilicones corresponding to formula (H):

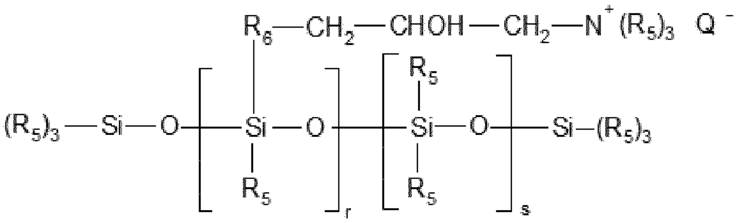

(H)

in which:

- $R_5$ represents a monovalent hydrocarbon-based radical having from 1 to 18 carbon atoms, and in particular a $C_1$-$C_{18}$ alkyl or $C_2$-$C_{18}$ alkenyl radical, for example methyl;
- $R_6$ represents a divalent hydrocarbon-based radical, particularly a $C_1$-$C_{18}$ alkylene radical or a divalent $C_1$-$C_{18}$, for example $C_1$-$C_8$, alkyleneoxy radical linked to the Si via an SiC bond;
- $Q^-$ is an anion, such as a halide ion, in particular a chloride ion, or an organic acid salt, in particular an acetate;
- r represents a mean statistical value ranging from 2 to 20 and in particular from 2 to 8;
- s represents a mean statistical value ranging from 20 to 200 and in particular from 20 to 50.

Such aminosilicones are particularly described in patent US 4 185 087.
d) the quaternary ammonium silicones of formula (I):

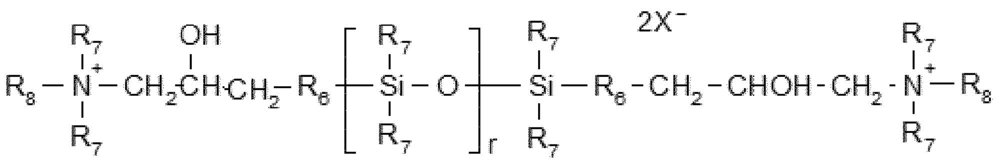

(I)

in which:

- $R_7$, which are identical or different, represent a monovalent hydrocarbon-based radical having from 1 to 18 carbon atoms, and in particular a $C_1$-$C_{18}$ alkyl radical, a $C_2$-$C_{18}$ alkenyl radical or a ring comprising 5 or 6 carbon atoms, for example methyl;
- $R_6$ represents a divalent hydrocarbon-based radical, particularly a $C_1$-$C_{18}$ alkylene radical or a divalent $C_1$-$C_{18}$, for

example $C_1$-$C_8$, alkyleneoxy radical linked to the Si via an SiC bond;

- $R_8$, which are identical or different, represent a hydrogen atom, a monovalent hydrocarbon-based radical having from 1 to 18 carbon atoms, and in particular a $C_1$-$C_{18}$ alkyl radical, a $C_2$-$C_{18}$ alkenyl radical or a -$R_6$-NHCOR$_7$ radical;
- $X^-$ is an anion such as a halide ion, particularly chloride, or an organic acid salt, particularly acetate;
- r represents a mean statistical value ranging from 2 to 200 and in particular from 5 to 100.

These silicones are described, for example, in patent EP-A 0 530 974;

e) the aminosilicones of formula (1):

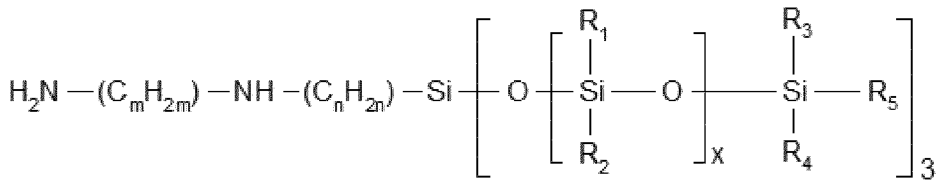

(J)

in which:

- $R_1$, $R_2$, $R_3$ and $R_4$, which are identical or different, denote a $C_1$-$C_4$ alkyl radical or a phenyl group,
- $R_5$ denotes a $C_1$-$C_4$ alkyl radical or a hydroxyl group,
- n is an integer ranging from 1 to 5,
- m is an integer ranging from 1 to 5, and
- x is chosen such that the amine number ranges from 0.01 to 1 meq/g;

f) multiblock polyoxyalkylene aminosilicones, of the type (AB)n, A being a polysiloxane block and B being a polyoxyalkylene block including at least one amine group.

Said silicones are preferably formed from repeating units having the following general formulae:

$$[-(SiMe_2O)xSiMe_2 - R -N(R'')- R'-O(C_2H_4O)_a(C_3H_6O)_b -R'-N(H)-R-]$$

or alternatively

$$[-(SiMe_2O)xSiMe_2 - R -N(R'')- R' - O(C_2H_4O)a(C_3H_6O)b -]$$

in which:

- a is an integer greater than or equal to 1, preferably ranging from 5 to 200 and more particularly ranging from 10 to 100;
- b is an integer between 0 and 200, preferably ranging from 4 to 100 and more particularly between 5 and 30;
- x is an integer ranging from 1 to 10 000 and more particularly from 10 to 5000;
- R" is a hydrogen atom or a methyl;
- R, which are identical or different, represent a linear or branched divalent $C_2$-$C_{12}$ hydrocarbon-based radical, optionally including one or more heteroatoms such as oxygen; preferably, R denotes an ethylene radical, a linear or branched propylene radical, a linear or branched butylene radical or a $CH_2CH_2CH_2OCH_2CH(OH)CH_2$- radical; preferentially, R denotes a $CH_2CH_2CH_2OCH_2CH(OH)CH_2$- radical;
- R', which are identical or different, represent a linear or branched divalent $C_2$-$C_{12}$ hydrocarbon-based radical, optionally including one or more heteroatoms such as oxygen; preferably, R' denotes an ethylene radical, a linear or branched propylene radical, a linear or branched butylene radical or a $CH_2CH_2CH_2OCH_2CH(OH)CH_2$- radical; preferentially, R' denotes -$CH(CH_3)$-$CH_2$-.

The siloxane blocks preferably represent between 50 mol% and 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular mass (Mw) of the silicone is preferably between 5000 and 1 000 000 and more particularly between 10 000 and 200 000.

Mention may particularly be made of the silicones sold under the name Silsoft A-843 or Silsoft A+ by Momentive.

g) and mixtures thereof.

Preferably, the aminosilicones of formula (B) are chosen from the aminosilicones corresponding to formula (E).

Preferably, the dyeing composition comprises at least one aminosilicone having the INCI name amodimethicone, preferably introduced in the form of an emulsion or microemulsion with surfactants.

Preferably, the dyeing composition comprises at least one aminosilicone having the INCI name amodimethicone as an emulsion or microemulsion with surfactants, having the INCI names trideceth-5 and trideceth-10.

The silicone(s) may be present in a total amount ranging from 0.01% to 20% by weight, preferably from 0.05% to 15% by weight, more preferentially from 0.1% to 10% by weight and even more preferentially still from 0.5% to 5% by weight relative to the total weight of the dyeing composition.

The aminosilicone(s) may be present in a total amount ranging from 0.01% to 20%, preferably from 0.05% to 15%, more preferentially from 0.1% to 10% and even more preferentially still from 0.5% to 5% by weight relative to the total weight of the dyeing composition.

**Associative polymers**

The composition for dyeing keratinous hair fibres used in the context of the colour-removing process according to the invention may also comprise at least one associative polymer other than the non-carboxylic anionic thickeners described previously.

It is recalled that *"associative polymers"* are polymers that are capable, in an aqueous medium, of reversibly associating with each other or with other molecules.

Their chemical structure more particularly comprises at least one hydrophilic zone and at least one hydrophobic zone.

*"Hydrophobic group"* means a radical or polymer with a saturated or unsaturated, linear or branched hydrocarbon-based chain, comprising at least 10 carbon atoms, preferably from 10 to 30 carbon atoms, in particular from 12 to 30 carbon atoms and more preferentially from 18 to 30 carbon atoms.

Preferentially, the hydrocarbon-based group originates from a monofunctional compound. By way of example, the hydrophobic group may be derived from a fatty alcohol such as stearyl alcohol, dodecyl alcohol or decyl alcohol. It may also denote a hydrocarbon-based polymer, for instance polybutadiene.

The associative polymers may be of non-ionic, anionic, cationic or amphoteric nature. Preferably, the associative polymer(s) are chosen from anionic associative polymers. Among the associative polymers of anionic type that may be mentioned are:

- (a) those comprising at least one hydrophilic unit and at least one fatty-chain allyl ether unit, more particularly those whose hydrophilic unit is formed by an ethylenic unsaturated anionic monomer, more particularly a vinylcarboxylic acid and most particularly an acrylic acid or a methacrylic acid or mixtures thereof.

Among these anionic associative polymers, those that are particularly preferred according to the invention are polymers formed from 20% to 60% by weight of acrylic acid and/or of methacrylic acid, from 5% to 60% by weight of lower alkyl (meth)acrylates, from 2% to 50% by weight of fatty-chain allyl ether, and from 0% to 1% by weight of a crosslinking agent which is a well-known copolymerizable unsaturated polyethylenic monomer, for instance diallyl phthalate, allyl (meth)acrylate, divinylbenzene, (poly)ethylene glycol dimethacrylate or methylenebisacrylamide.

Among the latter polymers, those most particularly preferred are crosslinked terpolymers of methacrylic acid, of ethyl acrylate and of polyethylene glycol (10 EO) stearyl alcohol ether (Steareth-10), notably those sold by the company Ciba under the names Salcare SC 80® and Salcare SC 90®, which are aqueous 30% emulsions of a crosslinked terpolymer of methacrylic acid, of ethyl acrylate and of steareth-10 allyl ether (40/50/10).

- (b) those comprising i) at least one hydrophilic unit of unsaturated olefinic carboxylic acid type, and ii) at least one hydrophobic unit of the type such as a ($C_{10}$-$C_{30}$) alkyl ester of an unsaturated carboxylic acid.

($C_{10}$-$C_{30}$) alkyl esters of unsaturated carboxylic acids that are useful in the invention comprise, for example, lauryl acrylate, stearyl acrylate, decyl acrylate, isodecyl acrylate and dodecyl acrylate, and the corresponding methacrylates, lauryl methacrylate, stearyl methacrylate, decyl methacrylate, isodecyl methacrylate and dodecyl methacrylate.

Anionic polymers of this type are described and prepared, for example, according to patents US 3 915 921 and US 4 509 949.

Among anionic associative polymers of this type, use will more particularly be made of those constituted of from 95% to 60% by weight of acrylic acid (hydrophilic unit), 4% to 40% by weight of $C_{10}$-$C_{30}$ alkyl acrylate (hydrophobic unit) and 0% to 6% by weight of crosslinking polymerizable monomer, or alternatively those constituted of from 98% to 96% by weight of acrylic acid (hydrophilic unit), 1% to 4% by weight of $C_{10}$-$C_{30}$ alkyl acrylate (hydrophobic unit) and 0.1% to 0.6%

by weight of crosslinking polymerizable monomer such as those described above.

Among said polymers above, those most particularly preferred according to the present invention are the products sold by the company Goodrich under the trade names Pemulen TR1®, Pemulen TR2®, Carbopol 1382®, and even more preferentially Pemulen TR1®, and the product sold by the company SEPPIC under the name Coatex SX®.

Mention may also be made of the acrylic acid/lauryl methacrylate/vinylpyrrolidone terpolymer sold under the name Acrylidone LM by the company ISP.

- (c) maleic anhydride/C30-C38 α-olefin/alkyl maleate terpolymers, such as the product (maleic anhydride/C30-C38 α-olefin/isopropyl maleate copolymer) sold under the name Performa V 1608® by the company Newphase Technologies.
- (d) acrylic terpolymers comprising:

  i) approximately 20% to 70% by weight of an α,β-monoethylenically unsaturated carboxylic acid [A],
  ii) approximately 20% to 80% by weight of an α,β-monoethylenically unsaturated non-surfactant monomer other than [A],
  iii) approximately 0.5% to 60% by weight of a non-ionic monourethane which is the reaction product of a monohydric surfactant with a monoethylenically unsaturated monoisocyanate,

  such as those described in patent application EP-A-0 173 109 and more particularly the terpolymer described in Example 3, namely a methacrylic acid/methyl acrylate/behenyl alcohol dimethyl-meta-isopropenylbenzylisocyanate ethoxylated (40 EO) terpolymer, as an aqueous 25% dispersion.
- (e) copolymers comprising, among their monomers, an α,β-monoethylenically unsaturated carboxylic acid and an ester of an α,β-monoethylenically unsaturated carboxylic acid and of an oxyalkylenated fatty alcohol.

Preferentially, these compounds also comprise, as monomer, an ester of an α,β-monoethylenically unsaturated carboxylic acid and of a $C_1$-$C_4$ alcohol.

An example of a compound of this type that may be mentioned is Aculyn 22® sold by the company Röhm & Haas, which is a methacrylic acid/ethyl acrylate/oxyalkylenated stearyl methacrylate terpolymer; and also Aculyn 88, also sold by the company Röhm & Haas.

Advantageously, the associative polymer(s) other than the non-carboxylic anionic thickeners are chosen from acrylic associative polymers, more preferentially carboxylic acrylic associative polymers.

Particularly preferably, the associative polymer(s) are chosen from copolymers including among their monomers an α,β-monoethylenically unsaturated carboxylic acid and an ester of an α,β-monoethylenically unsaturated carboxylic acid and of an oxyalkylenated fatty alcohol.

Advantageously, the total amount of the associative polymer(s) ranges from 0.05% to 15% by weight, preferably from 0.05% to 10% by weight, more preferentially from 0.1% to 5% by weight and even more preferentially from 0.1% to 1% by weight, relative to the total weight of the dyeing composition.

**Compound having at least one carboxylic acid group**

The composition for dyeing keratinous hair fibres used in the context of the colour-removing process according to the invention may also comprise at least one compound, other than the associative polymers as described previously, having at least one carboxylic acid group.

*"Carboxylic group"* means a COOH or COO- functional group, it being possible for the counterion of the COO- group to be chosen from alkali metals, alkaline-earth metals and quaternary ammoniums.

Preferably, the compound, other than the associative polymers, having at least one carboxylic acid group is chosen from polyurethanes, non-silicone acrylic polymers, silicone acrylic copolymers and mixtures thereof.

Polyurethanes and acrylic polymers

According to a preferred embodiment, the composition for dyeing keratinous hair fibres comprises one or more compounds, other than the associative polymers, having at least one carboxylic acid group chosen from polyurethanes, non-silicone acrylic polymers and mixtures thereof.

Preferably, the compound(s), other than the associative polymers, having at least one carboxylic acid group are in the form of aqueous dispersions of particles of polymer(s) chosen from polyurethanes, non-silicone acrylic polymers and mixtures thereof.

Preferably, the composition for dyeing keratinous hair fibres comprises one or more compounds, other than the associative polymers, having at least one carboxylic acid group in the form of aqueous dispersions of particles of

polymer(s) chosen from polyurethanes, non-silicone acrylic polymers and mixtures thereof.

Thus, in this embodiment, the polymer(s) used in the aqueous dispersions of particles of polymer(s) are other than the associative polymers.

The dispersion(s) may be simple dispersions in the aqueous medium of the cosmetic composition. As a particular case of dispersions, mention may be made of latexes.

The aqueous dispersion(s) of polymer particles may be chosen from aqueous dispersions of polyurethane particles.

More particularly, the polyurethane(s) present in the aqueous dispersions used in the present invention result from the reaction of:

- a prepolymer of formula (A) below:

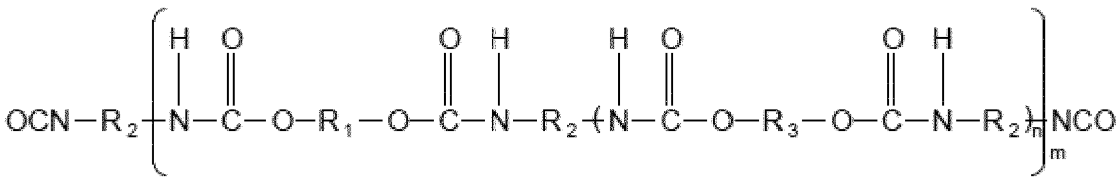

(A),

in which
- $R_1$ represents a divalent radical of a dihydroxylated compound;
- $R_2$ represents a radical of an aliphatic or cycloaliphatic polyisocyanate;
- $R_3$ represents a radical of a low molecular weight diol, optionally substituted with one or more ionic groups;
- n represents an integer ranging from 1 to 5, and
- m is greater than 1;
- at least one chain extender according to formula (B) below:

$$H_2N\text{-}R_4\text{-}NH_2 \quad (B),$$

in which $R_4$ represents an alkylene or alkylene oxide radical which is not substituted with one or more ionic or potentially ionic groups; and
- at least one chain extender according to formula (C) below:

$$H_2N\text{-}R_5\text{-}NH_2 \quad (C),$$

in which $R_5$ represents an alkylene radical substituted with one or more ionic or potentially ionic groups.

Among the dihydroxylated compounds that may be used according to the present invention, mention may be made particularly of the compounds having two hydroxyl groups and having a number-average molecular weight from approximately 700 to approximately 16 000, and preferably from approximately 750 to approximately 5000. By way of example of dihydroxylated compounds having a high molecular weight, mention may be made of polyol polyesters, polyol polyethers, polyhydroxylated polycarbonates, polyhydroxylated polyacetates, polyhydroxylated polyacrylates, polyhydroxylated amide polyesters, polyhydroxylated polyalkadienes, polyhydroxylated polythioethers, and mixtures thereof.

Preferably, the hydroxylated compounds are chosen from polyol polyesters, polyol polyethers, polyhydroxylated polycarbonates, and mixtures thereof.

The polyisocyanates that may be used according to the present invention are particularly chosen from organic diisocyanates having a molecular weight of approximately 112 to 1000, and preferably approximately 140 to 400.

Preferably, the polyisocyanates are chosen from diisocyanates and more particularly from those represented by the general formula $R_2(NCO)_2$, in which $R_2$ represents a divalent aliphatic hydrocarbon-based group having from 4 to 18 carbon atoms, a divalent cycloaliphatic hydrocarbon-based group having from 5 to 15 carbon atoms, a divalent araliphatic hydrocarbon-based group having from 7 to 15 carbon atoms or a divalent aromatic hydrocarbon-based group having from 6 to 15 carbon atoms.

Preferably, $R_2$ represents an organic diisocyanate. By way of example of organic diisocyanates, the following may particularly be chosen: tetramethylene diisocyanate, 1,6-hexamethylene diisocyanate, dodecamethylene diisocyanate, 1,3-diisocyanatocyclohexane, 1,4-diisocyanatocyclohexane, 3-isocyanatomethyl-3,5,5-trimethylcyclohexane isocyanate (isophorone diisocyanate or IPDI), bis(4-isocyanatocyclohexyl)methane, 1,3-bis(isocyanatomethyl)cyclohexane, 1,4-bis(isocyanatomethyl)cyclohexane, bis(4-isocyanato-3-methyl-cyclohexyl)methane, isomers of toluene diisocyanate (TDI) such as toluene 2,4-diisocyanate, toluene 2,6-diisocyanate and mixtures thereof, hydrogenated toluene diisocyanate, diphenylmethane 4,4'-diisocyanate and mixtures with its diphenylmethane 2,4-diisocyanate isomers and optionally diphenylmethane 2,2'-diisocyanate, naphthalene 1,5-diisocyanate isomers, and mixtures thereof.

Preferably, the diisocyanates are aliphatic and cycloaliphatic diisocyanates, and are more preferentially chosen from 1,6-hexamethylene diisocyanate, 3-isocyanatomethyl-3,5,5-trimethylcyclohexane isocyanate, and mixtures thereof.

According to the present invention, "low molecular weight diol" means a diol having a molecular weight from approximately 62 to 700, and preferably from 62 to 200. These diols may comprise aliphatic, alicyclic or aromatic groups. Preferably, they comprise only aliphatic groups.

Preferably, $R_3$ represents a low molecular weight diol having more than 20 carbon atoms, more preferentially chosen from ethylene glycol, diethylene glycol, 1,2-propanediol, 1,3-propanediol, 1,4-butanediol, 1,3-butylene glycol, neopentyl glycol, butylethylpropanediol, cyclohexanediol, 1,4-cyclohexanedimethanol, 1,6-hexanediol, bisphenol A (2,2-bis(4-hydroxyphenyl)propane), hydrogenated bisphenol A (2,2-bis(4-hydroxycyclohexyl)propane), and mixtures thereof.

The low molecular weight diols may optionally comprise ionic or potentially ionic groups. Examples of low molecular weight diols containing ionic or potentially ionic groups are particularly described in patent US 3 412 054. Such compounds are preferably chosen from dimethylolbutanoic acid, dimethylolpropionic acid, polycaprolactone diols containing a carboxyl group, and mixtures thereof.

If low molecular weight diols containing ionic or potentially ionic groups are used, they are preferably used in an amount such that less than 0.30 meq of COOH per gram of polyurethane is present in the polyurethane dispersion.

The prepolymer is extended by means of two chain extender families. The first family of chain extenders corresponds to the compounds of general formula (B).

The chain extenders of formula (B) are preferably chosen from alkylenediamines, such as hydrazine, ethylenediamine, propylenediamine, 1,4-butylenediamine, piperazine; alkylene oxide diamines, such as 3-{2-[2-(3-aminopropoxy)ethoxy]ethoxy}propylamine (also known as dipropylamine diethylene glycol or DPA-DEG available from Tomah Products, Milton, Wis.), 2-methyl-1,5-pentanediamine (Dytec A from DuPont), hexanediamine, isophorone diamine, 4,4-methylenedi(cyclohexylamine), ether-amines of the DPA series, available from Tomah Products, Milton, Wis., such as dipropylamine propylene glycol, dipropylamine dipropylene glycol, dipropylamine tripropylene glycol, dipropylamine poly(propylene glycol), dipropylamine ethylene glycol, dipropylamine poly(ethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol and dipropylamine cyclohexane-1,4-dimethanol; and mixtures thereof.

The second family of chain extenders corresponds to the compounds of general formula (C). Such compounds preferably have an ionic or potentially ionic group and two groups that can react with isocyanate groups. Such compounds may optionally comprise two groups that react with isocyanate groups and one group which is ionic or capable of forming an ionic group.

The ionic or potentially ionic group may preferably be chosen from ternary or quaternary ammonium groups or groups that can be converted into such groups, a carboxyl group, a carboxylate group, a sulfonic acid group and a sulfonate group. The at least partial conversion of groups that can be converted into a ternary or quaternary ammonium group salt may be performed before or during the mixing with water.

The chain extenders of formula (C) are preferably chosen from diaminosulfonates, for instance the sodium salt of N-(2-aminoethyl)-2-aminoethanesulfonic acid (ASA), the sodium salt of N-(2-aminoethyl)-2-aminopropionic acid, and mixtures thereof.

The polyurethane that may be used according to the present invention may optionally also comprise compounds which are located, respectively, at the chain ends and terminate said chains (chain terminators). Such compounds are particularly described in patents US 7 445 770 and/or US 7 452 770.

Preferably, the aqueous dispersion of polyurethane particles has a viscosity of less than 2000 mPa.s at 23°C, more preferentially less than 1500, and even better still less than 1000. Even more preferably, the aqueous polyurethane dispersion has a glass transition temperature of less than 0°C.

Preferably also, the aqueous polyurethane dispersion has a polyurethane (or active material, or dry matter) content, on the basis of the weight of the dispersion, of from 20% to 60% by weight, more preferentially from 25% to 55% by weight and even better still from 30% to 50% by weight. This means that the polyurethane content (dry matter) of the aqueous dispersion is preferably from 20% to 60% by weight, more preferentially from 25% to 55% by weight and even better still from 30% to 50% by weight, relative to the total weight of the dispersion.

Preferably also, the aqueous dispersion of polyurethane particles has a glass transition temperature (Tg) of less than or equal to -25°C, preferably less than -35°C and more preferentially less than -40°C.

The polyurethane particles may have a mean diameter ranging up to approximately 1000 nm, for example from approximately 50 nm to approximately 800 nm, better still from approximately 100 nm to approximately 500 nm. These

particle sizes may be measured with a laser particle size analyser (for example Brookhaven BI90).

As non-limiting examples of aqueous polyurethane dispersions, mention may be made of those sold under the name Baycusan® by Bayer, for instance Baycusan® C1000 (INCI name: polyurethane-34), Baycusan® C1001 (INCI name: polyurethane-34), Baycusan® C1003 (INCI name: polyurethane-32), Baycusan® C1004 (INCI name: polyurethane-35) and Baycusan® C1008 (INCI name: polyurethane-48).

Mention may also be made of the aqueous polyurethane dispersions of isophthalic acid/adipic acid copolymer/hexylene glycol/neopentyl glycol/dimethylol acid/isophorone diisocyanate (INCI name: Polyurethane-1, such as Luviset® PUR, BASF), the polyurethane of polycarbonate, polyurethane and aliphatic polyurethane of aliphatic polyester (such as the Neorez® series, DSM, such as Neorez® R989, Neorez® and R-2202).

According to a particular embodiment, the aqueous dispersion of polyurethane particles may be chosen from aqueous dispersions of particles of compounds having the INCI name polyurethane-35 or compounds having the INCI name polyurethane-34.

According to a preferred embodiment, the compound(s), other than the associative polymers, having at least one carboxylic acid group are in the form of aqueous dispersions of particles of non-silicone acrylic polymers, more preferentially in the form of aqueous dispersions of film-forming non-silicone acrylic polymer particles.

For the purposes of the invention, *"polymer"* means a compound corresponding to the repetition of one or more units (these units being derived from compounds known as monomers). This or these units are repeated at least twice and preferably at least three times.

*"Film-forming polymer"* means a polymer that is capable of forming, by itself or in the presence of an auxiliary film-forming agent, a macroscopically continuous film on a support, particularly on keratinous materials, and preferably a cohesive film.

For the purposes of the present invention, *"acrylic polymer"* means a polymer synthesized from at least one monomer chosen from (meth)acrylic acid and/or (meth)acrylic acid ester and/or (meth)acrylic acid amide.

The unit(s) derived from the (meth)acrylic acid monomers of the polymer may optionally be in the form of salt(s), particularly of alkali metal, alkaline-earth metal or ammonium salt(s), or organic base salt(s).

The (meth)acrylic acid esters (also known as (meth)acrylates) are advantageously chosen from alkyl (meth) acrylates, in particular $C_1$ to $C_{30}$, preferably $C_1$ to $C_{20}$ and better still $C_1$ to $C_{10}$ alkyl (meth)acrylates, aryl (meth)acrylates, in particular $C_6$ to $C_{10}$ aryl (meth)acrylates, and hydroxyalkyl (meth)acrylates, in particular $C_2$ to $C_6$ hydroxyalkyl (meth) acrylates.

Among the alkyl (meth)acrylates, mention may be made of methyl (meth)acrylate, ethyl (meth)acrylate, butyl (meth)acrylate, isobutyl (meth)acrylate, 2-ethylhexyl (meth)acrylate, lauryl (meth)acrylate and cyclohexyl (meth)acrylate.

Among the hydroxyalkyl (meth)acrylates, mention may be made of hydroxyethyl acrylate, 2-hydroxypropyl acrylate, hydroxyethyl methacrylate and 2-hydroxypropyl methacrylate.

Among the aryl (meth)acrylates, mention may be made of benzyl acrylate and phenyl acrylate.

The (meth)acrylic acid esters that are particularly preferred are alkyl, preferably $C_1$ to $C_{30}$, more preferentially $C_1$ to $C_{20}$, even better still $C_1$ to $C_{10}$, and even more particularly $C_1$ to $C_4$ alkyl (meth)acrylates.

According to the present invention, the alkyl group of the esters may be fluorinated, or even perfluorinated, i.e. some or all of the hydrogen atoms of the alkyl group are substituted with fluorine atoms.

As (meth)acrylic acid amides, examples that may be mentioned include (meth)acrylamides and also N-alkyl(meth)acrylamides, in particular N-(C2 to C12 alkyl)(meth)acrylamides. Among the N-alkyl(meth)acrylamides, mention may be made of N-ethylacrylamide, N-t-butylacrylamide, N-t-octylacrylamide and N-undecylacrylamide.

The acrylic polymer according to the invention may be a homopolymer or a copolymer, advantageously a copolymer, even better still a copolymer of (meth)acrylic acid and of (meth)acrylic acid esters.

Preferably, the acrylic polymer(s) according to the invention comprise one or more units derived from the following monomers:

a) (meth)acrylic acid; and

b) $C_1$ to $C_{30}$, more preferentially $C_1$ to $C_{20}$, better still $C_1$ to $C_{10}$, and even more particularly $C_1$ to $C_4$, alkyl (meth) acrylate.

Preferably, the aqueous dispersion of non-silicone acrylic polymer particles does not comprise any surfactant.

*"Surfactant"* means any agent that is capable of modifying the surface tension between two surfaces.

Among the acrylic polymers according to the invention, mention may be made of copolymers of (meth)acrylic acid and of methyl or ethyl (meth)acrylate, in particular copolymers of methacrylic acid and of ethyl acrylate such as the compound sold under the trade name Luvimer MAE by the company BASF, or the compound Polyacrylate-2 Crosspolymer sold under the trade name Fixate Superhold Polymer by the company Lubrizol, or the compound Acrylate Copolymer sold under the trade name Daitosol 3000VP3 by the company Daito Kasei Kogyo, or the compound Acrylate Polymer sold under the trade name Daitosol 3000 SLPN-PE1 by the company Daito Kasei Kogyo.

The acrylic polymer may optionally comprise one or more additional monomers, other than the (meth)acrylic acid and/or (meth)acrylic acid ester and/or (meth)acrylic acid amide monomers.

By way of additional monomer, mention will be made, for example, of styrene monomers, in particular styrene and α-methylstyrene, and preferably styrene.

In particular, the acrylic polymer may be a styrene/(meth)acrylate copolymer and particularly a polymer chosen from copolymers resulting from the polymerization of at least one styrene monomer and at least one $C_1$ to $C_{20}$, preferably $C_1$ to $C_{10}$, alkyl (meth)acrylate monomer.

The $C_1$ to $C_{10}$ alkyl (meth)acrylate monomer may be chosen from methyl acrylate, ethyl acrylate, propyl acrylate, butyl acrylate, hexyl acrylate, octyl acrylate and 2-ethylhexyl acrylate.

As non-silicone acrylic polymer, mention may be made of the styrene/(meth)acrylate copolymers sold under the name Joncryl 77 by the company BASF, under the name Yodosol GH41F by the company Akzo Nobel and under the name Syntran 5760 CG by the company Interpolymer.

Silicone acrylic copolymer

According to a preferred embodiment, the compound(s), other than the associative polymers, having at least one carboxylic acid group, are chosen from silicone acrylic copolymers.

Preferably, said silicone acrylic copolymers comprise:

- at least one acrylic, methacrylic or crotonic unit; and
- at least one polydimethylsiloxane (PDMS) unit.

For the purposes of the present invention, *"polydimethylsiloxanes"* (also known, in abbreviation, as PDMSs) means, in accordance with what is generally accepted, any organosilicon polymer or oligomer having a linear structure, of variable molecular weight, obtained by polymerization and/or polycondensation of suitably functionalized silanes, and constituted essentially of a repetition of main units in which the silicon atoms are connected together by oxygen atoms (siloxane -Si-O-Si- bond), comprising methyl radicals directly bonded via a carbon atom to said silicon atoms.

The PDMS chains that may be used to obtain the copolymer used according to the invention include at least one polymerizable radical group, preferably located on at least one of the ends of the chain, i.e. the PDMS may have, for example, a polymerizable radical group on each of the two ends of the chain or one polymerizable radical group on one end of the chain and one trimethylsilyl end group on the other end of the chain.

*"Polymerizable radical group"* means a radical capable of polymerizing with other polymerizable radical groups or monomers. Preferably, the polydimethylsiloxane unit comprises at least one polymerizable radical group.

Preferably, the polymerizable radical group comprises at least one vinyl group.

Preferably, the polydimethylsiloxane (PDMS) unit comprises at least one polymerizable radical group comprising at least one vinyl group, preferably at least two polymerizable radical groups comprising at least one vinyl group, preferably located on at least one of the ends of the chain.

As indicated previously, said silicone acrylic copolymer(s) comprise at least one acrylic, methacrylic or crotonic unit, i.e. at least one unit comprising a carboxylic group.

Preferably, said silicone acrylic copolymers comprise:

- at least one acrylic, methacrylic or crotonic unit, and at least one acrylic ester, methacrylic ester or vinyl ester unit; and
- at least one polydimethylsiloxane (PDMS) unit.

More preferentially, the dyeing composition comprises one or more silicone acrylic copolymers comprising:

- at least one crotonic unit and at least one unit chosen from an alkyl crotonate unit, the alkyl radical being a linear or branched saturated radical containing from 1 to 20 carbon atoms; a vinyl acetate unit; a vinyl alkyl ester unit, the alkyl radical being a linear or branched saturated radical containing from 2 to 20 carbon atoms; and mixtures thereof; and
- at least one polydimethylsiloxane (PDMS) unit.

*"Crotonic unit"* means a unit derived from a crotonic acid monomer or a salt thereof.

*"Alkyl crotonate unit"* means a unit derived from a crotonic acid ester monomer unit, having a linear or branched saturated alkyl radical containing from 1 to 20 carbon atoms. *"Vinyl alkyl ester unit"* means a unit derived from a vinyl ester monomer unit, having a linear or branched saturated alkyl radical containing from 2 to 20 carbon atoms.

*"Vinyl acetate unit"* means a unit derived from a vinyl acetate monomer.

According to a preferred embodiment, said silicone acrylic copolymer(s) comprise:

- at least one crotonic unit, at least one vinyl acetate unit and at least one vinyl alkyl ester unit, the alkyl radical being a linear or branched saturated radical containing from 2 to 20 carbon atoms, preferably from 2 to 18 carbon atoms, and
- at least one polydimethylsiloxane (PDMS) unit.

According to a particularly preferred embodiment, said silicone acrylic copolymer(s) comprise:

- at least one crotonic unit, at least one vinyl acetate unit and at least one vinyl alkyl ester unit, the alkyl radical being a linear or branched saturated radical containing from 6 to 16 carbon atoms, and
- at least one polydimethylsiloxane (PDMS) unit comprising at least one polymerizable radical group comprising at least one vinyl group.

More preferentially, the dyeing composition comprises one or more silicone acrylic copolymers comprising:

- at least one crotonic unit, at least one vinyl acetate unit and at least one vinyl alkyl ester unit, the alkyl radical being a linear or branched saturated radical containing from 2 to 20 carbon atoms, preferably from 2 to 18 carbon atoms, and
- at least one polydimethylsiloxane (PDMS) unit comprising at least one polymerizable radical group comprising at least one vinyl group.

Even more preferentially, the dyeing composition comprises one or more silicone acrylic copolymers comprising:

- at least one crotonic unit, at least one vinyl acetate unit and at least one vinyl alkyl ester unit, the alkyl radical being a linear or branched saturated radical containing from 6 to 16 carbon atoms, and
- at least one polydimethylsiloxane (PDMS) unit comprising at least one polymerizable radical group comprising at least one vinyl group.

Among the silicone acrylic copolymers which may be used in the context of the invention, mention may be made of the compound sold by the company Wacker Chemie AG under the trade name Belsil® P1101, having the INCI name Crotonic Acid/Vinyl C8-12 Isoalkyl Esters/VABis-Vinyldimethicone Crosspolymer.
Preferably, the dyeing composition comprises at least one aqueous dispersion of non-silicone acrylic polymer particles.
More preferentially, the dyeing composition comprises at least one aqueous dispersion of non-silicone acrylic polymer particles comprising one or more units derived from the following monomers:

a) (meth)acrylic acid; and
b) $C_1$ to $C_{30}$, more preferentially $C_1$ to $C_{20}$, better still $C_1$ to $C_{10}$, and even more particularly $C_1$ to $C_4$, alkyl (meth) acrylate.

Preferably, the aqueous dispersion of non-silicone acrylic polymer particles has an acrylic polymer (or active material, or dry matter) content, on the basis of the weight of the dispersion, of from 20% to 60% by weight, more preferentially from 22% to 55% by weight and better still from 25% to 50% by weight.
The total amount of the compound(s), other than the associative polymer(s), having at least one carboxylic acid group preferably ranges from 0.1% to 35% by weight, more preferentially from 0.5% to 30% by weight, better still from 1% to 25% by weight, and even more preferentially from 3% to 15% by weight, relative to the total weight of the dyeing composition.
The total amount of the aqueous dispersion(s) of particles of polymer(s), other than the associative polymer(s) as described previously, chosen from polyurethanes, non-silicone acrylic polymers, and mixtures thereof, preferably ranges from 0.1% to 50% by weight, more preferentially from 0.5% to 40% by weight, better still from 1% to 30% by weight, and even more preferentially from 3% to 20% by weight, relative to the total weight of the dyeing composition.
According to a preferred embodiment, the total amount of the aqueous dispersion(s) of particles of non-silicone acrylic polymer(s), other than the associative polymer(s) as described previously, preferably ranges from 0.1% to 50% by weight, more preferentially from 0.5% to 40% by weight, better still from 1% to 30% by weight, and even more preferentially from 3% to 20% by weight, relative to the total weight of the dyeing composition.

**Organic solvent**

The dyeing composition used in the context of the process according to the invention may comprise one or more organic solvents.
Examples of organic solvents that may be mentioned include lower $C_1$-$C_4$ alkanols, such as ethanol and

isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

Preferably, the dyeing composition comprises one or more organic solvents chosen from $C_1$-$C_4$ lower alkanols, more preferentially ethanol.

The organic solvents may be present in a total amount inclusively between 0.01% and 60% by weight approximately relative to the total weight of the dyeing composition, preferably between 0.05% and 50% by weight and more preferentially inclusively between 0.1% and 40% by weight relative to the total weight of the dyeing composition. The dyeing composition used in the context of the process according to the invention is preferably aqueous. The water content may range from 20% to 99% by weight, preferably from 30% to 98% by weight and more preferentially from 40% to 95% by weight relative to the total weight of the dyeing composition.

**Additives**

The composition for dyeing keratinous hair fibres used in the context of the process according to the invention may contain any adjuvant or additive usually used.

Among the additives that may be contained in the composition, mention may be made of reducing agents, softeners, antifoams, moisturizers, UV-screening agents, peptizers, solubilizers, fragrances, proteins, vitamins, polymers other than the polymers described previously, anionic, cationic, non-ionic or amphoteric surfactants, preserving agents, oils, waxes and mixtures thereof.

The composition for dyeing keratinous hair fibres may particularly be in the form of a suspension, a dispersion, a gel, an emulsion, particularly an oil-in-water (O/W) or water-in-oil (W/O) emulsion, or a multiple emulsion (W/O/W or polyol/O/W or O/W/O), in the form of a cream, a mousse, a stick, a dispersion of vesicles, particularly of ionic or non-ionic lipids, or a two-phase or multi-phase lotion.

A person skilled in the art may select the appropriate presentation form, and also the method for preparing it, on the basis of their general knowledge, taking into account firstly the nature of the constituents used, particularly their solubility in the support, and secondly the intended application of the composition.

**Application of composition D**

The process for removing colour from keratinous hair fibres, such as the hair, according to the invention may also comprise a step of applying, to the keratinous hair fibres, a composition D comprising at least one silicone compound comprising at least one carboxylic group, the application of said composition D taking place before the application of the colour-removing composition.

Preferably, the silicone compound comprising at least one carboxylic group is a silicone compound other than the silicone acrylic copolymer comprising at least one carboxylic group and than the aminosilicone as were described previously.

*"Carboxylic group"* means a COOH or $COO^-$ functional group, it being possible for the counterion of the $COO^-$ group to be chosen from alkali metals, alkaline-earth metals and quaternary ammoniums.

The silicones that may be used may be soluble or insoluble in composition D; they may be in the form of oil, wax, resin or gum; silicone oils and gums are preferred.

Silicones are particularly described in detail in Walter Noll's "Chemistry and Technology of Silicones" (1968), Academic Press.

Preferably, the silicone compound(s) comprising at least one carboxylic group are chosen from the organosiloxanes of formula (XXVII) below:

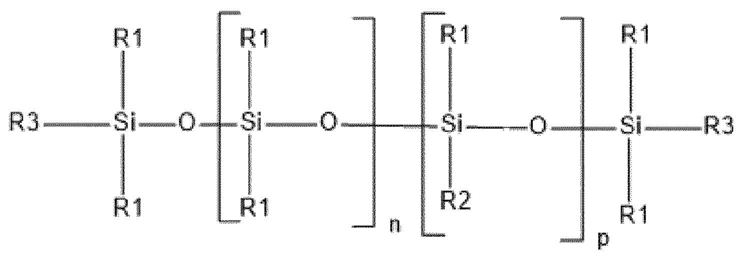

(XXVII)

in which:

- **R1** independently represents an alkyl group having from 1 to 20 carbon atoms, preferably from 1 to 10 carbon atoms; a hydroxyl group; an alkoxy group having from 1 to 20 carbon atoms or an aryl group having from 6 to 12 carbon atoms;
- **R2** independently represents a group R4-COOM with R4 representing a linear or branched alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof, and M representing a hydrogen atom; an alkali metal or alkaline-earth metal or a quaternary ammonium NR'3, with R', which are identical or different, representing H or alkyl having from 1 to 4 carbon atoms; a pyrrolidone radical comprising a carboxylic group COOH or a group Ra-(ORb)x-COOM with Ra representing a linear or branched alkylene group having from 1 to 4 carbon atoms, Rb representing an alkyl group having from 1 to 4 carbon atoms, **x** being an integer ranging from 1 to 200; and **M** representing a hydrogen atom, an alkali metal or alkaline-earth metal or a quaternary ammonium $NR'_3$, with **R',** which are identical or different, representing H or an alkyl having from 1 to 4 carbon atoms;
- **R3** independently represent an alkyl group having from 1 to 20 carbon atoms; a hydroxyl group; a group R4-COOM with **R4** representing a linear or branched alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof, and **M** representing a hydrogen atom; an alkali metal or alkaline-earth metal or a quaternary ammonium $NR'_3$, with **R',** which are identical or different, representing H or alkyl having from 1 to 4 carbon atoms; an alkoxy group having from 1 to 20 carbon atoms; an aryl group having from 6 to 12 carbon atoms or a group $R_a$-$(OR_b)_x$-COOM with $\mathbf{R_a}$ representing a linear or branched alkylene group having from 1 to 4 carbon atoms, $\mathbf{R_b}$ representing an alkyl group having from 1 to 4 carbon atoms, x being an integer ranging from 1 to 200; and **M** representing a hydrogen atom, an alkali metal or alkaline-earth metal or a quaternary ammonium $NR'_3$, with **R',** which is identical or different, representing H or an alkyl having from 1 to 4 carbon atoms;
- **n** denotes an integer ranging from 1 to 1000;
- **p** denotes an integer ranging from 0 to 1000;

it being understood that at least one of the radicals R2 and/or R3 comprises a carboxylic group COOH or COOM with M representing an alkali metal or alkaline-earth metal or a quaternary ammonium $NR'_3$, with **R',** which are identical or different, representing H or an alkyl having from 1 to 4 carbon atoms.

Particularly, the silicone compound(s) comprising at least one carboxylic group may be chosen from the organosiloxanes of formula (XXVIII) below:

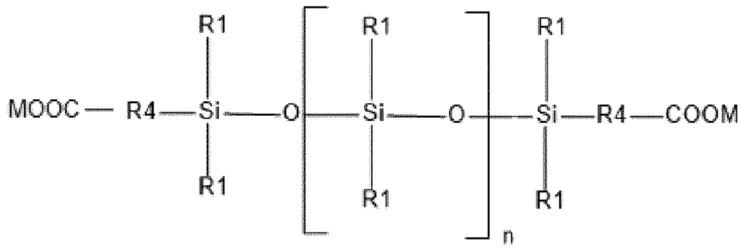

(XXVIII),

in which:

- **R1** independently represents a linear or branched alkyl group having from 1 to 20 carbon atoms, preferably from 1 to 10 carbon atoms and better still from 1 to 6 carbon atoms, preferentially methyl;
- **R4** independently represents a linear or branched alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group $R_a$-$(OR_b)_x$- with $\mathbf{R_a}$ representing a linear or branched alkylene group having from 1 to 4 carbon atoms, $\mathbf{R_b}$ representing an alkylene group having from 1 to 4 carbon atoms, and x being an integer ranging from 1 to 200;
- **M** independently represents a hydrogen atom, an alkali metal or alkaline-earth metal or a quaternary ammonium $NR'_3$, with **R',** which are identical or different, representing H or an alkyl having from 1 to 4 carbon atoms;
- **n** denotes an integer ranging from 1 to 1000;
- the organosiloxanes of formula (XXIX) below:

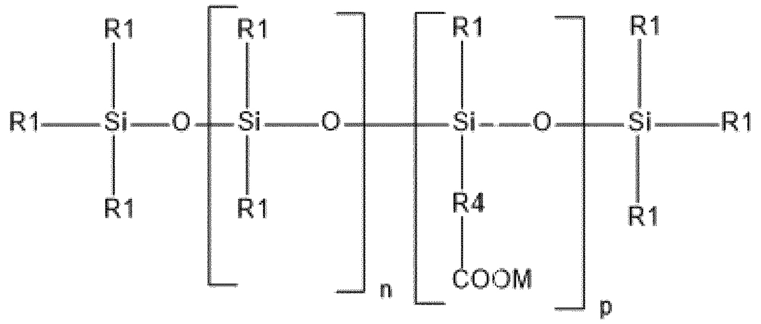

(XXIX),

in which:

- **R1** independently represents an alkyl group having from 1 to 10 carbon atoms, preferably from 1 to 6 carbon atoms, more preferentially a methyl;
- **R4** represents a linear or branched, saturated or unsaturated alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group $R_a$-$(OR_b)_x$- with $\mathbf{R_a}$ representing a linear or branched alkylene group having from 1 to 4 carbon atoms, $\mathbf{R_b}$ representing an alkylene group having from 1 to 4 carbon atoms, and x being an integer ranging from 1 to 200;
- **M** represents a hydrogen atom, an alkali metal or alkaline-earth metal or a quaternary ammonium $NR'_3$, with **R',** which are identical or different, representing H or an alkyl having from 1 to 4 carbon atoms;
- **p** denotes an integer ranging from 1 to 1000;
- **n** denotes an integer ranging from 1 to 1000;
- the organosiloxanes of formula (XXX) below:

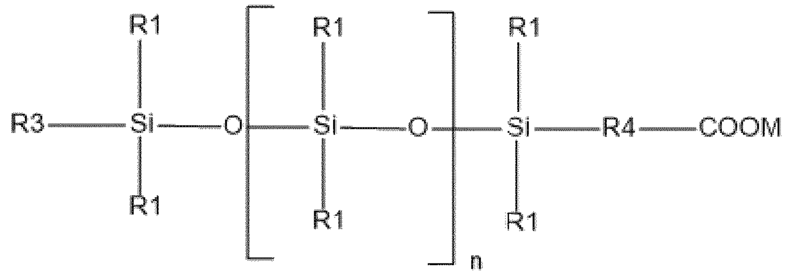

(XXX)

in which:

- **R1** independently represents an alkyl group having from 1 to 20 carbon atoms, preferably from 1 to 10 carbon atoms and better still from 1 to 6 carbon atoms, preferentially methyl;
- **R4** represents a linear or branched alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group $R_a$-$(OR_b)_x$- with $\mathbf{R_a}$ representing a linear or branched alkylene group having from 1 to 4 carbon atoms, $\mathbf{R_b}$ representing an alkylene group having from 1 to 4 carbon atoms, and **x** being an integer ranging from 1 to 200;
- **R3** represents an alkyl group having from 1 to 20 carbon atoms, an alkoxy group having from 1 to 20 carbon atoms or an aryl group having from 6 to 12 carbon atoms;
- **M** independently represents a hydrogen atom, an alkali metal or alkaline-earth metal or a quaternary ammonium $NR'_3$, with **R',** which are identical or different, representing H or an alkyl having from 1 to 4 carbon atoms;
- **n** denotes an integer ranging from 1 to 1000;
- the organosiloxanes of formula (XXXI) below:

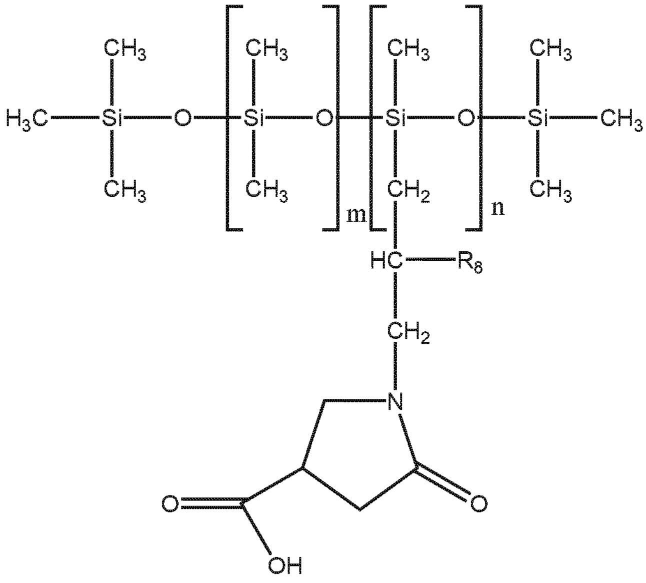

(XXXI)

in which:

- **R8** represents an alkyl group having from 1 to 6 carbon atoms, preferably a methyl;
- **m** denotes an integer ranging from 1 to 1000;
- **n** denotes an integer ranging from 1 to 1000;
- and mixtures thereof.

Among the organosiloxanes of formula (XXVIII), mention may be made of polydimethylsiloxanes (PDMS) bearing a carboxyl end function, such as the compounds sold by the company Momentive under the trade name Silform INX (INCI name: Bis-Carboxydecyl Dimethicone).

Among the organosiloxanes of formula (XXIX), mention may be made of polydimethylsiloxanes (PDMS) bearing a pendent carboxyl function, such as the compounds sold by the company Shin-Etsu under the trade name X-22-3701E.

Among the organosiloxanes of formula (XXX), mention may be made of polydimethylsiloxanes (PDMS) bearing a carboxyl end function, such as the compounds sold by the company Shin-Etsu under the trade name X-22-3710.

Among the organosiloxanes of formula (XXXI), mention may be made of the compounds sold by the company Grant Industries under the trade name Grandsil SiW-PCA-10 (INCI name: Dimethicone (and) PCA Dimethicone (and) Butylene Glycol (and) Decyl Glucoside).

The silicone compounds comprising a carboxylic group may correspond, for example, to the compounds described in the patent application EP 186 507 in the name of Chisso Corporation.

Preferably, the silicone compound(s) comprising at least one carboxylic group are chosen from the organopolysiloxanes of formula (XXVIII), the organopolysiloxanes of formula (XXIX) and mixtures thereof.

More preferentially, the silicone compound(s) comprising at least one carboxylic group are chosen from the organopolysiloxanes of formula (XXVIII) below:

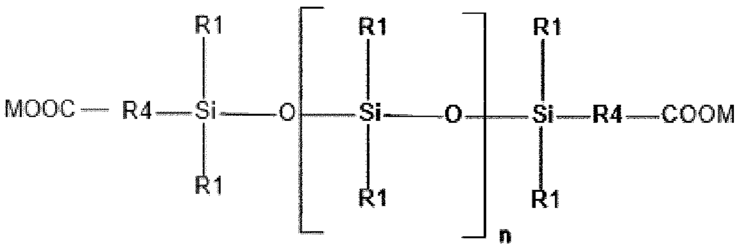

(XXVIII)

in which:

- **R1** independently represents a linear or branched alkyl group having from 1 to 20 carbon atoms, preferably from 1 to

10 carbon atoms and better still from 1 to 6 carbon atoms, preferentially methyl;

- **R4** independently represents a linear or branched alkylene group having from 1 to 20 carbon atoms, preferably from 4 to 16 carbon atoms, optionally interrupted with at least one heteroatom chosen from a sulfur atom, a nitrogen atom, an oxygen atom and mixtures thereof; or a divalent group $R_a$-$(OR_b)_x$- with $\mathbf{R_a}$ representing a linear or branched alkylene group having from 1 to 4 carbon atoms, $\mathbf{R_b}$ representing an alkylene group having from 1 to 4 carbon atoms, and x being an integer ranging from 1 to 200;
- **M** independently represents a hydrogen atom, an alkali metal or alkaline-earth metal or a quaternary ammonium $NR'_3$, with **R',** which are identical or different, representing H or an alkyl having from 1 to 4 carbon atoms;
- **n** denotes an integer ranging from 1 to 1000.

Advantageously, the total amount of silicone compound(s) comprising at least one carboxylic group ranges from 0.01% to 20% by weight, preferably from 0.1% to 15% by weight, more preferentially from 0.5% to 10% by weight, better still from 1% to 5% by weight, relative to the total weight of the composition D.

## Oils

Composition D may comprise one or more oil(s) other than the silicone compounds comprising a carboxylic group described previously.

Preferably, composition D comprises one or more oil(s). More preferentially, composition D comprises one or more oil(s) chosen from alkanes.

*"Oil"* means a fatty substance that is liquid at room temperature (25°C) and at atmospheric pressure (760 mmHg or $1.013 \times 10^5$ Pa).

The oil may be volatile or nonvolatile.

*"Volatile oil"* means an oil that can evaporate on contact with the skin in less than one hour, at room temperature and atmospheric pressure. The volatile oil is a cosmetic volatile oil, which is liquid at room temperature. More specifically, a volatile oil has an evaporation rate of between 0.01 and 200 $mg/cm^2/min$, limits included (see protocol for measuring the evaporation rate indicated in the text below).

*"Nonvolatile oil"* means an oil that remains on the skin or the keratinous hair fibre at room temperature and atmospheric pressure. More specifically, a nonvolatile oil has an evaporation rate of strictly less than 0.01 mg/cm2/min (see protocol for measuring the evaporation rate indicated in the text below).

Preferably, the composition comprises one or more oil(s) chosen from $C_6$-$C_{16}$ alkanes and/or mixtures thereof.

As regards the $C_6$-$C_{16}$ alkanes, they may be linear or branched, and optionally cyclic.

Mention may particularly be made of branched $C_8$-$C_{16}$ alkanes, such as $C_8$-$C_{16}$ isoalkanes (also known as isoparaffins), isododecane, isodecane or isohexadecane, and for example the oils sold under the Isopar or Permethyl trade names, and mixtures thereof.

Mention may also be made of linear alkanes, preferably of plant origin, comprising from 7 to 15 carbon atoms, in particular from 9 to 14 carbon atoms and more particularly from 11 to 13 carbon atoms.

As examples of linear alkanes that are suitable for the invention, mention may be made of n-heptane (C7), n-octane (C8), n-nonane (C9), n-decane (C10), n-undecane (C11), n-dodecane (C12), n-tridecane (C13), n-tetradecane (C14) and n-pentadecane (C15), and mixtures thereof, and in particular the mixture of n-undecane (C11) and n-tridecane (C13) described in Example 1 of patent application WO 2008/155 059 by the company Cognis. Mention may also be made of n-dodecane (C12) and n-tetradecane (C14) sold by Sasol respectively under the references Parafol 12-97 and Parafol 14-97, and also the mixtures thereof.

As examples of alkanes that are suitable for the invention, mention may be made of the alkanes described in patent applications WO 2007/068 371 and WO 2008/155 059. These alkanes are obtained from fatty alcohols, which are themselves obtained from coconut kernel oil or palm oil.

According to a particular embodiment, the composition comprises isododecane. Such a compound is, for example, the isododecane sold under the reference Isododecane by Ineos.

Preferably, composition D comprises one or more oil(s) chosen from $C_8$-$C_{16}$ alkanes, more preferentially from isododecane, isohexadecane, tetradecane and/or mixtures thereof.

More preferentially, composition D comprises isododecane.

Composition D may comprise one or more oil(s) other than the silicone compounds comprising a carboxylic group, present in a total amount of between 30% and 99% by weight, preferably between 50% and 99% by weight and better still between 70% and 99% by weight, relative to the total weight of composition D.

Composition D may comprise at least one colouring agent chosen from pigments, direct dyes and mixtures thereof as described previously.

**Colour-removing composition**

As indicated previously, the process for removing colour from keratinous hair fibres, in particular the hair, comprises the application, to said keratinous hair fibres which have been previously dyed using at least one composition for dyeing keratinous hair fibres as defined above, of at least one colour-removing composition comprising at least one alkyl or alkylene carbonate.

This colour-removing composition consists in creating a chemical action intended to alter the integrity of the coloured coating formed around the keratinous hair fibres in order to lead to the elimination of this coloured coating from said keratinous hair fibres.

Alkyl or alkylene carbonate

*"Alkyl carbonate"* means an alkyl or dialkyl carbonate.

Preferably, the alkyl or alkylene carbonate is a $C_1$-$C_{30}$, preferably $C_1$-$C_6$ alkyl carbonate, or a $C_1$-$C_{30}$, preferably $C_1$-$C_6$ alkylene carbonate.

More preferentially, the alkyl or alkylene carbonate is chosen from alkylene carbonates, better still from propylene carbonate, butylene carbonate, pentylene carbonate and mixtures thereof.

Particularly preferably, the alkyl or alkylene carbonate is chosen from propylene carbonate.

Advantageously, the total content of alkyl or alkylene carbonate ranges from 10% to 60% by weight, preferably from 15% to 50% by weight, relative to the total weight of the colour-removing composition.

Glycol ether

The colour-removing composition used in the context of the process according to the invention may also comprise at least one glycol ether.

*"Glycol ether"* means one or more glycol ethers comprising a hydroxyl group or no hydroxyl groups.

Preferably, the glycol ether is chosen from those of formula $R_{11}(-O-CH(CH_3)-CH_2)_nOH$, in which $R_{11}$ denotes a $C_1$-$C_{30}$, preferably $C_1$-$C_6$ and more preferentially $C_1$-$C_4$ alkyl radical, and in which n is from 1 to 50, preferably from 2 to 50, more preferably still from 2 to 10, preferentially 2 to 5.

Advantageously, the glycol ether is of formula $CH_3(-O-CH(CH_3)-CH_2)_nOH$, in which n is from 2 to 50, preferably from 2 to 10, more preferentially from 2 to 5.

Particularly preferably, the glycol ether is chosen from tripropylene glycol methyl ether, tripropylene glycol ethyl ether, tripropylene glycol propyl ether, tripropylene glycol butyl ether and mixtures thereof, preferably tripropylene glycol methyl ether.

The total content of glycol ether(s) may range from 0.1% to 40% by weight, preferably from 1% to 30% by weight and more preferentially from 5% to 20% by weight relative to the total weight of the colour-removing composition.

Polyol

The colour-removing composition used in the context of the process according to the invention may also comprise at least one polyol other than the glycol ethers described previously.

For the purposes of the present invention, *"polyol"* means an organic compound constituted of a hydrocarbon-based chain optionally interrupted with one or more oxygen atoms and bearing at least two free hydroxyl groups (-OH) borne by different carbon atoms, it being possible for this compound to be cyclic or acyclic, linear or branched, and saturated or unsaturated.

More particularly, the polyol(s) comprise from 2 to 30 hydroxyl groups, more preferentially from 2 to 10 hydroxyl groups, even more preferentially still from 2 to 3 hydroxyl groups.

The colour-removing composition may comprise one or more polyols chosen from diglycerol, glycerol, propylene glycol, propane-1,3-diol, 1,3-butylene glycol, pentane-1,2-diol, octane-1,2-diol, dipropylene glycol, hexylene glycol, ethylene glycol, polyethylene glycols, sorbitol, sugars such as glucose, and mixtures thereof.

Particularly preferably, the polyol is propylene glycol.

Advantageously, the content of polyol, when it is present, ranges from 1% to 70% by weight, preferably from 5% to 60% by weight, more preferentially from 10% to 50% by weight, relative to the total weight of the colour-removing composition.

The colour-removing composition used in the context of the process according to the invention may comprise water.

According to another particular embodiment, the colour-removing composition does not comprise any water.

Moreover, the additives as mentioned above may be included in the colour-removing composition used in the

context of the process according to the invention.

The colour-removing composition used in the context of the process according to the invention may comprise one or more non-carboxylic anionic thickeners as described previously, in a total content ranging preferably from 0.01% to 10% by weight, preferentially from 0.1% to 5% by weight, better still from 0.2% to 2% by weight, relative to the total weight of the composition.

**Tongs for keratinous hair fibres**

As indicated previously, the process for removing the colour from keratinous hair fibres, in particular the hair, comprises applying, to said keratinous hair fibres which have been previously dyed using the dyeing composition as described previously and from which the colour has been removed by means of the colour-removing composition, tongs comprising two arms A and B, between which arms said keratinous hair fibres pass.

The tongs for the keratinous hair fibres, particularly the hair, as described previously, may have an axis of articulation (Z) about which the arms A and B are articulated.

According to a preferred embodiment, arms A and B are articulated about the axis of articulation (Z) and can move angularly towards one another.

Each arm A and B comprises a supporting part and an active part.

The supporting part corresponds to the part for gripping the tongs by the user. The active part corresponds to the part in contact with the keratinous hair fibres.

Said arms A and B are movable between a spaced-apart position in which the active parts are at a distance from one another, and a brought-together position in which the active parts are brought towards one another.

According to a preferred embodiment, the active parts of arms A and B are arranged facing one another.

According to a preferred embodiment, the arms A and B are articulated about the axis of articulation (Z) and can move angularly towards one another in order for the active parts of arms A and B to be in contact with one another.

When the active parts of arms A and B are in contact, the keratinous hair fibres are gripped between the active parts of arms A and B.

Said active parts of arms A and B can comprise an active face and an opposite face, said active face having bristles.

Said bristles can be arranged in the form of tufts of bristles.

There are free spaces between each tuft of bristles.

Preferably, the active faces of arms A and B have tufts of bristles and free spaces.

The tufts of bristles are generally made from a natural material such as natural silks, or animal hair, such as squirrel, boar or goat hair.

Using natural materials for producing tongs of this kind considerably increases the manufacturing costs thereof, and therefore tongs have been developed in which the tufts of bristles are made from synthetic hair that substantially reproduces the mechanical properties of tongs in which the tufts of bristles have been made from natural hair.

Each of the active parts of arms A and B can comprise rows of tufts of bristles.

There are free spaces between each row of tufts of bristles.

Preferably, the active faces of arms A and B have rows of tufts of bristles separated by free spaces.

According to a preferred embodiment, the rows of tufts of bristles of the active parts are arranged parallel to one another.

In this embodiment, each row of tufts of bristles is separated by free spaces.

The keratinous hair fibres can be placed between the tufts of bristles so as to form waves. In the brought-together position of the active parts, said bristles of the arms A and B intermingle so as to grip said keratinous hair fibres without blocking them.

Preferably, in the brought-together position of the active parts of arms A and B, the rows of tufts of arm A intermingle with the rows of tufts of bristles of arm B so as to grip said keratinous hair fibres without blocking them.

In the brought-together position of arms A and B, the tongs according to the invention are able to move along the keratinous hair fibres, i.e. from the root to the tip of the keratinous hair fibres, without blocking them.

By moving the tongs according to the invention along the keratinous hair fibres while maintaining the brought-together position of arms A and B, said tufts of bristles of arms A and B apply a frictional force to the keratinous hair fibres which have been dyed, then from which the colour has been removed, in order to remove the rest of the coloured coating present on said keratinous hair fibres.

The frictional force applied to the keratinous hair fibres is between 5 N and 20 N.

The invention may be better understood from reading the following detailed description, and by referring to the appended drawings, in which:

- Figure 1 shows a schematic elevation view of an example of tongs for keratinous hair fibres produced in accordance

with the invention;

- Figure 2 shows, in isolation, the active faces of the active parts of arms A and B of Figure 1, placed side by side.

Figure 1 shows tongs 1 for keratinous hair fibres, particularly the hair, as described above. The tongs 1 comprise two arms A and B, between which said keratinous hair fibres pass. The arms A and B are articulated about an axis of articulation (Z) and can move angularly towards one another, particularly via one or more springs (not shown).
The arms A and B are able to close over the keratinous hair fibres, driven by an external force, with a view to moving along said keratinous hair fibres.
Each arm A and B comprises a supporting part 2 and an active part 3.
The active parts 3 of arms A and B can be arranged facing one another.
The two arms A and B can be articulated about the axis of articulation (Z) and can move angularly towards one another in order for the active parts 3 of arms A and B to be in contact with one another.
Said active parts 3 of arms A and B comprise an active face 4 and an opposite face 5.
The active faces 4 of arms A and B have bristles 6. Preferably, the opposite faces of arms A and B do not have bristles.
The bristles can be grouped together into tufts of bristles 6.
Figure 2 shows the active faces 4 of the active parts 3 of arms A and B side by side.
The active faces of arms A and B have bristles 6, said bristles being organized in the form of tufts of bristles 6.
There may be free spaces 8 between each tuft of bristles 6.
The active faces of arms A and B can comprise rows 7 of tufts of bristles 6.
There may be free spaces 8 between each row 7 of tufts of bristles 6.
The rows 7 of tufts of bristles 6 may be parallel to one another.
Thus, the keratinous hair fibres can be placed between the tufts of bristles so as to form waves.
The rows 7 of tufts of bristles 6 of the active face 4 of arm A are complementary to the rows 7 of tufts of bristles 6 of the active face 4 of arm B.
To use the tongs, the user can place a lock of keratinous hair fibres between arms A and B at the active parts thereof when the tongs are in the spaced-apart position (i.e. the active parts 3 are at a distance from one another).
The user then applies a pressing force to the tongs so as to bring the tongs into the brought-together position (i.e. the active parts are brought towards one another).
In the brought-together position of the active parts, said bristles of the arms A and B intermingle so as to grip said keratinous hair fibres without blocking them.
The user can slide the tongs along the lock, i.e. from the root to the tip of the lock, while maintaining a certain pressing force.
The tongs can be repeatedly passed along the locks of keratinous hair fibres.

**Protocol**

The composition for dyeing keratinous hair fibres and the optional composition D described above may be used on dry or wet keratinous hair fibres, and also on any type of fair or dark, natural or coloured, permanent-waved, bleached or relaxed keratinous hair fibres.
According to a preferred embodiment, the dyeing composition and composition D are applied simultaneously to the keratinous hair fibres.
According to another preferred embodiment, composition D is applied to the keratinous hair fibres after the dyeing composition is applied to the keratinous hair fibres.
According to another preferred embodiment, composition D is applied to the keratinous hair fibres before the composition for dyeing keratinous hair fibres is applied.
According to a particular embodiment of the invention, the keratinous hair fibres, such as the hair, are washed before applying the composition for dyeing keratinous hair fibres and the optional composition D.
Preferably, a washing, rinsing, draining or drying step is performed after the dyeing composition is applied to the keratinous hair fibres and optionally before applying composition D to the keratinous hair fibres.
The application of the composition for dyeing keratinous hair fibres and the optional composition D to the keratinous hair fibres is generally performed at room temperature (between 15 and 25°C).
According to a preferred embodiment, the application of the composition for dyeing keratinous hair fibres and the optional composition D to the keratinous hair fibres is performed at a temperature of between 15°C and 50°C, more preferentially between 20°C and 40°C.
After applying the dyeing composition to the keratinous hair fibres, it is possible to wait for between 1 minute and 6 hours, in particular between 1 minute and 2 hours, more particularly between 1 minute and 1 hour, more preferentially between 1 minute and 30 minutes, before, for example, applying composition D to the keratinous hair fibres or, for

example, a washing, rinsing, draining or drying step.

Preferably, there is no leave-on time after applying the dyeing composition to the keratinous hair fibres and before applying composition D to the keratinous hair fibres.

After applying the dyeing composition and the optional composition D, the fibres may be left to dry or may be dried, for example at a temperature of greater than or equal to 30°C. The process according to the invention may thus comprise a step of applying heat to the keratinous hair fibres using a heating tool.

The heat application step of the process of the invention can be carried out using a hood, a hairdryer, a straightening iron or a curling iron, a Climazon...

Preferably, the heat application step of the process of the invention is performed using a hairdryer.

When the process of the invention involves a step of applying heat to the keratinous hair fibres, the step of applying heat to the keratinous hair fibres takes place after applying the composition for dyeing keratinous hair fibres and the optional composition D to the keratinous hair fibres.

During the step of applying heat to the keratinous hair fibres, a mechanical action may be exerted on the locks, such as combing, brushing or running the fingers through.

When the step of applying heat to the keratinous hair fibres is performed using a hood or a hairdryer, the temperature is preferably between 30°C and 110°C, preferentially between 50°C and 90°C.

When the step of applying heat to the keratinous hair fibres is performed using a straightening iron, the temperature is preferably between 110°C and 220°C, preferably between 140°C and 200°C.

In a particular variant, the process of the invention involves a step (b1) of applying heat using a hood, a hairdryer or a Climazon, preferably a hairdryer, and a step (b2) of applying heat using a straightening or curling iron, preferably a straightening iron.

Step (b1) may be performed before step (b2).

During step (b1), also referred to as the drying step, the keratinous hair fibres may be dried, for example at a temperature of greater than or equal to 30°C. According to a particular embodiment, this temperature is greater than 40°C. According to a particular embodiment, this temperature is greater than 45°C and less than 110°C.

Preferably, if the keratinous hair fibres are dried, they are dried, in addition to a supply of heat, with a flow of air. This flow of air during drying makes it possible to improve the strand separation of the coating.

During the drying, a mechanical action may be exerted on the locks, such as combing, brushing or running the fingers through.

During step (b2), the passage of the straightening or curling iron, preferably the straightening iron, may be performed at a temperature ranging from 110°C to 220°C, preferably between 140°C and 200°C.

After the heating step, a shaping step may be performed, for example with a straightening iron; the temperature for the shaping step is between 110°C and 220°C, preferably between 140°C and 200°C.

Preferably, the step of applying the dyeing composition to the keratinous hair fibres is repeated several times.

According to a preferred embodiment, the process of the invention is a process for removing colour from keratinous hair fibres which have been previously dyed, particularly comprising a step of extemporaneous mixing, at the time of use, of at least two compositions A and B to obtain the composition for dyeing keratinous hair fibres as defined above and of applying the dyeing composition to the keratinous hair fibres, with:

- composition A comprising at least one (poly)carbodiimide compound as described previously; and
- composition B comprising at least one colouring agent chosen from pigments, direct dyes and mixtures thereof.

According to a preferred embodiment, the process of the invention is a process for removing colour from keratinous hair fibres which have been previously dyed, particularly comprising a step of extemporaneous mixing, at the time of use, of at least two compositions A and B to obtain the composition for dyeing keratinous hair fibres as defined above and of applying the dyeing composition to the keratinous hair fibres, with:

- composition A comprising at least one (poly)carbodiimide compound as described previously;
- composition B comprising at least one compound, other than the associative polymers as described previously, having at least one carboxylic acid group as described previously; composition A and/or composition B comprising at least one colouring agent chosen from pigments, direct dyes and mixtures thereof.

According to another preferred embodiment, the process of the invention is a process for removing colour from keratinous hair fibres which have been previously dyed, particularly comprising a step of extemporaneous mixing, at the time of use, of at least two compositions A and B to obtain the composition for dyeing keratinous hair fibres as defined above and of applying the dyeing composition to the keratinous hair fibres, with:

- composition A comprising at least one (poly)carbodiimide compound as described previously;

- composition B comprising at least one compound, other than the associative polymers as described previously, having at least one carboxylic acid group as described previously; composition A and/or composition B comprising at least one colouring agent chosen from pigments, direct dyes and mixtures thereof; and

a composition D as described previously being applied to the keratinous hair fibres before and/or after, preferably after, the application of the mixture of compositions A and B to the keratinous hair fibres.

Preferably, compositions A and B are mixed preferably less than 15 minutes before application to the keratinous hair fibres, more preferentially less than 10 minutes before application, better still less than 5 minutes before application.

The weight ratio between composition A and composition B preferably ranges from 0.1 to 10, preferentially from 0.2 to 5 and better still from 0.5 to 2, or even from 0.6 to 1.5.

In a particular embodiment, the weight ratio between composition A and composition B is equal to 1.

Preferably, composition A comprises at least one aminosilicone as described previously. Preferably, composition A comprises at least one non-carboxylic anionic thickener as described previously.

Preferably, composition B comprises at least one aminosilicone as described previously. Preferably, composition B comprises at least one associative polymer, other than the non-carboxylic anionic thickeners, as described previously.

Preferably, composition B comprises at least one compound, other than the associative polymers, having at least one carboxylic acid group as described previously.

The total amount of the (poly)carbodiimide compound(s) preferably ranges from 0.01% to 40% by weight, more preferentially from 0.1% to 30% by weight, better still from 0.5% to 20% by weight and even more preferentially from 1% to 12% by weight relative to the total weight of composition A.

The total amount of the compound(s) having at least one carboxylic group preferably ranges from 2% to 60% by weight, more preferentially from 5% to 40% by weight and better still from 5% to 20% by weight relative to the total weight of composition B.

The aminosilicone(s) may be present in a total amount ranging from 0.01% to 20%, preferably from 0.05% to 15%, more preferentially from 0.1% to 10% and even more preferentially still from 0.5% to 5% by weight relative to the total weight of composition A and/or composition B.

The non-carboxylic anionic thickener(s) may be present in a total amount ranging from 0.01% to 20% by weight, preferably from 0.1% to 10% by weight, better still from 0.1% to 5% by weight, and even better still from 0.1% to 3% by weight, relative to the total weight of composition A.

Next, as indicated previously, the process for removing colour from keratinous hair fibres which have been previously dyed according to the invention comprises the application of at least one colour-removing composition, as defined above, to keratinous hair fibres which have been previously dyed using at least one composition for dyeing keratinous hair fibres as defined above.

Preferably, the colour-removing composition is left on for 30 seconds to 60 minutes, preferentially from 1 to 40 minutes, more preferentially from 1 to 30 minutes and better still from 2 to 20 minutes.

According to a particular embodiment, the process according to the invention also comprises a step of massaging the keratinous hair fibres, after the application of the colour-removing composition.

During this massaging step, a particular tool such as an exfoliating glove may be used.

Preferably, during a massaging step, the fingers are passed along the lock five times. The total duration of the five passes may range from 30 seconds to 2 minutes, for example, per 1 g of lock.

The step of massaging the keratinous hair fibres may be repeated several times, for example twice, optionally with an intermediate leave-on time.

The step of applying the colour-removing composition may be repeated several times, optionally with intermediate rinsing.

The time between the step of applying the composition for dyeing keratinous hair fibres and the step of applying the colour-removing composition may range from a few minutes to several days, for example several tens of days. Preferably, the time between the step of applying the composition for dyeing keratinous hair fibres and the step of applying the colour-removing composition ranges from 1 hour to 30 days, more preferentially from 1 day to 15 days.

The optional massaging step may subsequently be followed by a step of washing the keratinous hair fibres, for example using a shampoo. The keratinous hair fibres may then be massaged, rinsed and dried.

Preferably, there is no step of washing, rinsing, draining or drying of the keratinous hair fibres between applying the colour-removing composition and applying the tongs as described previously.

Following the application of a colour-removing composition as described above, the process according to the invention comprises applying, to said keratinous hair fibres, tongs as described previously.

The time between the step of applying the colour-removing composition and the step of applying the tongs may range from a few seconds to a few minutes, preferably from 30 seconds to 60 minutes, preferentially from 1 to 40 minutes, more preferentially from 1 to 30 minutes, better still from 2 to 20 minutes.

Preferably, the tongs as described previously are applied from the root to the tip of the keratinous hair fibres.

Preferably, from 10 to 150 passes of the tongs as described previously are applied along the keratinous hair fibres; more preferentially, from 10 to 100 passes of the tongs as described previously are applied along the keratinous hair fibres.

The tongs as described previously can be applied to wet or dry keratinous hair fibres.

Preferably, the tongs as described previously are applied to wet keratinous hair fibres.

The step of applying the tongs as described previously may subsequently be followed by a step of washing the keratinous hair fibres, for example using a shampoo. The keratinous hair fibres may then be massaged, rinsed and dried.

Preferably, the process according to the invention comprises a step of washing the keratinous hair fibres, for example by means of shampoo, following the application of the tongs to the keratinous hair fibres.

Preferably, the invention is a process for removing colour from keratinous hair fibres which have been previously dyed, particularly comprising:

i) applying, to the keratinous hair fibres, the composition for dyeing keratinous hair fibres as described above, then
ii) optionally a leave-on time of said dyeing composition on the fibres of from 1 minute to 30 minutes, preferably from 1 to 20 minutes, then
iii) optionally a step of washing, rinsing, draining or drying said fibres, then
iv) optionally applying, to the keratinous hair fibres, a composition D comprising at least one silicone compound comprising at least one carboxylic group as described previously; then
v) optionally a leave-on time of said composition D on the keratinous hair fibres of from 1 minute to 30 minutes, preferably from 1 to 20 minutes, and then
vi) optionally a step of washing, rinsing, draining or drying the keratinous hair fibres,
vii) applying, to the previously dyed keratinous hair fibres, the colour-removing composition as described above,
viii) optionally a step of massaging the keratinous hair fibres, preferably at the root of the keratinous hair fibres,
ix) optionally a leave-on time of said dyeing composition on the fibres of from 1 minute to 30 minutes, preferably from 1 to 20 minutes, then
x) applying, to the keratinous hair fibres, one or more passes of tongs as described above, then
xi) optionally a step of washing, rinsing, draining or drying the keratinous hair fibres.

According to a preferred embodiment, the process for removing the colour from previously dyed keratinous hair fibres is a process for removing the colour from keratinous hair fibres comprising, in particular:

i) a step of extemporaneous mixing, at the time of use, of at least two compositions A and B to obtain the composition for dyeing keratinous hair fibres as defined above, with:

- composition A comprising at least one (poly)carbodiimide compound as described previously;
- composition B comprising at least one colouring agent chosen from pigments, direct dyes and mixtures thereof; then

ii) applying said dyeing composition to the keratinous hair fibres, then
iii) optionally applying, to the keratinous hair fibres, a composition D comprising at least one silicone compound comprising at least one carboxylic group as described previously; then
iv) applying, to the keratinous hair fibres previously dyed by means of said dyeing composition, at least one colour-removing composition as described previously; then
v) optionally a step of massaging the keratinous hair fibres, preferably at the root of the keratinous hair fibres,
vi) applying, to said keratinous hair fibres, tongs as described above, then
vii) optionally a step of washing the keratinous hair fibres, for example using a shampoo.

According to a preferred embodiment, the process for removing the colour from previously dyed keratinous hair fibres is a process for removing the colour from keratinous hair fibres comprising, in particular:

i) a step of extemporaneous mixing, at the time of use, of at least two compositions A and B to obtain the composition for dyeing keratinous hair fibres as defined above, with:

- composition A comprising at least one (poly)carbodiimide compound as described previously;
- composition B comprising at least one compound, other than the associative polymers as described previously, having at least one carboxylic acid group as described previously; composition A and/or composition B comprising at least one colouring agent chosen from pigments, direct dyes and mixtures thereof; then

ii) applying said dyeing composition to the keratinous hair fibres, then
iii) optionally applying, to the keratinous hair fibres, a composition D comprising at least one silicone compound comprising at least one carboxylic group as described previously; then
iv) applying, to the keratinous hair fibres previously dyed by means of said dyeing composition, at least one colour-removing composition as described previously; then
v) optionally a step of massaging the keratinous hair fibres, preferably at the root of the keratinous hair fibres,
vi) applying, to said keratinous hair fibres, tongs as described above, then
vii) optionally a step of washing the keratinous hair fibres, for example using a shampoo.

**Multi-compartment device (kit)**

The present invention also relates to a device for dyeing and removing colour from keratinous hair fibres such as the hair, comprising a plurality of compartments containing:

- in a first compartment, a dyeing composition comprising:
- at least one (poly)carbodiimide compound as described previously;
- at least one colouring agent chosen from pigments, direct dyes and mixtures thereof,
- in a second compartment, a composition for removing colour from keratinous hair fibres previously dyed by means of said dyeing composition comprising at least one alkyl or alkylene carbonate, as defined previously, and
- tongs as described previously.

According to a preferred embodiment, the device for dyeing and removing colour from keratinous hair fibres such as the hair comprises a plurality of compartments containing:

- in a first compartment, a composition A comprising at least one (poly)carbodiimide compound as described previously;
- in a second compartment, a composition B comprising at least one colouring agent chosen from pigments, direct dyes and mixtures thereof,
- in a third compartment, a composition for removing colour from keratinous hair fibres previously dyed by means of said dyeing composition comprising at least one alkyl or alkylene carbonate, as defined previously, and
- tongs as described previously.

According to a preferred embodiment, the device for dyeing and removing colour from keratinous hair fibres such as the hair comprises a plurality of compartments containing:

- in a first compartment, a composition A comprising at least one (poly)carbodiimide compound as described previously;
- in a second compartment, a composition B comprising at least one colouring agent chosen from pigments, direct dyes and mixtures thereof,
- in a third compartment, a composition for removing colour from keratinous hair fibres previously dyed by means of said dyeing composition comprising at least one alkyl or alkylene carbonate, as defined previously,
- tongs as described previously, and
- optionally in a fourth compartment a composition D comprising at least one silicone compound comprising at least one carboxylic group as described previously.

According to a preferred embodiment, the device for dyeing and removing colour from keratinous hair fibres such as the hair comprises a plurality of compartments containing:

- in a first compartment, a composition A comprising at least one (poly)carbodiimide compound as described previously;
- in a second compartment, a composition B comprising at least one compound, other than the associative polymers, having at least one carboxylic acid group as described previously,
  composition A and/or composition B comprising at least one colouring agent chosen from pigments, direct dyes and mixtures thereof,
- in a third compartment, a composition for removing colour from keratinous hair fibres previously dyed by means of said dyeing composition comprising at least one alkyl or alkylene carbonate, as defined previously,
- tongs as described previously, and
- optionally in a fourth compartment a composition D comprising at least one silicone compound comprising at least one

carboxylic group as described previously.

The present invention will now be described more specifically by means of examples. The examples make it possible to support specific features, variants and preferred embodiments of the invention.

**EXAMPLES**

The (poly)carbodiimide(s) of the invention are accessible via synthetic methods known to those skilled in the art starting from commercial products or reagents that can be synthesized according to chemical reactions that are also known to those skilled in the art. Mention may be made, for example, of the book Sciences of Synthesis - Houben - Weyl Methods of Molecular Transformations, 2005, Georg Thiem Verlag Kg, Rudigerstrasse 14, D-70469 Stuttgart, or the American patent US 4 284 730 or the Canadian patent application CA 2 509 861.

More particularly, the process for preparing the (poly)carbodiimides of the invention involves, in a first step, a diisocyanate reagent (1):

$$O{=}C{=}N\text{-}L_1\text{-}N{=}C{=}O \qquad (1),$$

in which formula (1) $L_1$ is as defined previously, which reacts in the presence of a carboimidation catalyst (2) such as those described in US 4 284 730, particularly phosphorus-based catalysts particularly chosen from phospholene oxides and phospholene sulfoxides, diaza- and oxaza-phospholanes, preferably under an inert atmosphere (nitrogen or argon), and in particular in a polar solvent which is preferably aprotic such as THF, glyme, diglyme, 1,4-dioxane or DMF, at a temperature between room temperature and the reflux temperature of the solvent, preferably approximately 140°C; to give the carbodiimide diisocyanate compound (3):

$$O{=}C{=}N\text{-}L_1\text{-}(N{=}C{=}N\text{-}L_1)_n\text{-}N{=}C{=}O \qquad (3),$$

in which formula (3) $L_1$ and n are as defined previously. Benzoyl halogen such as benzoyl chloride may be added to deactivate the catalyst.

To obtain "symmetrical" (poly)carbodiimides, during the second step of the preparation process, compound (3) reacts with 1 molar equivalent (1 eq.) of nucleophilic reagent $R_1$-$X_1$-H then 0.5 eq. of reagent H-E-H with $R_1$, $X_1$ and E as defined previously, to give the "symmetrical" compound (4) according to the invention:

$$[R_1\text{-}X_1\text{-}C(O)\text{-}NH\text{-}L_1\text{-}(N{=}C{=}N\text{-}L_1)_n\text{-}NH\text{-}C(O)]_2\text{-}E \qquad (4),$$

in which formula (4) $R_1$, $X_1$, $L_1$, n and E are as defined previously. According to one variant to obtain compound (4) from (3), it is possible first to add 0.5 eq. of reagent H-EH and then 1 eq. of reagent $R_1$-$X_1$-H.

To obtain "dissymmetrical" (poly)carbodiimides, during the second step of the preparation process, compound (3) reacts with 1 molar equivalent (1 eq.) of nucleophilic reagent $R_1$-$X_1$-H, then 1 eq. of reagent H-E-H with $R_1$, $X_1$ and E as defined above, to give compound (5):

$$R_1\text{-}X_1\text{-}C(O)\text{-}NH\text{-}L_1\text{-}(N{=}C{=}N\text{-}L_1)_n\text{-}NH\text{-}C(O)\text{-}E\text{-}H \qquad (5),$$

in which formula (5) $R_1$, $X_1$, $L_1$, n and E are as defined previously.

According to one variant to obtain compound (5) from (3), it is possible first to add 1 eq. of reagent $R_1$-$X_1$-H, then 0.5 eq. of reagent H-E-H.

During a third step, compound (5) reacts with 1 eq. of compound (6) $R_2$-$X_2$-C(O)-NH-$L_1$-(N=C=N-$L_1$)$_z$-N=C=O (6); said compound (6) is prepared beforehand from compound (3'):

$$O{=}C{=}N\text{-}L_1\text{-}(N{=}C{=}N\text{-}L_1)_z\text{-}N{=}C{=}O \qquad (3'),$$

in which formula (3') $L_1$ and z are as defined previously, which reacts with 1 eq. of nucleophilic reagent $R_2$-$X_2$-H with $L_1$, $R_2$, $X_2$ and z as defined previously, to give the dissymmetrical compound (7):

$$R_1\text{-}X_1\text{-}C(O)\text{-}NH\text{-}L_1\text{-}(N{=}C{=}N\text{-}L_1)_n\text{-}NH\text{-}C(O)\text{-}E\text{-}C(O)\text{-}NH\text{-}L_1\text{--}(N{=}C{=}N\text{-}L_1)_z\text{-}NH\text{-}C(O)\text{-}X_2\text{-}R_2 \qquad (7),$$

in which formula (7) $R_1$, $X_1$, $L_1$, $R_2$, $X_2$, n, z and E are as defined previously.

It is also possible to react 1 molar equivalent of compound O=C=N-$L_1$-(N=C=N-$L_1$)$_z$-N=C=O (3') with 1/w molar

equivalent of H-E-H, then 1 eq. of nucleophilic reagent $R_2$-$X_2$-H to give compound (8):

$$H\text{-}[E\text{-}C(O)\text{-}NH\text{-}L_1\text{-}(N{=}C{=}N\text{-}L_1)_z]_w\text{-}NH\text{-}C(O)\text{-}X_2\text{-}R_2 \qquad (8),$$

in which formula (8) $L_1$, $R_2$, $X_2$, z and E are as defined previously, and w is an integer between 1 and 3; more preferentially, w = 1.

This last compound (8) can then react with 1 eq. of compound (4'): $R_1$-$X_1$-C(O)-NH-$L_1$-(N=C=N-$L_1$)$_n$-N=C=O (4'), (said compound (4') being able to be synthesized by reaction of 0.5 eq. of nucleophilic reagent $R_1$-$X_1$-H with 1 equivalent of compound (3)), to give the (poly)carbodiimide (9) of the invention:

$$R_1\text{-}X_1\text{-}C(O)\text{-}NH\text{-}L_1\text{-}(N{=}C{=}N\text{-}L_1)_n\text{-}NH\text{-}C(O)\text{-}[E\text{-}C(O)\text{-}NH\text{-}L_1\text{-}(N{=}C{=}N\text{-}L_1)_z]_w\text{-}NH \quad C(O)\text{-} \quad X_2\text{-}R_2 \qquad (9),$$

in which formula (9) $L_1$, $R_1$, $X_1$, $R_2$, $X_2$, n, z, w and E are as defined previously.

The (poly)carbodiimide compounds, and similarly all the reaction intermediates and reagents, may be purified via conventional methods known to those skilled in the art, such as extraction with water and water-immiscible organic solvent, precipitation, centrifugation, filtration and/or chromatography.

**Example 1: Process for synthesizing the (poly)carbodiimide compound**

50 g of 4,4'-dicyclohexylmethane diisocyanate and 0.5 g of 4,5-dihydro-3-methyl-1-phenyl-1H-phosphole 1-oxide were placed with stirring in a 500 ml three-necked roundbottomed flask equipped with a thermometer, a stirrer and a reflux tube.

The reaction medium was heated at 140°C under nitrogen for 4 hours, the reaction being monitored by infrared spectroscopy by means of the absorption of the isocyanate functions between 2200 and 2300 $cm^{-1}$, and then cooled to 120°C.

A mixture of 5.3 g of polyethylene glycol monomethyl ether and 1.2 g of 1,4-butanediol are introduced with stirring into the reaction medium. The temperature of 120°C is maintained until the isocyanate functions have totally disappeared, monitored by infrared spectroscopy at 2200-2300 $cm^{-1}$, and is then cooled to room temperature.

After cooling to room temperature, the reaction medium is poured dropwise with vigorous stirring into a 500 ml glass beaker containing 85 g of distilled water, to give the desired product in the form of a translucent yellow liquid.

**Example 2:**

The compositions as described below were prepared: the amounts are expressed as g of starting material as obtained/100 g, unless otherwise mentioned.

Dye composition:

[Table 1]

| Composition | A |
|---|---|
| Polycarbodiimide(1) | 24 |
| Hydroxyethyl acrylate/sodium acryloyldimethyl taurate copolymer (2) | 2 |
| Amodimethicone (and) Trideceth-5 (and) Trideceth-10 (3) | 5 |
| Ethanol | 20 |
| Water | qs 100 |

(1) synthesized according to the synthetic process described in Example 1 (containing 40% active material in water),
(2) sold by the company SEPPIC under the name Sepinov EMT10 (containing 90% active material),
(3) sold by the company Wacker under the name Belsil ADM LOG 1 (containing 15% active material).

[Table 2]

| Composition | B |
|---|---|
| Acrylates copolymer (4) | 40 |
| Acrylates/Steareth-20 methacrylate copolymer (5) | 3 |
| Amodimethicone (and) Trideceth-5 (and) Trideceth-10 (3) | 5 |
| Ethanol | 3.8 |
| Mica and red iron oxide (CI77491) | 9.3 |
| Yellow iron oxide (CI 77492) | 2.7 |
| Preserving agent(s) | qs |
| Water | qs 100 |
| (4) sold by the company Daito Kasei Kogyo under the trade name Daitosol 3000SLPN-PE1 (aqueous dispersion containing 30% active material)<br>(5) sold by the company Röhm & Haas under the trade name Aculyn 22® (oxyalkylenated methacrylic acid/ethyl acrylate/stearyl methacrylate terpolymer containing 30% active material) | |

Composition A is mixed with composition B in a 50/50 mass ratio to obtain the dyeing composition C.
Next, composition D as described below was prepared: the amounts are expressed as g of starting material as obtained/100 g.

[Table 3]

| Composition | D |
|---|---|
| Bis-carboxydecyl Dimethicone (6) | 2 |
| Isododecane | qs 100 |
| (6) sold by the company Momentive Performance Materials under the trade name Silform INX | |

Colour-removing composition:
In parallel, the colour-removing composition according to the invention is prepared and described in the table below: the amounts are expressed as g of starting material as obtained/100 g.

[Table 4]

| Composition | E |
|---|---|
| Propylene carbonate | 39.5 |
| PPG-3 Methyl Ether | 10 |
| Ammonium Polyacryloyldimethyl Taurate(7) | 0.5 |
| Propylene glycol | qs 100 |
| (7) sold by the company Clariant under the trade name Hostacerin AMPS | |

Dyeing protocol:

Dyeing composition C is applied using a small brush to locks of dry natural hair containing 90% white hairs, at an amount of 0.8 g of composition per gram of lock. The locks of hair are then disentangled and dried with a hair dryer at medium heat.
Next, composition D is applied to said locks of hair previously treated with composition C, at an amount of 0.5 g of composition per gram of lock.
The locks of hair are then disentangled and dried with a hair dryer at medium heat.
The locks of hair are then subjected to a process of accelerated ageing by placing them in an oven at a temperature of 60°C for 48 hours before applying the colour-removing composition.
This step of accelerated ageing makes it possible to simulate the natural ageing of the coloured coating on the

hair *in vivo;* this ageing corresponds to 2 weeks at room temperature *in vivo.*

Protocol for removing colour:

Three different processes for removing colour from the locks coloured with composition C + D were carried out:

- a comparative process 1 in which only the colour-removing composition E is applied to the locks of dyed hair;
- a comparative process 2 in which only the tongs as described above are applied to the locks of dyed hair (Brosse Pince Ceramik Defrisage from the company Anself);
- a process 3 according to the invention in which the colour-removing composition E is applied to the lock of dyed hair followed by the application of the tongs as described above to the locks of hair (Brosse Pince Ceramik Defrisage from the company Anself).

In the comparative process 1, the colour-removing composition E is applied to a lock of dyed hair using the fingers at an amount of 2 g of composition per g of lock.
The lock is then combed 6 times using a comb in order to correctly distribute composition E over the lock of hair.
The lock of hair is left for 4 minutes at room temperature, then the shampooing protocol described below is carried out.
In the comparative process 2, water is applied to a lock of dyed hair using the fingers at an amount of 2 g of water per g of lock.
The lock is then combed 6 times using a comb in order to correctly distribute the water over the lock of hair.
The lock of hair is then tonged between the arms A and B of the tongs according to the invention, and 100 passes of the tongs along the lock of hair, from the root to the tip, are then carried out.
The shampooing protocol described below is then carried out.
In the process 3 according to the invention, the colour-removing composition E is applied to a lock of dyed hair using the fingers at an amount of 2 g of composition per g of lock.
The lock is then combed 6 times using a comb in order to correctly distribute composition E over the lock of hair.
The lock of hair is then tonged between the arms A and B of the tongs according to the invention, and 100 passes of the tongs along the lock of hair, from the root to the tip, are then carried out.
The shampooing protocol described below is then carried out.

Shampooing protocol:

The locks of hair are combed, moistened with water at 35°C and then passed between the fingers five times for 5 seconds. The locks of hair are then squeezed dry between two fingers.
A standard shampoo (Garnier Ultra Doux) is applied uniformly to the dyed locks at an amount of 0.4 g of standard shampoo per gram of locks, the locks of hair being massaged gently along the length (6 passes) for 15 seconds, from the root to the tip.
The locks of hair are then placed on a watch glass and left to stand for 1 minute.
Next, the locks of hair are rinsed with water while passing the locks between the fingers (15 passes). The locks of hair are then squeezed dry between two fingers before the next shampoo wash.
The locks of hair are combed and dried with a hairdryer.

Results

The persistence of the colour of the locks was evaluated in the CIE L*a*b* system, using a Minolta Spectrophotometer CM3600A colorimeter (illuminant D65, angle 10°, specular component included).
In this L*a*b* system, L* represents the intensity of the colour, a* indicates the green/red colour axis and b* the blue/yellow colour axis.
The persistence of the colouring is evaluated by the colour difference ΔE between the dyed locks before the step of removing the colour, then after having undergone the protocol for removing the colour from previously dyed locks of hair described above. The lower the value of ΔE, the more the colour is persistent in the face of the protocol for removing the colour from previously dyed locks of hair described above.
The ΔE value is calculated according to the following equation:

$$\Delta E = \sqrt{(L^* - L_o^*)^2 + (a^* - a_o^*)^2 + (b^* - b_o^*)^2}$$

In this equation, L*a*b* represent the values measured after dyeing the hair and after the colour removal step, and L0*a0*b0* represent the values measured after dyeing the hair but before the colour removal step.

[Table 5]

| Protocols | L* | a* | b* | ΔE |
|---|---|---|---|---|
| Dyed control locks | 43.4 | 20.3 | 28.7 | - |
| Colour-removing process 1 (comparative) | 49.9 | 19.7 | 27.8 | 6.6 |
| Colour-removing process 2 (comparative) | 50.4 | 19.4 | 27.4 | 7.2 |
| Colour-removing process 3 (invention) | 65.8 | 1.8 | 15.1 | 32.1 |

It is observed that the process for removing the colour from dyed keratinous hair fibres according to the invention has an excellent ability to remove colour from keratinous hair fibres compared to the comparative processes.

Thus, the process for removing colour from keratinous hair fibres according to the invention makes it possible to obtain efficient removal of the colour from a lock of hair which has been previously dyed using a composition for dyeing keratinous hair fibres comprising a (poly)carbodiimide compound and a colouring agent chosen from pigments, direct dyes and mixtures thereof.

## Claims

1. Process for removing the colour from keratinous hair fibres previously dyed by means of at least one composition for dyeing keratinous hair fibres, comprising:

    - at least one (poly)carbodiimide compound; and
    - at least one colouring agent chosen from pigments, direct dyes and mixtures thereof;

    said process comprising the following steps:

    i) applying, to the keratinous hair fibres previously dyed by means of said dyeing composition, at least one colour-removing composition, comprising:

    - at least one alkyl or alkylene carbonate; and

    ii) applying, to said keratinous hair fibres, tongs (1) comprising two arms A and B, between which arms said keratinous hair fibres pass,

    each arm A and B comprising a supporting part (2) and an active part (3),
    said arms A and B being movable between a spaced-apart position in which the active parts (3) are at a distance from one another, and a brought-together position in which the active parts (3) are brought towards one another, said active parts (3) comprising an active face (4) and an opposite face (5), said active face (4) having bristles (6), wherein, in the brought-together position of the active parts (3), said bristles (6) of the arms A and B intermingle so as to grip said keratinous hair fibres without blocking them.

2. Process according to Claim 1, **characterized in that** the (poly)carbodiimide compound(s) is (are) chosen from the compounds of formula (I) below:

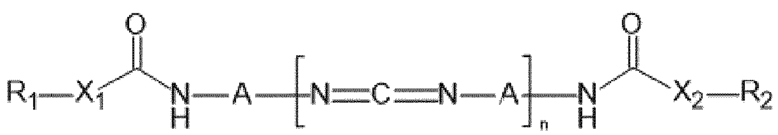

(I)

in which:

- $X_1$ and $X_2$ independently represent an oxygen atom O, a sulfur atom S or an NH group;

- $R_1$ and $R_2$ independently represent a group chosen from a hydrocarbon-based radical, preferably alkyl, optionally interrupted with one or more heteroatom(s), a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatom(s) and with one or more groups chosen from alkoxysilyl, hydroxysilyl, acetoxysilyl, vinylsilyl, acrylalkylsilyl, methacrylalkylsilyl, **crotonylalkylsilyl, carboxyanhydridoalkylsilyl, carboxyalkylsilyl, hydroxyalkylsilyl,** aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

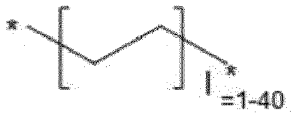
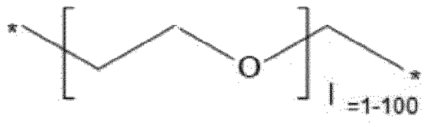
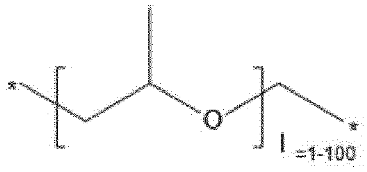
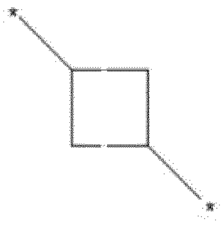
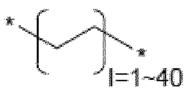
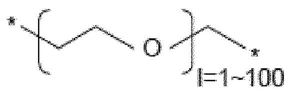
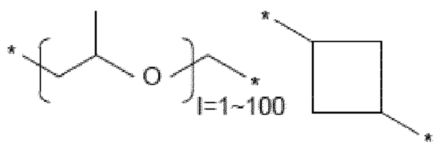
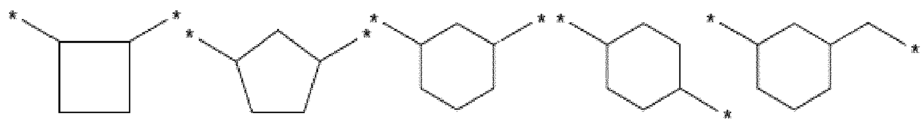
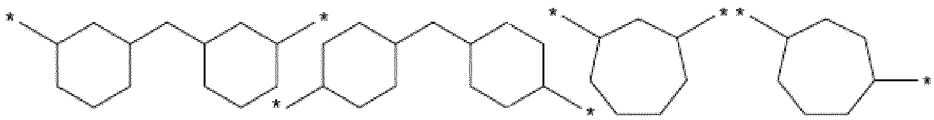
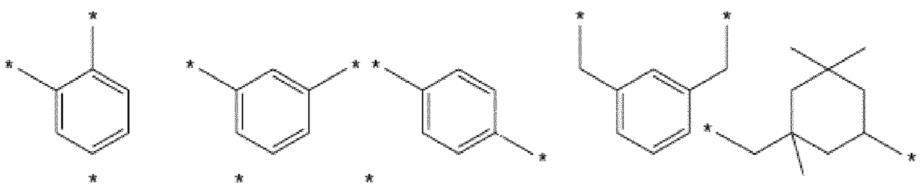
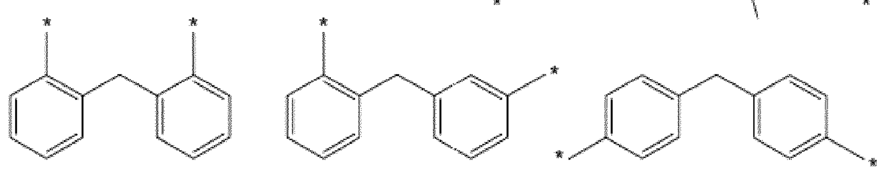

**3.** Process according to Claim 1, **characterized in that** the (poly)carbodiimide compound(s) is (are) chosen from the compounds of formula (II) below:

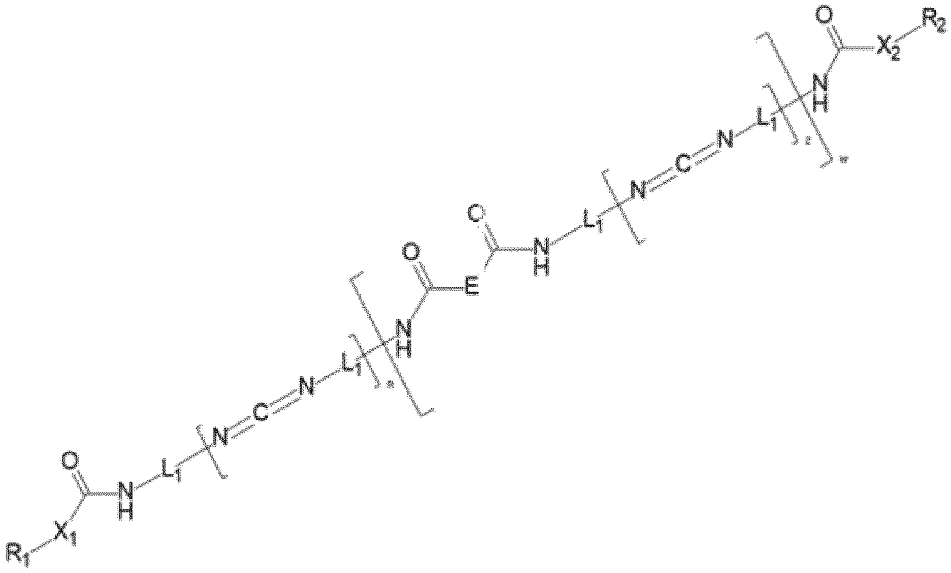

(II),

in which

- $X_1$ and $X_2$ independently represent an oxygen atom O, a sulfur atom S or an NH group;
- $R_1$ and $R_2$ independently represent a hydrocarbon-based radical optionally interrupted with one or more heteroatom(s);
- n and z denote an integer ranging from 1 to 20, with $n+z \geq 2$ and w denoting an integer ranging from 1 to 3;
- $L_1$ independently represents a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- E independently represents a group chosen from:

$$-O-R_3-O-; -S-R_4-S-; -R_5-N(R_6)-R_4-N(R_6)-R_5-,$$

in which $R_3$ and $R_4$ independently represent a divalent hydrocarbon-based radical optionally interrupted with one or more heteroatom(s);
- $R_5$ independently represents a covalent bond or a saturated divalent hydrocarbon-based radical, optionally interrupted with one or more heteroatom(s);
- $R_6$ independently represents a hydrogen atom or a hydrocarbon-based radical, optionally interrupted with one or more heteroatom(s).

4. Process according to Claim 3, **characterized in that** the (poly)carbodiimide compound(s) is (are) chosen from the compounds of formula (II) in which:

- $X_1$ and $X_2$ independently represent an oxygen atom;
- $R_1$ and $R_2$ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with $n+z \geq 2$ and w is equal to 1;
- L1 is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- E independently represents a group chosen from:

$$- -O-R_3-O-; -S-R_4-S-; -R_5-N(R_6)-R_4-N(R_6)-R_5-;$$

in which R3 and R4 are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof;
- when $R_5$ is not a covalent bond, $R_5$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures

thereof; and
- $R_6$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

5. Process according to Claim 3 or 4, **characterized in that** the (poly)carbodiimide compound(s) is (are) chosen from the compounds of formula (II) in which:

- $X_1$ and $X_2$ independently represent an oxygen atom;
- $R_1$ and $R_2$ are, independently, monoalkyl ethers of (poly)alkylene glycol, in which a hydroxyl group has been removed;
- n and z denote an integer ranging from 1 to 20, with $n+z \geq 2$ and w is equal to 1;
- $L_1$ is a $C_3$-$C_{15}$ cycloalkylene radical;
- E independently represents a group chosen from:

$$-O-R_3-O-;\ -S-R_4-S-;\ -R_5-N(R_6)-R_4-N(R_6)-R_5-;$$

in which $R_3$ and $R_4$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof;
- when $R_5$ is not a covalent bond, $R_5$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof; and
- $R_6$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

6. Process according to any one of Claims 3 to 5, **characterized in that** the (poly)carbodiimide compound(s) is (are) chosen from the compounds of formula (II) in which:

- $X_1$ and $X_2$ independently represent an oxygen atom;
- $R_1$ and $R_2$ independently represent the compound of formula (VI) below:

$$R_{13}-[O-CH_2-C(H)(R_{14})]_q- \qquad (VI),$$

in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;
- n and z denote an integer ranging from 2 to 20, with n+z ranging from 4 to 10 and w is equal to 1;
- $L_1$ is a $C_3$-$C_{15}$ cycloalkylene radical such as cyclopentylene, cycloheptylene, cyclohexylene and 4,4-dicyclohexylenemethane, and
- E represents a group $-O-R_3-O-$ wherein $R_3$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatom(s), and mixtures thereof.

7. Process according to any one of Claims 3 to 6, **characterized in that** the (poly)carbodiimide compound(s) is (are) chosen from the compounds of formula (II) in which:

- $X_1$ and $X_2$ independently represent an oxygen atom;
- $R_1$ and $R_2$ independently represent the compound of formula (VI) below:

$$R_{13}-[O-CH_2-C(H)(R_{14})]_q- \qquad (VI),$$

in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;
- n and z denote an integer ranging from 2 to 20, with n+z ranging from 4 to 10 and w is equal to 1;
- $L_1$ is a $C_3$-$C_{15}$ cycloalkylene radical such as cyclopentylene, cycloheptylene, cyclohexylene and 4,4-dicyclohexylenemethane, preferably 4,4-dicyclohexylenemethane; and
- E represents a group $-O-R_3-O-$ in which $R_3$ represents a linear or branched $C_1$-$C_{18}$ alkylene radical such as

methylene, propylene, butylene, ethylene, optionally interrupted with one or more heteroatom(s).

**8.** Process according to any one of Claims 3 to 7, **characterized in that** the (poly)carbodiimide compound(s) is (are) chosen from the compounds of formula (XII) below:

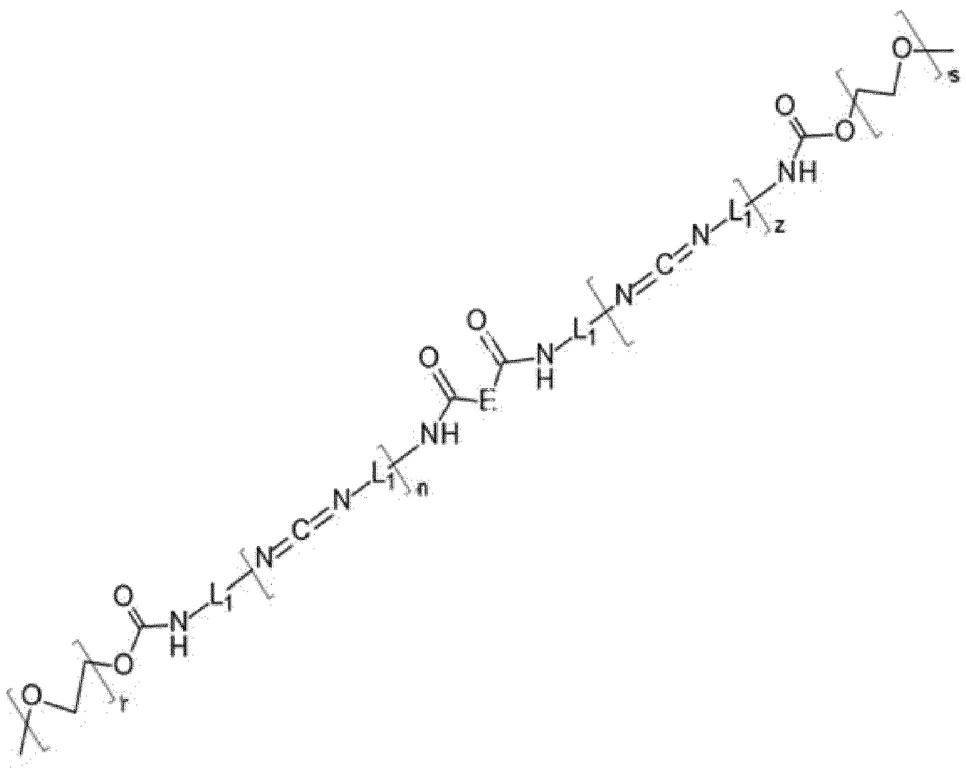

(XII),

in which $L_1$ is 4,4-dicyclohexylenemethane, n and z denote an integer ranging from 2 to 20, with n+z ranging from 4 to 10, E represents a group $-O-R_3-O-$ in which $R_3$ represents a linear or branched $C_1$-Cis alkylene radical such as methylene, propylene, butylene, ethylene, optionally interrupted with one or more heteroatom(s), and r and s denote an integer ranging from 4 to 30.

**9.** Process according to any one of the preceding claims, **characterized in that** the total amount of the (poly) carbodiimide compound(s) ranges from 0.01% to 20% by weight, preferably from 0.1% to 15% by weight, more preferentially from 0.2% to 10% by weight, even more preferentially from 0.5% to 8% by weight, better still from 1% to 6% by weight, relative to the total weight of the dyeing composition.

**10.** Process according to any one of the preceding claims, **characterized in that** the total amount of colouring agent(s) ranges from 0.001% to 20% by weight and preferably from 0.005% to 15% by weight relative to the total weight of the dyeing composition; preferably, the colouring agent(s) are chosen from pigments.

**11.** Process according to any one of the preceding claims, **characterized in that** the alkyl or alkylene carbonate is a $C_1$-$C_{30}$, preferably $C_1$-$C_6$ alkyl carbonate or $C_1$-$C_{30}$, preferably $C_1$-$C_6$ alkylene carbonate; more preferentially, the alkyl or alkylene carbonate is chosen from alkylene carbonates, better still from propylene carbonate, butylene carbonate, pentylene carbonate and mixtures thereof.

**12.** Process according to any one of the preceding claims, **characterized in that** the total content of alkyl or alkylene carbonate ranges from 10% to 60% by weight, preferably from 15% to 50% by weight, relative to the total weight of the colour-removing composition.

**13.** Process according to any one of the preceding claims, **characterized in that** the two arms A and B are articulated about an axis of articulation (Z) and move angularly towards one another in order for the active parts (3) of arms A and B to be in contact with one another.

**14.** Process according to any one of the preceding claims, **characterized in that** said bristles (6) are arranged in the form of tufts of bristles, preferably made from a natural material, such as natural silks, or animal hair, such as squirrel, boar or goat hair.

**15.** Process according to Claim 14, **characterized in that** the active faces of arms A and B have rows (7) of tufts of bristles (6) separated by free spaces (8), the rows (7) of tufts of bristles (6) of the active parts (3) being arranged parallel to one another.

**16.** Process according to any one of the preceding claims, **characterized in that** it comprises a step of washing the keratinous hair fibres, for example by means of shampoo, following the application of the tongs to the keratinous hair fibres.

**17.** Device for dyeing and removing colour from keratinous hair fibres such as the hair, comprising a plurality of compartments containing:

- in a first compartment, a composition A comprising at least one (poly)carbodiimide compound as defined in any one of Claims 2 to 8, and
- in a second compartment, a composition B comprising at least one colouring agent chosen from pigments, direct dyes and mixtures thereof,
- in a third compartment, a composition for removing colour from keratinous hair fibres previously dyed by means of said dyeing composition comprising at least one alkyl or alkylene carbonate, as defined in either one of Claims 11 and 12, and
- tongs (1) as defined in any one of Claims 1 and 13 to 15.

**Patentansprüche**

**1.** Verfahren zur Entfernung von Farbe aus vorher mit mindestens einer Zusammensetzung zum Färben von keratinischen Haarfasern gefärbten keratinischen Haarfasern, wobei die Zusammensetzung Folgendes umfasst:

- mindestens eine (Poly)carbodiimidverbindung und
- mindestens ein Farbmittel, das aus Pigmenten, Direktfarbstoffen und Mischungen davon ausgewählt ist;

wobei das Verfahren die folgenden Schritte umfasst:

i) Aufbringen auf die vorher mit der Färbezusammensetzung gefärbten keratinischen Haarfasern mindestens einer Farbentfernungszusammensetzung, welche Folgendes umfasst:

- mindestens ein Alkyl- oder Alkylencarbonat; und

ii) Anlegen an die keratinischen Haarfasern einer Zange (1) mit zwei Armen A und B, zwischen denen die keratinischen Haarfasern hindurchgehen,

wobei jeder Arm A und B einen tragenden Teil (2) und einen aktiven Teil (3) umfasst,
wobei die Arme A und B zwischen einer beabstandeten Position, in der sich die aktiven Teile (3) in einem Abstand voneinander befinden, und einer zusammengebrachten Position, in der die aktiven Teile (3) aufeinander zugebracht sind, beweglich sind,
wobei die aktiven Teile (3) eine aktive Fläche (4) und eine gegenüberliegende Fläche (5) umfassen, wobei die aktive Fläche (4) Borsten (6) aufweist,
wobei in der zusammengebrachten Position der aktiven Teile (3) die Borsten (6) der Arme A und B so ineinandergreifen, dass sie die keratinischen Haarfasern greifen, ohne sie zu blockieren.

**2.** Verfahren nach Anspruch 1, **dadurch gekennzeichnet, dass** die (Poly)carbodiimidverbindung bzw. die (Poly) carbodiimidverbindungen aus den Verbindungen der folgenden Formel (I) ausgewählt ist bzw. sind:

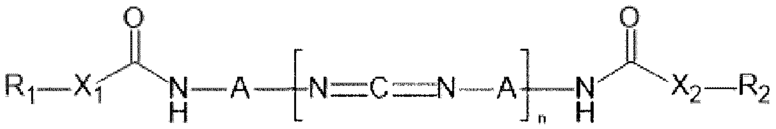

(I)

wobei:

- $X_1$ und $X_2$ unabhängig für ein Sauerstoffatom O, ein Schwefelatom S oder eine NH-Gruppe stehen;
- $R_1$ und $R_2$ unabhängig für eine Gruppe stehen, die aus einem Rest auf Kohlenwasserstoffbasis, vorzugsweise Alkyl, der gegebenenfalls durch ein oder mehrere Heteroatome unterbrochen ist, eine Gruppe, die aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen ausgewählt ist, und einen Rest auf Kohlenwasserstoffbasis, vorzugsweise Alkyl, der gegebenenfalls durch ein oder mehrere Heteroatome und durch eine oder mehrere Gruppen, die aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen ausgewählt sind, unterbrochen ist, ausgewählt ist;
- n eine ganze Zahl im Bereich von 1 bis 1000 bedeutet; und
- A für ein Monomer steht, das aus den folgenden Verbindungen ausgewählt ist:

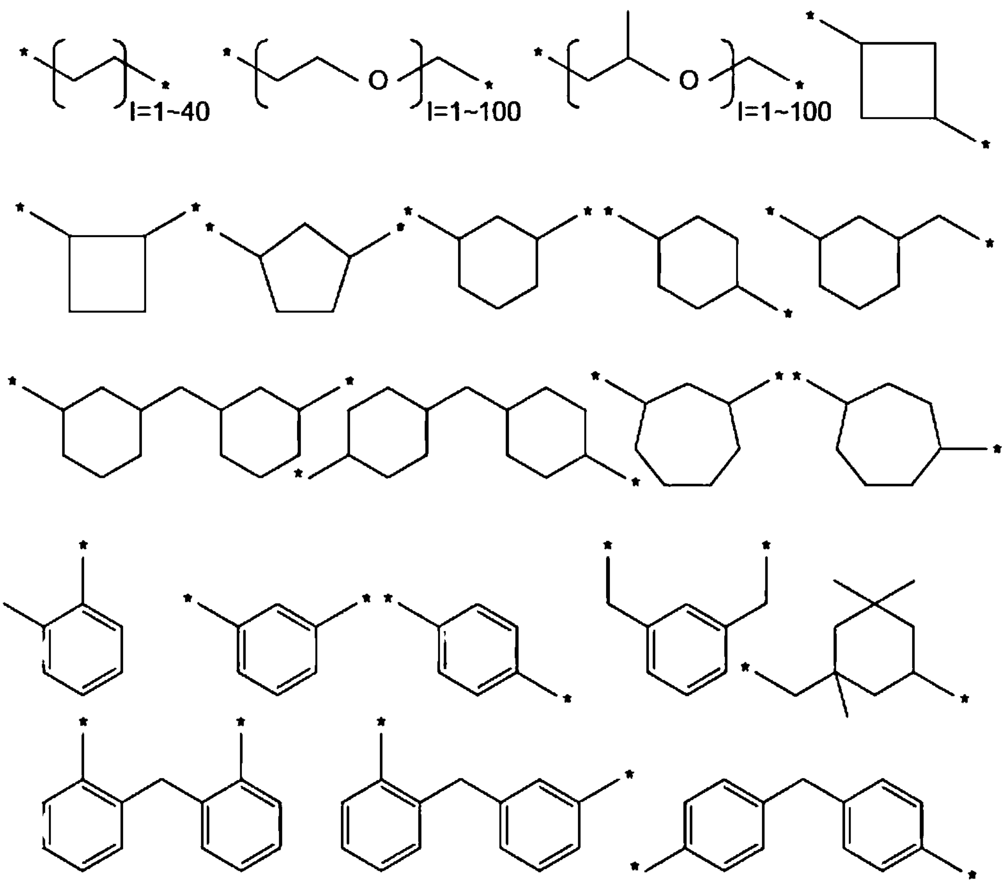

**3.** Verfahren nach Anspruch 1, **dadurch gekennzeichnet, dass** die (Poly)carbodiimidverbindung bzw. die (Poly) carbodiimidverbindungen aus den Verbindungen der folgenden Formel (II) ausgewählt ist bzw. sind:

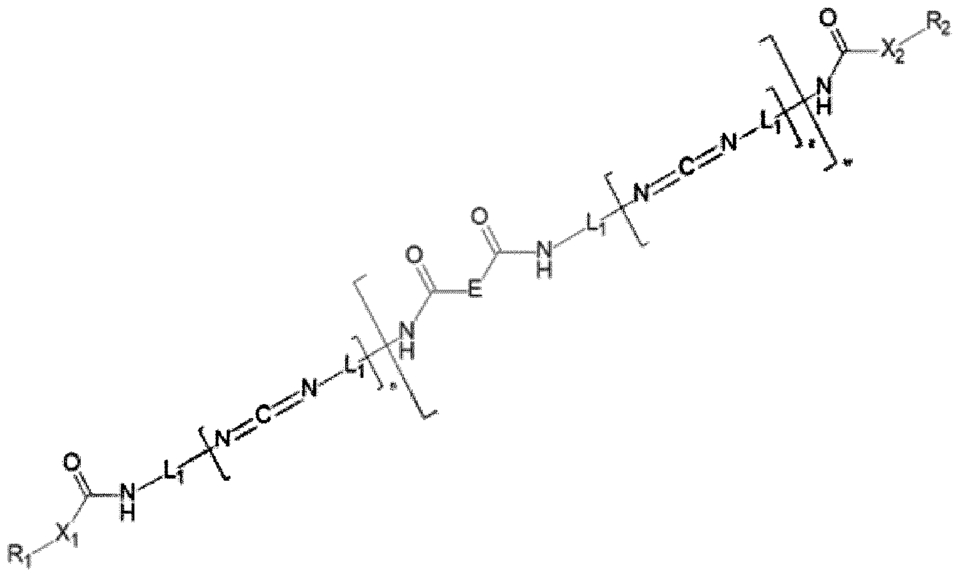

(II),

wobei

- $X_1$ und $X_2$ unabhängig für ein Sauerstoffatom O, ein Schwefelatom S oder eine NH-Gruppe stehen;
- $R_1$ und $R_2$ unabhängig für einen Rest auf Kohlenwasserstoffbasis, der gegebenenfalls durch ein oder mehrere Heteroatome unterbrochen ist, stehen;
- n und z eine ganze Zahl im Bereich von 1 bis 20 bedeuten, wobei $n+z \geq 2$ und wobei w eine ganze Zahl im Bereich von 1 bis 3 bedeutet;
- $L_1$ unabhängig für einen zweiwertigen aliphatischen $C_1$-$C_{18}$-Rest auf Kohlenwasserstoffbasis, einen $C_3$-$C_{15}$-Cycloalkylenrest, eine $C_3$-$C_{12}$-Heterocycloalkylengruppe oder eine $C_6$-$C_{14}$-Arylengruppe und Mischungen davon steht;
- E unabhängig für eine Gruppe steht, die ausgewählt ist aus:

$$-O-R_3-O-; -S-R_4-S-; -R_5-N(R_6)-R_4-N(R_6)-R_5-,$$

wobei $R_3$ und $R_4$ unabhängig für einen zweiwertigen Rest auf Kohlenwasserstoffbasis, der gegebenenfalls durch ein oder mehrere Heteroatome unterbrochen ist, stehen;
- $R_5$ unabhängig für eine kovalente Bindung oder einen gesättigten zweiwertigen Rest auf Kohlenwasserstoffbasis, der gegebenenfalls durch ein oder mehrere Heteroatome unterbrochen ist, steht;
- $R_6$ unabhängig für ein Wasserstoffatom oder einen Rest auf Kohlenwasserstoffbasis, der gegebenenfalls durch ein oder mehrere Heteroatome unterbrochen ist, steht.

**4.** Verfahren nach Anspruch 3, **dadurch gekennzeichnet, dass** die (Poly)carbodiimidverbindung bzw. die (Poly) carbodiimidverbindungen aus den Verbindungen der Formel (II) ausgewählt ist bzw. sind, wobei:

- $X_1$ und $X_2$ unabhängig für ein Sauerstoffatom stehen;
- $R_1$ und $R_2$ unabhängig aus Dialkylaminoalkoholen, Hydroxycarbonsäurealkylestern und Monoalkylethern von (Poly)alkylenglykol, in denen eine Hydroxylgruppe entfernt wurde, und Mischungen davon ausgewählt sind;
- n und z eine ganze Zahl im Bereich von 1 bis 20 mit $n+z \geq 2$ bedeuten und w gleich 1 ist;
- $L_1$ aus einem zweiwertigen aliphatischen $C_1$-$C_{18}$-Rest auf Kohlenwasserstoffbasis, einem $C_3$-$C_{15}$-Cycloalkylenrest, einer $C_3$-$C_{12}$-Heterocycloalkylengruppe oder einer $C_6$-$C_{14}$-Arylengruppe und Mischungen davon ausgewählt ist;
- E unabhängig für eine Gruppe steht, die ausgewählt ist aus:

$$- -O-R_3-O-; -S-R_4-S-; -R_5-N(R_6)-R_4-N(R_6)-R_5-;$$

wobei R3 und R4 unabhängig aus einem $C_6$-$C_{14}$-Arylenrest, einem $C_3$-$C_{12}$-Cycloalkylenrest, einem linearen

oder verzweigten $C_1$-$C_{18}$-Alkylenrest, der gegebenenfalls durch ein oder mehrere Heteroatome unterbrochen ist, und Mischungen davon ausgewählt sind;
- dann, wenn $R_5$ keine kovalente Bindung ist, $R_5$ aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt ist; und
- $R_6$ aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt ist.

**5.** Verfahren nach Anspruch 3 oder 4, **dadurch gekennzeichnet, dass** die (Poly)carbodiimidverbindung bzw. die (Poly) carbodiimidverbindungen aus den Verbindungen der Formel (II) ausgewählt ist bzw. sind, wobei:

- $X_1$ und $X_2$ unabhängig für ein Sauerstoffatom stehen;
- $R_1$ und $R_2$ unabhängig für Monoalkylether von (Poly)alkylenglykol, in denen eine Hydroxylgruppe entfernt wurde, stehen;
- n und z eine ganze Zahl im Bereich von 1 bis 20 mit $n+z \geq 2$ bedeuten und w gleich 1 ist;
- $L_1$ für einen $C_3$-$C_{15}$-Cycloalkylenrest steht;
- E unabhängig für eine Gruppe steht, die ausgewählt ist aus:

$$-O-R_3-O-; -S-R_4-S-; -R_5-N(R_6)-R_4-N(R_6)-R_5-;$$

wobei $R_3$ und $R_4$ unabhängig aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt sind;
- dann, wenn $R_5$ keine kovalente Bindung ist, $R_5$ aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt ist; und
- $R_6$ aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt ist.

**6.** Verfahren nach einem der Ansprüche 3 bis 5, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:

- $X_1$ und $X_2$ unabhängig für ein Sauerstoffatom stehen;
- $R_1$ und $R_2$ unabhängig für die Verbindung der folgenden Formel (VI) stehen:

$$R_{13}-[O-CH_2-C(H)(R_{14})]_q- \qquad (VI),$$

wobei $R_{13}$ für eine $C_1$-$C_4$-Alkylgruppe oder ein Phenyl, vorzugsweise eine $C_1$-$C_4$-Alkylgruppe, weiter bevorzugt ein Methyl, steht, $R_{14}$ für ein Wasserstoffatom oder eine $C_1$-$C_4$-Alkylgruppe, vorzugsweise ein Wasserstoffatom, steht und q eine ganze Zahl im Bereich von 4 bis 30 bedeutet;
- n und z eine ganze Zahl im Bereich von 2 bis 20 mit n+z im Bereich von 4 bis 10 bedeuten und w gleich 1 ist;
- $L_1$ für einen $C_3$-$C_{15}$-Cycloalkylenrest wie Cyclopentylen, Cycloheptylen, Cyclohexylen und 4,4-Dicyclohexylenmethan steht; und
- E für eine Gruppe -O-$R_3$-O- steht, wobei $R_3$ aus einem $C_6$-$C_{14}$-Arylenrest, einem $C_3$-$C_{12}$-Cycloalkylenrest, einem linearen oder verzweigten $C_1$-$C_{18}$-Alkylenrest, der gegebenenfalls durch ein oder mehrere Heteroatome unterbrochen ist, und Mischungen davon ausgewählt ist.

**7.** Verfahren nach einem der Ansprüche 3 bis 6, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:

- $X_1$ und $X_2$ unabhängig für ein Sauerstoffatom stehen;
- $R_1$ und $R_2$ unabhängig für die Verbindung der folgenden Formel (VI) stehen:

$$R_{13}-[O-CH_2-C(H)(R_{14})]_q- \qquad (VI),$$

wobei $R_{13}$ für eine $C_1$-$C_4$-Alkylgruppe oder ein Phenyl, vorzugsweise eine $C_1$-$C_4$-Alkylgruppe, weiter bevorzugt

ein Methyl, steht, $R_{14}$ für ein Wasserstoffatom oder eine $C_1$-$C_4$-Alkylgruppe, vorzugsweise ein Wasserstoffatom, steht und q eine ganze Zahl im Bereich von 4 bis 30 bedeutet;
- n und z eine ganze Zahl im Bereich von 2 bis 20 mit n+z im Bereich von 4 bis 10 bedeuten und w gleich 1 ist;
- $L_1$ für einen $C_3$-$C_{15}$-Cycloalkylenrest wie Cyclopentylen, Cycloheptylen, Cyclohexylen und 4,4-Dicyclohexylenmethan, vorzugsweise 4,4-Dicyclohexylenmethan, steht; und
- E für eine Gruppe -O-$R_3$-O- steht, wobei $R_3$ für einen linearen oder verzweigten $C_1$-$C_{18}$-Alkylenrest wie Methylen, Propylen, Butylen, Ethylen, der gegebenenfalls durch ein oder mehrere Heteroatome unterbrochen ist, steht.

8. Verfahren nach einem der Ansprüche 3 bis 7, **dadurch gekennzeichnet, dass** die (Poly)carbodiimidverbindung bzw. die (Poly)carbodiimidverbindungen aus den Verbindungen der folgenden Formel (XII) ausgewählt ist bzw. sind:

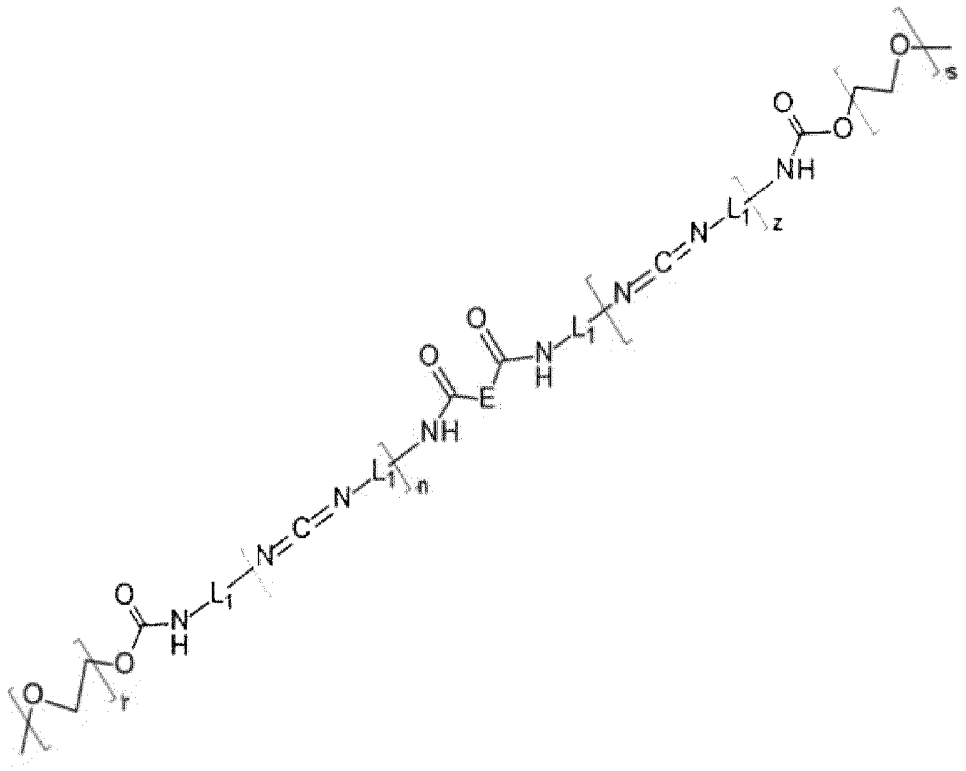

(XII)

,

wobei $L_1$ für 4,4-Dicyclohexylenmethan steht, n und z eine ganze Zahl im Bereich von 2 bis 20 mit n+z im Bereich von 4 bis 10 bedeuten, E für eine Gruppe -O-$R_3$-O- steht, wobei $R_3$ für einen linearen oder verzweigten $C_1$-$C_{18}$-Alkylenrest wie Methylen, Propylen, Butylen, Ethylen, der gegebenenfalls durch ein oder mehrere Heteroatome unterbrochen ist, steht und r und s eine ganze Zahl im Bereich von 4 bis 30 bedeuten.

9. Verfahren nach einem der vorhergehenden Ansprüche, **dadurch gekennzeichnet, dass** die Gesamtmenge der (Poly)carbodiimidverbindung bzw. der (Poly)carbodiimidverbindungen im Bereich von 0,01 bis 20 Gew.-%, vorzugsweise von 0,1 bis 15 Gew.-%, weiter bevorzugt von 0,2 bis 10 Gew.-%, noch weiter bevorzugt von 0,5 bis 8 Gew.-%, noch besser von 1 bis 6 Gew.-%, bezogen auf das Gesamtgewicht der Färbezusammensetzung, liegt.

10. Verfahren nach einem der vorhergehenden Ansprüche, **dadurch gekennzeichnet, dass** die Gesamtmenge an Farbmittel(n) im Bereich von 0,001 bis 20 Gew.-% und vorzugsweise von 0,005 bis 15 Gew.-%, bezogen auf das Gesamtgewicht der Färbezusammensetzung, liegt; wobei das Farbmittel bzw. die Farbmittel vorzugsweise aus Pigmenten ausgewählt ist bzw. sind.

11. Verfahren nach einem der vorhergehenden Ansprüche, **dadurch gekennzeichnet, dass** es sich bei dem Alkyl- oder Alkylencarbonat um ein $C_1$-$C_{30}$- und vorzugsweise $C_1$-$C_6$-Alkylcarbonat oder $C_1$-$C_{30}$- und vorzugsweise $C_1$-$C_6$-Alkylencarbonat handelt; wobei das Alkyl- oder Alkylencarbonat weiter bevorzugt aus Alkylencarbonaten, noch besser

aus Propylencarbonat, Butylencarbonat, Pentylencarbonat und Mischungen davon, ausgewählt ist.

**12.** Verfahren nach einem der vorhergehenden Ansprüche, **dadurch gekennzeichnet, dass** der Gesamtgehalt an Alkyl- oder Alkylencarbonat im Bereich von 10 bis 60 Gew.-%, vorzugsweise von 15 bis 50 Gew.-%, bezogen auf das Gesamtgewicht der Farbentfernungszusammensetzung, liegt.

**13.** Verfahren nach einem der vorhergehenden Ansprüche, **dadurch gekennzeichnet, dass** die zwei Arme A und B um eine Gelenkachse (Z) gelenkig angebracht sind und sich winklig aufeinander zu bewegen, damit die aktiven Teile (3) der Arme A und B miteinander in Kontakt kommen.

**14.** Verfahren nach einem der vorhergehenden Ansprüche, **dadurch gekennzeichnet, dass** die Borsten (6) in Form von Büscheln von Borsten angeordnet sind, die vorzugsweise aus einem natürlichen Material, wie Naturseide, oder Tierhaar, wie Eichhörnchen-, Wildschwein- oder Ziegenhaar, gefertigt sind.

**15.** Verfahren nach Anspruch 14, **dadurch gekennzeichnet, dass** die aktiven Flächen der Arme A und B Reihen (7) von Büscheln von Borsten (6) aufweisen, die durch Freiräume (8) voneinander getrennt sind, wobei die Reihen (7) von Büscheln von Borsten (6) der aktiven Teile (3) parallel zueinander angeordnet sind.

**16.** Verfahren nach einem der vorhergehenden Ansprüche, **dadurch gekennzeichnet, dass** es einen Schritt des Waschens der keratinischen Haarfasern, beispielsweise mit Shampoo, nach dem Anlegen der Zange an die keratinischen Haarfasern umfasst.

**17.** Vorrichtung zum Färben und Entfernen von Farbe von keratinischen Haarfasern wie dem Haar mit mehreren Kompartimenten, die Folgendes enthalten:

- in einem ersten Kompartiment eine Zusammensetzung A, die mindestens eine (Poly)carbodiimidverbindung gemäß einem der Ansprüche 2 bis 8 umfasst, und
- in einem zweiten Kompartiment eine Zusammensetzung B, die mindestens ein Farbmittel, das aus Pigmenten, Direktfarbstoffen und Mischungen davon ausgewählt ist, umfasst,
- in einem dritten Kompartiment eine Zusammensetzung zum Entfernen von Farbe von vorher mit der Färbezusammensetzung gefärbten keratinischen Haarfasern, umfassend mindestens ein Alkyl- oder Alkylencarbonat gemäß einem der Ansprüche 11 und 12, und
- eine Zange (1) gemäß einem der Ansprüche 1 und 13 bis 15.

## Revendications

**1.** Procédé pour retirer la couleur de fibres kératiniques capillaires préalablement colorées à l'aide d'au moins une composition de coloration des fibres kératiniques capillaires comprenant :

- au moins un composé (poly)carbodiimide ; et
- au moins un agent colorant choisi parmi les pigments, les colorants directs et leurs mélanges ;

ledit procédé comprenant les étapes suivantes :

i) l'application sur les fibres kératiniques capillaires préalablement colorées à l'aide de ladite composition de coloration d'au moins une composition pour retirer la couleur comprenant :

- au moins un carbonate d'alkyle ou d'alkylène ; et

ii) l'application sur lesdites fibres kératiniques capillaires d'une pince (1) comprenant deux branches A et B entre lesquelles passent lesdites fibres kératiniques capillaires, chaque branche A et B comprenant une partie de support (2) et une partie active (3), lesdites branches A et B étant mobiles entre une position écartée dans laquelle les parties actives (3) sont à distance l'une de l'autre et une position rapprochée dans laquelle les parties actives (3) sont rapprochées l'une de l'autre,

lesdites parties actives (3) comprenant une face active (4) et une face opposée (5), ladite face active (4) présentant des poils (6),
dans lequel, en position rapprochée des parties actives (3), lesdits poils (6) des branches A et B s'interpénètrent

de façon à enserrer lesdites fibres kératiniques capillaires sans les bloquer.

**2.** Procédé selon la revendication 1, **caractérisé en ce que** le ou les composé(s) (poly)carbodiimide est (sont) choisi(s) parmi les composés de formule (I) suivante :

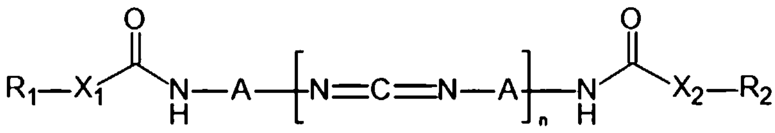

(I)

dans laquelle :

- $X_1$ et $X_2$ représentent, indépendamment, un atome d'oxygène O, un atome de soufre S ou un groupement NH ;
- $R_1$ et $R_2$ représentent indépendamment un groupement choisi parmi un radical hydrocarboné, de préférence alkyle, éventuellement interrompu par un ou plusieurs hétéroatome(s), un groupement choisi parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tel que propylépoxyde ou butylépoxyde, et azacyclopropane, et un radical hydrocarboné, de préférence alkyle, éventuellement interrompu par un ou plusieurs hétéroatome(s) et par un ou plusieurs groupements choisis parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tels que propylépoxyde ou butylépoxyde, et azacyclopropane ;
- n désigne un nombre entier allant de 1 à 1000 ; et
- A est un monomère choisi parmi les composés ci-dessous :

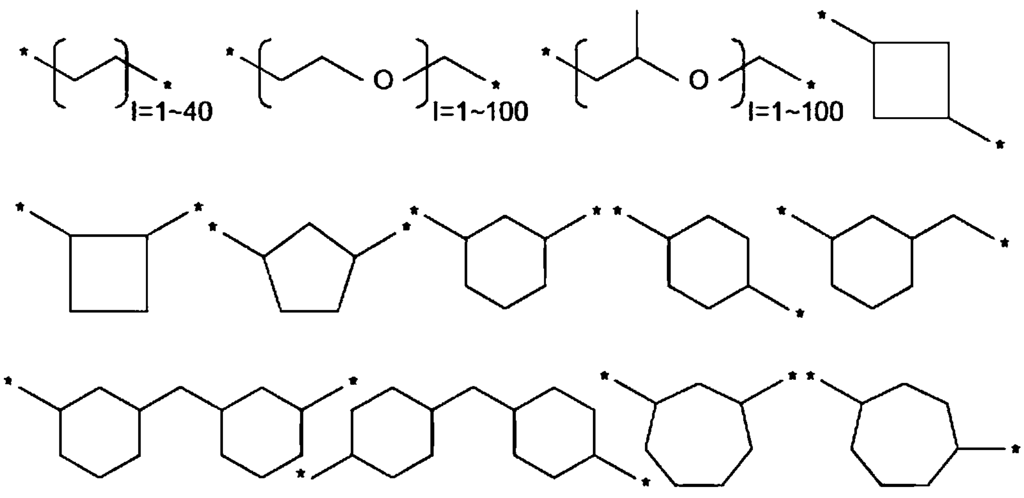

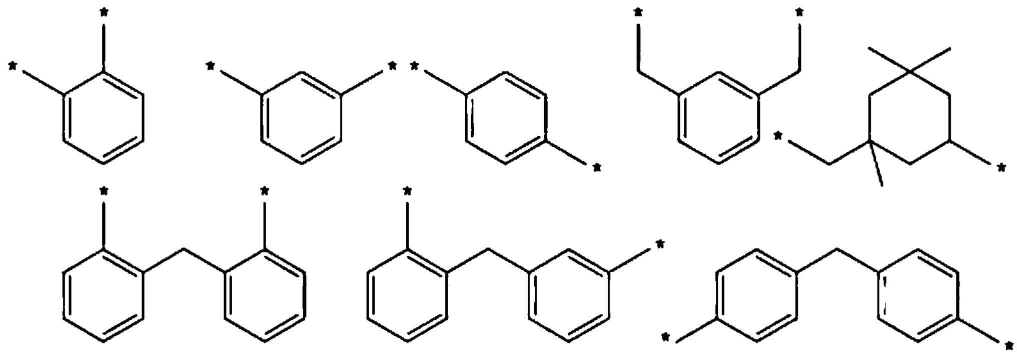

3. Procédé selon la revendication 1, **caractérisé en ce que** le ou les composé(s) (poly)carbodiimide est (sont) choisi(s) parmi les composés de formule (II) suivante :

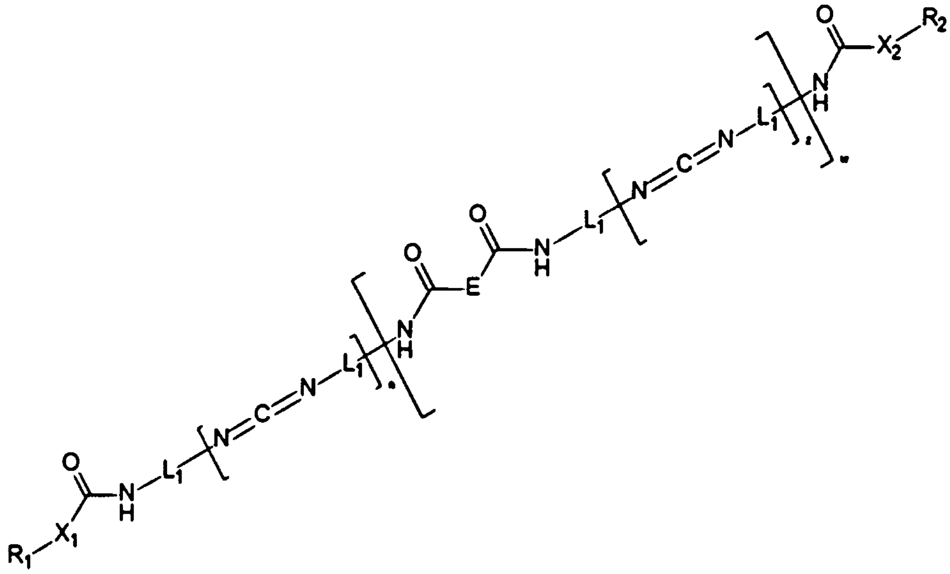

(II),

dans laquelle

- $X_1$ et $X_2$ représentent, indépendamment, un atome d'oxygène O, un atome de soufre S ou un groupement NH ;
- $R_1$ et $R_2$ représentent indépendamment un radical hydrocarboné éventuellement interrompu par un ou plusieurs hétéroatome(s) ;
- n et z désignent un nombre entier allant de 1 à 20, avec $n+z \geq 2$ et w désigne un nombre entier allant de 1 à 3 ;
- $L_1$ représente indépendamment un radical hydrocarboné aliphatique divalent en $C_1$-$C_{18}$, un radical cycloalkylène en $C_3$-$C_{15}$, un groupement hétérocycloalkylène en $C_3$-$C_{12}$, ou un groupement arylène en $C_6$-$C_{14}$, et leurs mélanges ;
- E représente indépendamment un groupement choisi parmi :

$$-O-R_3-O-; -S-R_4-S-; -R_5-N(R_6)-R_4-N(R_6)-R_5-,$$

dans lequel $R_3$ et $R_4$ représentent indépendamment un radical hydrocarboné divalent éventuellement interrompu par un ou plusieurs hétéroatome(s) ;
- $R_5$ représente indépendamment une liaison covalente ou un radical hydrocarboné saturé divalent, éventuellement interrompu par un ou plusieurs hétéroatome(s) ;
- $R_6$ représente indépendamment un atome d'hydrogène, ou un radical hydrocarboné éventuellement interrompu par un ou plusieurs hétéroatome(s).

**4.** Procédé selon la revendication 3, **caractérisé en ce que** le ou les composé(s) (poly)carbodiimide est (sont) choisi(s) parmi les composés de formule (II) dans laquelle :

- $X_1$ et $X_2$ représentent indépendamment un atome d'oxygène ;
- $R_1$ et $R_2$, sont choisis indépendamment parmi les dialkylaminoalcools, les esters alkyliques d'acide hydroxycarboxylique et les éthers monoalkyliques de (poly)alkylèneglycol, dans lesquels un groupement hydroxy a été retiré, et leurs mélanges;
- n et z désignent un nombre entier allant de 1 à 20, avec $n+z \geq 2$ et w est égal à 1 ;
- $L_1$ est choisi parmi un radical hydrocarboné aliphatique divalent en $C_1$-$C_{18}$, un radical cycloalkylène en $C_3$-$C_{15}$, un groupement hétérocycloalkylène en $C_3$-$C_{12}$, ou un groupement arylène en $C_6$-$C_{14}$, et leurs mélanges ;
- E représente indépendamment un groupement choisi parmi :

- $-O-R_3-O-$; $-S-R_4-S-$; $-R_5-N(R_6)-R_4-N(R_6)-R_5-$;

dans lequel R3 et R4 sont choisis indépendamment parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ;
- lorsque $R_5$ n'est pas une liaison covalente, $R_5$ est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ; et
- $R_6$ est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges.

**5.** Procédé selon la revendication 3 ou 4, **caractérisé en ce que** le ou les composé(s) (poly)carbodiimide est (sont) choisi(s) parmi les composés de formule (II) dans laquelle :

- $X_1$ et $X_2$ représentent indépendamment un atome d'oxygène ;
- $R_1$ et $R_2$, sont indépendamment des éthers monoalkyliques de (poly)alkylèneglycol, dans lesquels un groupement hydroxy a été retiré ;
- n et z désignent un nombre entier allant de 1 à 20, avec $n+z \geq 2$ et w est égal à 1 ;
- $L_1$ est un radical cycloalkylène en $C_3$-$C_{15}$ ;
- E représente indépendamment un groupement choisi parmi :

$-O-R_3-O-$; $-S-R_4-S-$; $-R_5-N(R_6)-R_4-N(R_6)-R_5-$ ;

dans lequel $R_3$ et $R_4$ sont choisis indépendamment parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ;
- lorsque $R_5$ n'est pas une liaison covalente, $R_5$ est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ; et
- $R_6$ est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges.

**6.** Procédé selon l'une quelconque des revendications 3 à 5, **caractérisé en ce que** le ou les composé(s) (poly) carbodiimide est (sont) choisi(s) parmi les composés de formule (II) dans laquelle :

- $X_1$ et $X_2$ représentent indépendamment un atome d'oxygène ;
- $R_1$ et $R_2$, représentent indépendamment le composé de formule (VI) suivante :

$$R_{13}-[O-CH_2-C(H)(R_{14})]_q- \qquad (VI),$$

dans laquelle $R_{13}$ représente un groupe alkyle en $C_1$-$C_4$ ou un phényle, de préférence un groupe alkyle en $C_1$-$C_4$, plus préférentiellement un méthyle, $R_{14}$ représente un atome d'hydrogène ou un groupe alkyl en $C_1$-$C_4$, de préférence un atome d'hydrogène et q désigne un nombre entier allant de 4 à 30 ;
- n et z désignent un nombre entier allant de 2 à 20, avec n+z allant de 4 à 10 et w est égal à 1 ;
- $L_1$ est un radical cycloalkylène en $C_3$-$C_{15}$ tel que cyclopentylène, cycloheptylène, cyclohexylène et le 4,4-dicyclohexylène méthane, et

- E représente un groupement -O-$R_3$-O- dans lequel $R_3$ est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges.

7. Procédé selon l'une quelconque des revendications 3 à 6, **caractérisé en ce que** le ou les composé(s) (poly) carbodiimide est (sont) choisi(s) parmi les composés de formule (II) dans laquelle :

- $X_1$ et $X_2$ représentent indépendamment un atome d'oxygène ;
- $R_1$ et $R_2$, représentent indépendamment le composé de formule (VI) suivante :

$$R_{13}\text{-}[O\text{-}CH_2\text{-}C(H)(R_{14})]_q\text{-} \qquad (VI),$$

dans laquelle $R_{13}$ représente un groupe alkyle en $C_1$-$C_4$ ou un phényle, de préférence un groupe alkyle en $C_1$-$C_4$, plus préférentiellement un méthyle, $R_{14}$ représente un atome d'hydrogène ou un groupe alkyle en $C_1$-$C_4$, de préférence un atome d'hydrogène et q désigne un nombre entier allant de 4 à 30 ;
- n et z désignent un nombre entier allant de 2 à 20, avec n+z allant de 4 à 10, et w est égal à 1 ;
- $L_1$ est un radical cycloalkylène en $C_3$-$C_{15}$ tel que le cyclopentylène, le cycloheptylène, le cyclohexylène et le 4,4-dicyclohexylène méthane, de préférence le 4,4-dicyclohexylène méthane ; et
- E représente un groupement- -O-$R_3$-O- dans lequel $R_3$ représente un radical alkylène linéaire ou ramifié en $C_1$-$C_{18}$ tel que le méthylène, propylène, butylène, éthylène, éventuellement interrompu par un ou plusieurs hétéroatome(s).

8. Procédé selon l'une quelconque des revendications 3 à 7, **caractérisé en ce que** le ou les composé(s) (poly) carbodiimide est(sont) choisi(s) parmi les composés de formule (XII) suivante :

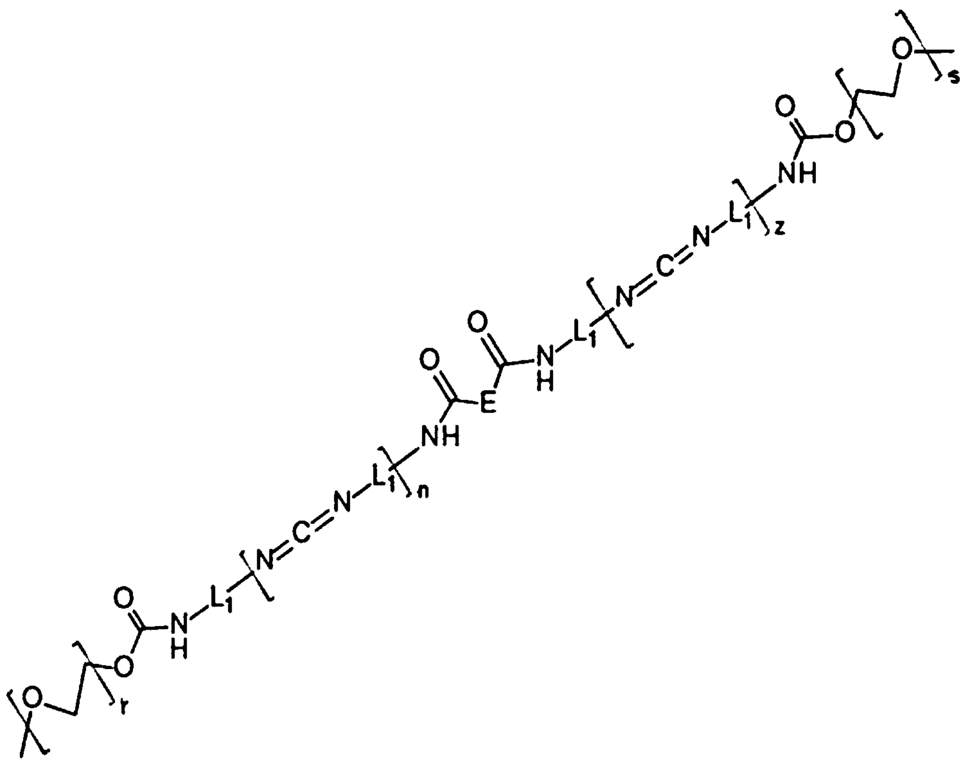

(XII),

dans laquelle $L_1$ est le 4,4-dicyclohexylène méthane, n et z désignent un nombre entier allant de 2 à 20, avec n+z allant de 4 à 10, E représente un groupement- -O-$R_3$-O- dans lequel $R_3$ représente un radical alkylène linéaire ou ramifié en $C_1$-$C_{13}$ tel que méthylène, propylène, butylène éthylène, éventuellement interrompu par un ou plusieurs hétéroatome(s), et r et s désignent un nombre entier allant de 4 à 30.

9. Procédé selon l'une quelconque des revendications précédentes, **caractérisé en ce que** la quantité totale du ou des composé(s) (poly)carbodiimide va de 0,01 à 20% en poids, de préférence de 0,1 à 15% en poids, plus préférentiellement de 0,2 à 10% en poids, encore plus préférentiellement de 0,5 à 8% en poids, mieux de 1 à 6% en poids par rapport au poids total de la composition de coloration.

**10.** Procédé selon l'une quelconque des revendications précédentes, **caractérisé en ce que** la quantité totale en agent(s) colorant(s) va de 0,001 à 20 % en poids, de préférence de 0,005 à 15 % en poids par rapport au poids total de la composition de coloration, de préférence le ou les agents colorants sont choisis parmi les pigments.

**11.** Procédé selon l'une quelconque des revendications précédentes, **caractérisé en ce que** le carbonate d'alkyle ou d'alkylène est un carbonate d'alkyle en $C_1$-$C_{30}$, de préférence en $C_1$-$C_6$, ou d'alkylène en $C_1$-$C_{30}$, de préférence en $C_1$-$C_6$, plus préférentiellement le carbonate d'alkyle ou d'alkylène est choisi parmi les carbonates d'alkylène, mieux parmi le carbonate de propylène, le carbonate de butylène, le carbonate de pentylène et leurs mélanges.

**12.** Procédé selon l'une quelconque des revendications précédentes, **caractérisé en ce que** la teneur totale en carbonate d'alkyle ou d'alkylène va de 10 à 60% en poids, de préférence de 15 à 50% en poids par rapport au poids total de la composition pour retirer la couleur.

**13.** Procédé selon l'une quelconque des revendications précédentes, **caractérisé en ce que** les deux branches A et B sont articulées autour d'un axe d'articulation (Z) et se rapprochent angulairement l'une par rapport à l'autre afin que les parties actives (3) des branches A et B soient en contact l'une par rapport à l'autre.

**14.** Procédé selon l'une quelconque des revendications précédentes, **caractérisé en ce que** lesdits poils (6) sont disposés sous forme de touffes de poils, de préférence réalisés en un matériau naturel, tel que des soies naturelles, ou des poils d'animaux, tels que des poils d'écureuil, de sanglier ou de chèvre.

**15.** Procédé selon la revendication 14, **caractérisé en ce que** les faces actives des branches A et B présentent des rangées (7) de touffes de poils (6) séparées par des espaces libres (8), les rangées (7) de touffes de poils (6) des parties actives (3) étant disposées parallèlement les unes aux autres.

**16.** Procédé selon l'une quelconque des revendications précédentes, **caractérisé en ce qu'**il comprend une étape de lavage des fibres kératiniques capillaires, par exemple à l'aide d'un shampoing, à la suite de l'application de la pince sur les fibres kératiniques capillaires.

**17.** Dispositif pour la coloration et le retrait de la couleur des fibres kératiniques capillaires, telles que les cheveux, comprenant plusieurs compartiments contenant :

- dans un premier compartiment, une composition A comprenant au moins un composé (poly)carbodiimide tel(s) que défini(s) selon l'une quelconque des revendications 2 à 8, et
- dans un deuxième compartiment, une composition B comprenant au moins un agent colorant choisi parmi les pigments, les colorants directs, et leurs mélanges,
- dans un troisième compartiment, une composition pour retirer la couleur des fibres kératiniques capillaires préalablement colorées à l'aide de ladite composition de coloration comprenant au moins un carbonate d'alkyle ou d'alkylène tel que défini selon l'une quelconque des revendications 11 à 12; et
- une pince (1) telle que défini selon l'une quelconque des revendications 1, 13 à 15.

[Fig 1]

Fig. 1

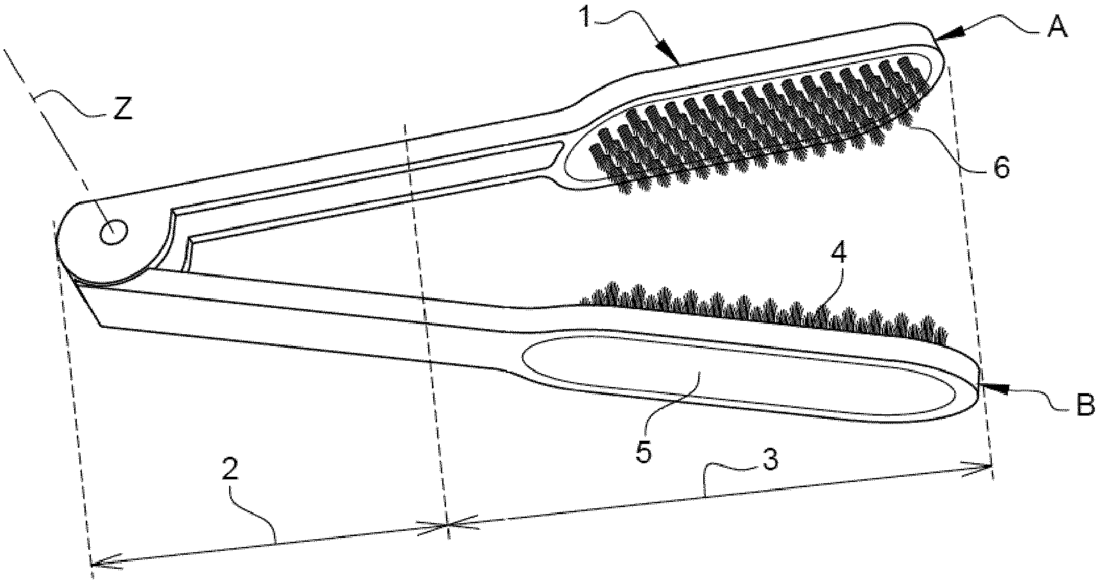


[Fig 2]

Fig. 2

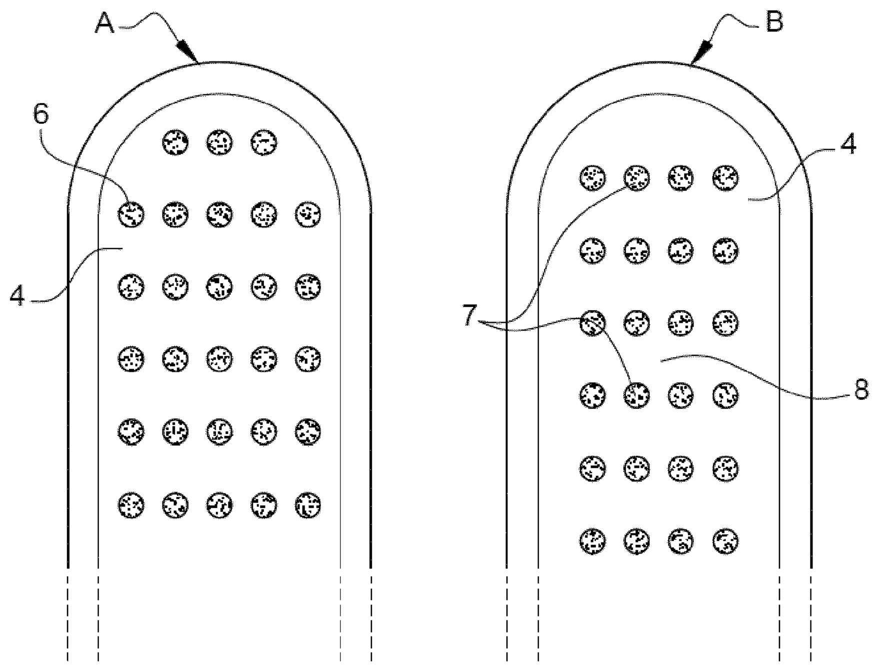

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003034619 B
- FR 2679771
- EP 1184426 A
- JP 09188830 A
- JP 10158450 A
- JP 10158541 A
- JP 07258460 A
- JP 05017710 A
- US 4578266 A
- WO 9515144 A
- WO 9501772 A
- EP 714954 A
- EP 503853 A
- WO 0031154 A
- EP 750899 A
- US 5089578 A
- US 4957732 A
- EP 186507 A
- EP 342834 A
- US 4185087 A
- EP 0530974 A
- US 3915921 A
- US 4509949 A
- EP 0173109 A
- US 3412054 A
- US 7445770 B
- US 7452770 B
- WO 2008155059 A
- WO 2007068371 A
- US 4284730 A
- CA 2509861


### Non-patent literature cited in the description


- *Cosmetics and Toiletries*, February 1990, vol. 105, 53-64
- *Chinese Journal of Polymer Science*, 2000, vol. 18 (40), 323-336
- *Macromolecules*, 2000, vol. 33 (10), 3694-3704
- *Langmuir*, 2000, vol. 16 (12), 5324-5332
- *Polym. Preprint, Div. Polym. Chem.*, 1999, vol. 40 (2), 220-221
- Silicones are particularly described in detail in Walter Noll's. Chemistry and Technology of Silicones. Academic Press, 1968
- **TODD** ; **BYERS**. Volatile silicone fluids for cosmetics. *Cosmetics and Toiletries*, vol. 91, 27-32
- Methods of Molecular Transformations. **HOUBEN - WEYL**. Sciences of Synthesis. Georg Thiem Verlag Kg, 2005